# EUROPEAN PATENT APPLICATION

(11) **EP 4 155 319 A1**
(43) Date of publication of application: **29.03.2023**
(21) Application number: 21832365.7
(22) Date of filing: 29.06.2021
(51) Int. Cl.: C07K 16/28, C12N 15/13, A61P 35/00

(54) **4-1BB BINDING PROTEIN AND APPLICATION THEREOF**

(30) Priority: 30.06.2020 CN 202010619500
(71) Applicant: Nona Biosciences (Suzhou) Co., Ltd., Suzhou, Jiangsu 215000 (CN)
(72) Inventor: SHI, Lei, Shanghai 201203 (CN); HUANG, Bing, Shanghai 201203 (CN); HE, Yun, Shanghai 201203 (CN); GAN, Xin, Shanghai 201203 (CN); CHEN, Fei, Shanghai 201203 (CN); ZHAO, Jianxun, Shanghai 201203 (CN); XIANG, Bo, Shanghai 201203 (CN)
(74) Representative: Maiwald GmbH
(86) International application number: PCT/CN2021/103158
(87) International publication number: WO 2022/002063

(57) **Abstract**

The present invention discloses a 4-1BB-binding protein and use thereof. The 4-1BB-binding protein comprises a heavy chain variable region, wherein the heavy chain variable region comprises HCDR1, HCDR2 and HCDR3, wherein the HCDR1 comprises a sequence as set forth in SEQ ID NO: 15 or a variant 1 thereof, or SEQ ID NO: 16; the HCDR2 comprises a sequence selected from SEQ ID NO: 60 or a variant 2 thereof, and SEQ ID NO: 50 or a variant 4 thereof; the HCDR3 comprises a sequence as set forth in SEQ ID NO: 103 or a variant 3 thereof, SEQ ID NO: 333 or a variant 5 thereof, SEQ ID NO: 110, SEQ ID NO: 111, SEQ ID NO: 116, SEQ ID NO: 326, SEQ ID NO: 331, SEQ ID NO: 334 or SEQ ID NO: 96. The 4-1BB-binding protein of the present invention is a fully human antibody having the binding activity to human 4-1BB and cynomolgus monkey 4-1BB. Some 4-1BB-binding proteins are only half the size of conventional IgG antibodies; they can be well used for bispecific antibodies.

## Description

The present application claims priority to Chinese Patent Application No. 202010619500.9 filed on Jun. 30, 2020, the content of which is incorporated herein by reference in its entirety.

### TECHNICAL FIELD

The present application relates to the field of biomedicine, and in particular to a 4-1BB-binding protein and use thereof.

### BACKGROUND

4-1BB, also known as CD137, the member 9 of the tumor necrosis factor receptor superfamily (TNFRSF9), is an activation inducible co-stimulatory receptor molecule. 4-1BB is expressed mainly on lymphocyte cell lines, such as activated T cells, activated natural killer (NK) cells, activated thymocytes, and intradermal lymphocytes. In addition, 4-1BB is also expressed on dendritic cells, monocytes, centrocytes, and eosinophils. In addition to specific binding of the antigen recognition receptor (TCR) on the T cell surface to the MHC molecule antigen peptide, T cell activation also requires a co-stimulatory signal provided by the binding of the co-stimulatory molecule on the antigen-presenting cell (APC) surface to the corresponding receptor on the T cell surface. The binding of 4-1BB to its ligand 4-1BBL can provide a co-stimulatory signal to activate T cells, enhancing cytokines and immune functions. 4-1BB has also been shown to promote central memory T cell responses, which may support the persistence in tumor-specific T cell therapy and resistance to exhaustion in 4-1BB agonist-treated patients^{[1,2]}. Overexpression of anti-4-1BB single-chain antibody fragments (scFvs) on tumors leads to the elimination of CD4+ T cell and NK cell-dependent tumor clearance^{[3,4,5]}. Systemic injection of anti-4-1BB antibodies in mouse models has also been shown to cause delay in tumor growth^{[6]}.

It has been shown that activating 4-1BB antibodies can activate the 4-1BB downstream pathway in place of their ligands. Currently, activating 4-1BB antibodies under clinical research include urelumab (BMS-663513) and utomilumab (PF-05082566). Utomilumab is a ligand-blocking IgG2 antibody, and Urelumab is a non-ligand-blocking IgG4 antibody. Utomilumab and Urelumab can enhance the function of T cells and promote anti-tumor effects either *in vivo* or *in vitro.* However, clinical trials have shown that at doses greater than 1 mg/kg of urelumab, pneumonia toxicity occurs in some patients (see NCT00309023, NCT00612664 and NCT01471210). Utomilumab is safer than Urelumab, but has less 4-1BB activation activity. Therefore, there is an urgent need to develop safer and more effective novel 4-1BB-targeting therapies.

In order to solve the technical problems of lack of anti-tumor effects, safety and the like in the prior art, the present invention provides a 4-1BB-targeting fully human antibody, a bispecific antibody based on the antibody and a tumor-specific target, and use thereof. The present application provides an anti-4-1BB antigen-binding protein having one or more of the following properties: 1) being capable of binding to human and monkey-derived 4-1BB proteins; 2) being capable of stimulating immune cells to secrete IFN-γ, IL2 and/or TNFα; 3) being capable of inhibiting tumor growth and/or tumor cell proliferation; and 4) being capable of activating the 4-1BB signaling pathway. The present application also provides use of the antigen-binding protein in the prevention and treatment of tumors.

### SUMMARY

The present invention addresses the technical problems that the antibody drugs in the prior art lacks anti-tumor effects and their safety is not good enough, and the like and provides a 4-1BB-targeting binding protein and use thereof, particularly a 4-1BB-targeting fully human antibody, a bispecific antibody based on the antibody and a tumor-specific target, and use thereof.

A first aspect of the present invention provides a 4-1BB-binding protein comprising a heavy chain variable region, wherein the heavy chain variable region comprises HCDR1, HCDR2 and HCDR3, wherein the HCDR1 comprises a sequence as set forth in SEQ ID NO: 15 or a variant 1 thereof, or SEQ ID NO: 16; the HCDR2 comprises a sequence selected from SEQ ID NO: 60 or a variant 2 thereof, and SEQ ID NO: 50 or a variant 4 thereof; the HCDR3 comprises a sequence as set forth in SEQ ID NO: 103 or a variant 3 thereof, SEQ ID NO: 333 or a variant 5 thereof, SEQ ID NO: 110, SEQ ID NO: 111, SEQ ID NO: 116, SEQ ID NO: 326, SEQ ID NO: 331, SEQ ID NO: 334 or SEQ ID NO: 96;
wherein:
the the variant 1 comprises mutations of one or more of T3I, S6N/G/R and Y7F; preferably, the sequence of variant 1 is as set forth in SEQ ID NO: 19, SEQ ID NO: 20, SEQ ID NO: 22 SEQ ID NO: 23, SEQ ID NO: 300 or SEQ ID NO: 24 in the sequence listing;
the variant 2 comprises mutations of one or more of S1G/N/D, G2S/A, S3D, G5D/N/F/S/V and S6T/N/D; preferably, the sequence of variant 2 is as set forth in any one of SEQ ID NOs: 57-59, SEQ ID NO: 61, SEQ ID NO: 49, SEQ ID NOs: 308-317 and SEQ ID NOs: 63-71 in the sequence listing;
the variant 3 comprises mutations of one or more of G2R/D/A/K, S3A/T, S4G/N/A/T/H, E5T/V/M/G, T6A, D7G/S, H9Y/S/N, Y10H, Y11F, N12G/D and V13I/M/T; preferably, the amino acid sequence of variant 3 is as set forth in any one of SEQ ID NO: 101, SEQ ID NO: 102, SEQ ID NOs: 104-106, SEQ ID NO: 108, SEQ ID NO: 109 and SEQ ID NOs: 112-115 in the sequence listing;
the variant 4 comprises mutations of N1I or N1Q; preferably, the amino acid sequence of variant 4 is as set forth in SEQ ID NO: 53 or 54 in the sequence listing;
the variant 5 comprises mutations of E4A/P and/or N13Y; preferably, the amino acid sequence of variant 5 is as set forth in SEQ ID NOs: 327-330 in the sequence listing;
wherein the variant 1, the variant 2, the variant 3, the variant 5 and the variant 4 at least maintains the functions of pre-mutation sequences.

Preferably, the HCDR1, the HCDR2 and the HCDR3 described above comprise sequences as set forth in SEQ ID NO: 16, SEQ ID NO: 50 and SEQ ID NO: 96, respectively; or SEQ ID NO: 16, SEQ ID NO: 53 and SEQ ID NO: 96, respectively; or SEQ ID NO: 16, SEQ ID NO: 54 and SEQ ID NO: 96, respectively; or SEQ ID NO: 19, SEQ ID NO: 57 and SEQ ID NO: 101, respectively; or SEQ ID NO: 15, SEQ ID NO: 58 and SEQ ID NO: 102, respectively; or SEQ ID NO: 20, SEQ ID NO: 59 and SEQ ID NO: 103, respectively; or SEQ ID NO: 20, SEQ ID NO: 60 and SEQ ID NO: 104, respectively; or SEQ ID NO: 15, SEQ ID NO: 61 and SEQ ID NO: 105, respectively; or SEQ ID NO: 15, SEQ ID NO: 60 and SEQ ID NO: 106, respectively; or SEQ ID NO: 20, SEQ ID NO: 63 and SEQ ID NO: 108, respectively; or SEQ ID NO: 20, SEQ ID NO: 60 and SEQ ID NO: 108, respectively; or SEQ ID NO: 22, SEQ ID NO: 64 and SEQ ID NO: 109, respectively; or SEQ ID NO: 15, SEQ ID NO: 60 and SEQ ID NO: 110, respectively; or SEQ ID NO: 15, SEQ ID NO: 65 and SEQ ID NO: 111, respectively; or SEQ ID NO: 22, SEQ ID NO: 66 and SEQ ID NO: 112, respectively; or SEQ ID NO: 15, SEQ ID NO: 49 and SEQ ID NO: 113, respectively; or SEQ ID NO: 20, SEQ ID NO: 60 and SEQ ID NO: 103, respectively; or SEQ ID NO: 15, SEQ ID NO: 63 and SEQ ID NO: 104, respectively; or SEQ ID NO: 15, SEQ ID NO: 60 and SEQ ID NO: 114, respectively; or SEQ ID NO: 23, SEQ ID NO: 67 and SEQ ID NO: 105, respectively; or SEQ ID NO: 24, SEQ ID NO: 68 and SEQ ID NO: 103, respectively; or SEQ ID NO: 15, SEQ ID NO: 60 and SEQ ID NO: 105, respectively; or SEQ ID NO: 20, SEQ ID NO: 69 and SEQ ID NO: 115, respectively; or SEQ ID NO: 15, SEQ ID NO: 70 and SEQ ID NO: 116, respectively; or SEQ ID NO: 20, SEQ ID NO: 71 and SEQ ID NO: 115, respectively; or SEQ ID NO: 300, SEQ ID NO: 308 and SEQ ID NO: 326, respectively; or SEQ ID NO: 300, SEQ ID NO: 309 and SEQ ID NO: 327, respectively; or SEQ ID NO: 300, SEQ ID NO: 310 and SEQ ID NO: 328, respectively; or SEQ ID NO: 300, SEQ ID NO: 308 and SEQ ID NO: 329, respectively; or SEQ ID NO: 300, SEQ ID NO: 311 and SEQ ID NO: 330, respectively; or SEQ ID NO: 300, SEQ ID NO: 312 and SEQ ID NO: 331, respectively; or SEQ ID NO: 300, SEQ ID NO: 308 and SEQ ID NO: 332, respectively; or SEQ ID NO: 300, SEQ ID NO: 313 and SEQ ID NO: 330, respectively; or SEQ ID NO: 300, SEQ ID NO: 314 and SEQ ID NO: 329, respectively; or SEQ ID NO: 300, SEQ ID NO: 315 and SEQ ID NO: 331, respectively; or SEQ ID NO: 300, SEQ ID NO: 314 and SEQ ID NO: 327, respectively; or SEQ ID NO: 300, SEQ ID NO: 316 and SEQ ID NO: 331, respectively; or SEQ ID NO: 300, SEQ ID NO: 308 and SEQ ID NO: 333, respectively; or SEQ ID NO: 300, SEQ ID NO: 317 and SEQ ID NO: 334, respectively, in the sequence listing. See Table a below.

**Table a**

| Antibody No. | HCDR1 SEQ ID NOs: | HCDR1 sequence | HCDR2 SEQ ID NOs: | HCDR2 sequence | HCDR3 SEQ ID NOs: | HCDR3 sequence |
|---|---|---|---|---|---|---|
| PR000197 | 16 | GGSFSGY | 50 | NHSGS | 96 | LTGPFDY |
| PR000447 | 16 | GGSFSGY | 53 | IHSGS | 96 | LTGPFDY |
| PR000448 | 16 | GGSFSGY | 54 | QHSGS | 96 | LTGPFDY |
| PR000980 | 16 | GGSFSGY | 54 | QHSGS | 96 | LTGPFDY |
| PR001758 | 19 | GFTFSRY | 57 | SGSGDD | 101 | EKAGTTGDYYYNVDV |
| PR001759 | 15 | GFTFSSY | 58 | GGSGGD | 102 | EGSNGTDDNYYDVDV |
| PR001760 | 20 | GFTFSNY | 59 | SGSGVS | 103 | EGSSETDDHYYNVDV |
| PR001763 | 20 | GFTFSNY | 60 | SGSGGS | 104 | EGSNGTDDYHYDIDV |
| PR001764 | 15 | GFTFSSY | 61 | SGGGGS | 105 | EGTTETDDYHYNMDV |
| PR001766 | 15 | GFTFSSY | 60 | SGSGGS | 106 | EKTGTTGDYYYDMDV |
| PR001768 | 20 | GFTFSNY | 63 | SGSGDS | 108 | EGTGTTSDYYYNVDV |
| PR001771 | 20 | GFTFSNY | 60 | SGSGGS | 108 | EGTGTTSDYYYNVDV |
| PR001774 | 22 | GFTFSSF | 64 | SGSGDT | 109 | EATAMASDYYYGVDV |
| PR001775 | 15 | GFTFSSY | 60 | SGSGGS | 110 | ERAYDYSNYVDFDY |
| PR001776 | 15 | GFTFSSY | 65 | SSSGGS | 111 | DGVTTPSYYYYYDMDV |
| PR001780 | 22 | GFTFSSF | 66 | SGSGDN | 112 | EDTAVASDYYYNIDV |
| PR001781 | 15 | GFTFSSY | 49 | SGSGGN | 113 | ERTGTTGDYYYNVDV |
| PR001830 | 20 | GFTFSNY | 60 | SGSGGS | 103 | EGSSETDDHYYNVDV |
| PR001831 | 15 | GFTFSSY | 63 | SGSGDS | 104 | EGSNGTDDYHYDIDV |
| PR001833 | 15 | GFTFSSY | 60 | SGSGGS | 114 | EGATETDDYHFNTDV |
| PR001834 | 23 | GFTFSGY | 67 | SGSGNN | 105 | EGTTETDDYHYNMDV |
| PR001836 | 24 | GFIFSNF | 68 | NGSGFN | 103 | EGSSETDDHYYNVDV |
| PR001837 | 15 | GFTFSSY | 60 | SGSGGS | 105 | EGTTETDDYHYNMDV |
| PR001838 | 20 | GFTFSNY | 69 | DGSGGD | 115 | EGSHGTDDSHYDVDV |
| PR001840 | 15 | GFTFSSY | 70 | NSDGSS | 116 | KGSSSYYHYSIEDD |
| PR004469 | 20 | GFTFSNY | 71 | DASGGD | 115 | EGSHGTDDSHYDVDV |
| PR007286 | 300 | GFTFSDY | 308 | SSSGST | 326 | VKPVAGTWDWFDP |
| PR007287 | 300 | GFTFSDY | 309 | SSSGSI | 327 | EREAVAGTLDFDN |
| PR007288 | 300 | GFTFSDY | 310 | SGSGSI | 328 | EREAVAGTLDFDY |
| PR007289 | 300 | GFTFSDY | 308 | SSSGST | 329 | EREPVAGTLDFDN |
| PR007290 | 300 | GFTFSDY | 311 | SSSGTT | 330 | EREEVAGTLDFDY |
| PR007291 | 300 | GFTFSDY | 312 | SGNGST | 331 | VRPGGSGNYWDWFDP |
| PR007292 | 300 | GFTFSDY | 308 | SSSGST | 332 | EREEVAGTLDYDN |
| PR007293 | 300 | GFTFSDY | 313 | NSSGST | 330 | EREEVAGTLDFDY |
| PR007294 | 300 | GFTFSDY | 314 | SGSGTT | 329 | EREPVAGTLDFDN |
| PR007295 | 300 | GFTFSDY | 315 | SNNGST | 331 | VRPGGSGNYWDWFDP |
| PR007296 | 300 | GFTFSDY | 314 | SGSGTT | 327 | EREAVAGTLDFDN |
| PR007297 | 300 | GFTFSDY | 316 | SRSGST | 331 | VRPGGSGNYWDWFDP |
| PR007298 | 300 | GFTFSDY | 308 | SSSGST | 333 | EREEVAGTLDFDN |
| PR007299 | 300 | GFTFSDY | 317 | SSSGRT | 334 | EGRFF |
| PR007300 | 300 | GFTFSDY | 315 | SNNGST | 331 | VRPGGSGNYWDWFDP |
| PR007381 | 20 | GFTFSNY | 71 | DASGGD | 115 | EGSHGTDDSHYDVDV |

A gene encoding an FR of the heavy chain variable region is preferably derived from germline V gene IGHV4-34, IGHV3-23, IGHV3-11 or IGHV3-74, wherein: HFWR1 preferably comprises an amino acid sequence as set forth in any one of SEQ ID NO: 1, SEQ ID NO: 3, SEQ ID NOs: 5-8, SEQ ID NOs: 294-299 and SEQ ID NOs: 10-13; HFWR2 preferably comprises an amino acid sequence as set forth in any one of SEQ ID NO: 27, SEQ ID NOs: 32-36, SEQ ID NOs: 301-307 and SEQ ID NOs: 38-46; HFWR3 preferably comprises an amino acid sequence as set forth in any one of SEQ ID NO: 74, SEQ ID NOs: 79-83, SEQ ID NOs: 318-325 and SEQ ID NOs: 85-92; HFWR4 preferably comprises an amino acid sequence as set forth in SEQ ID NO: 118, SEQ ID NO: 119, SEQ ID NO: 335, SEQ ID NO: 336 or SEQ ID NO: 123. Preferably, the HFWR1 with an amino acid sequence as set forth in SEQ ID NO: 3, the HFWR2 with an amino acid sequence as set forth in SEQ ID NO: 27, the HFWR3 with an amino acid sequence as set forth in SEQ ID NO: 74, and the HFWR4 with an amino acid sequence as set forth in SEQ ID NO: 118; the HFWR1 with an amino acid sequence as set forth in SEQ ID NO: 5, the HFWR2 has an amino acid sequence as set forth in SEQ ID NO: 32, the HFWR3 with an amino acid sequence as set forth in SEQ ID NO: 79, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 33, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 7, the HFWR2 with an amino acid sequence as set forth in SEQ ID NO: 32, the HFWR3 with an amino acid sequence as set forth in SEQ ID NO: 81, and the HFWR4 with an amino acid sequence as set forth in SEQ ID NO: 119; the HFWR1 with an amino acid sequence as set forth in SEQ ID NO: 8, the HFWR2 with an amino acid sequence as set forth in SEQ ID NO: 34, the HFWR3 with an amino acid sequence as set forth in SEQ ID NO: 82, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 35, the HFWR3 with an amino acid sequence as set forth in SEQ ID NO: 83, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 36, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 38, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 39, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 1, the HFWR2 with an amino acid sequence as set forth in SEQ ID NO: 40, the HFWR3 with an amino acid sequence as set forth in SEQ ID NO: 85, and the HFWR4 with an amino acid sequence as set forth in SEQ ID NO: 119; the HFWR1 with an amino acid sequence as set forth in SEQ ID NO: 10, the HFWR2 with an amino acid sequence as set forth in SEQ ID NO: 41, the HFWR3 with an amino acid sequence as set forth in SEQ ID NO: 86, and the HFWR4 with an amino acid sequence as set forth in SEQ ID NO: 118; the HFWR1 with an amino acid sequence as set forth in SEQ ID NO: 6, the HFWR2 with an amino acid sequence as set forth in SEQ ID NO: 35, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 11, the HFWR2 with an amino acid sequence as set forth in SEQ ID NO: 42, the HFWR3 with an amino acid sequence as set forth in SEQ ID NO: 87, and the HFWR4 with an amino acid sequence as set forth in SEQ ID NO: 119; the HFWR1 with an amino acid sequence as set forth in SEQ ID NO: 5, the HFWR2 with an amino acid sequence as set forth in SEQ ID NO: 32, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 8, the HFWR2 with an amino acid sequence as set forth in SEQ ID NO: 34, the HFWR3 with an amino acid sequence as set forth in SEQ ID NO: 88, and the HFWR4 with amino acid sequence as set forth in SEQ ID NO: 119; the HFWR1 with an amino acid sequence as set forth in SEQ ID NO: 12, the HFWR2 with an amino acid sequence as set forth in SEQ ID NO: 34, the HFWR3 with an amino acid sequence as set forth in SEQ ID NO: 89, and the HFWR4 with an amino acid sequence as set forth in SEQ ID NO: 123; the HFWR1 with an amino acid sequence as set forth in SEQ ID NO: 6, the HFWR2 with an amino acid sequence as set forth in SEQ ID NO: 34, the HFWR3 with an amino acid sequence as set forth in SEQ ID NO: 88, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 43, the HFWR3 with an amino acid sequence as set forth in SEQ ID NO: 90, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 44, the HFWR3 with an amino acid sequence as set forth in SEQ ID NO: 91, and the HFWR4 with an amino acid sequence as set forth in SEQ ID NO: 119; the HFWR1 with an amino acid sequence as set forth in SEQ ID NO: 13, the HFWR2 with an amino acid sequence as set forth in SEQ ID NO: 34, the HFWR3 with an amino acid sequence as set forth in SEQ ID NO: 90, and the HFWR4 with an amino acid sequence as set forth in SEQ ID NO: 119; the HFWR1 with an amino acid sequence as set forth in SEQ ID NO: 6, the HFWR2 with an amino acid sequence as set forth in SEQ ID NO: 45, the HFWR3 with an amino acid sequence as set forth in SEQ ID NO: 80, and the HFWR4 with an amino acid sequence as set forth in SEQ ID NO: 119; the HFWR1 with an amino acid sequence as set forth in SEQ ID NO: 1, the HFWR2 with an amino acid sequence as set forth in SEQ ID NO: 46, the HFWR3 with an amino acid sequence as set forth in SEQ ID NO: 92, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 301, the HFWR3 with an amino acid sequence as set forth in SEQ ID NO: 318, and the HFWR4 with an amino acid sequence as set forth in SEQ ID NO: 335; the HFWR1 with an amino acid sequence as set forth in SEQ ID NO: 295, the HFWR2 with an amino acid sequence as set forth in SEQ ID NO: 302, the HFWR3 with an amino acid sequence as set forth in SEQ ID NO: 319, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 302, the HFWR3 with an amino acid sequence as set forth in SEQ ID NO: 320, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 302, the HFWR3 with an amino acid sequence as set forth in SEQ ID NO: 321, and the HFWR4 with an amino acid sequence as set forth in SEQ ID NO: 335; the HFWR1 with an amino acid sequence as set forth in SEQ ID NO: 296, the with an amino acid sequence as set forth in SEQ ID NO: 302, the HFWR3 with an amino acid sequence as set forth in SEQ ID NO: 322, and the HFWR4 with an amino acid sequence as set forth in SEQ ID NO: 335; the HFWR1 with an amino acid sequence as set forth in SEQ ID NO: 297, the HFWR2 with an amino acid sequence as set forth in SEQ ID NO: 303, the HFWR3 with an amino acid sequence as set forth in SEQ ID NO: 318, and the HFWR4 with an amino acid sequence as set forth in SEQ ID NO: 335; the HFWR1 with an amino acid sequence as set forth in SEQ ID NO: 294, the HFWR2 with an amino acid sequence as set forth in SEQ ID NO: 301, the HFWR3 with an amino acid sequence as set forth in SEQ ID NO: 322, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 302, the HFWR3 with an amino acid sequence as set forth in SEQ ID NO: 323, and the HFWR4 with an amino acid sequence as set forth in SEQ ID NO: 118; the HFWR1 with an amino acid sequence as set forth in SEQ ID NO: 298, the HFWR2 with an amino acid sequence as set forth in SEQ ID NO: 302, the HFWR3 with an amino acid sequence as set forth in SEQ ID NO: 322, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 301, the HFWR3 with an amino acid sequence as set forth in SEQ ID NO: 318, and the HFWR4 with an amino acid sequence as set forth in SEQ ID NO: 120; the HFWR1 with an amino acid sequence as set forth in SEQ ID NO: 298, the HFWR2 with an amino acid sequence as set forth in SEQ ID NO: 302, the HFWR3 with an amino acid sequence as set forth in SEQ ID NO: 322, and the HFWR4 with an amino acid sequence as set forth in SEQ ID NO: 335; the HFWR1 with an amino acid sequence as set forth in SEQ ID NO: 294, the HFWR2 with an amino acid sequence as set forth in SEQ ID NO: 301, the HFWR3 with an amino acid sequence as set forth in SEQ ID NO: 318, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 304, the HFWR3 with an amino acid sequence as set forth in SEQ ID NO: 324, and the HFWR4 with an amino acid sequence as set forth in SEQ ID NO: 118; the HFWR1 with an amino acid sequence as set forth in SEQ ID NO: 294, the HFWR2 with an amino acid sequence as set forth in SEQ ID NO: 305, the HFWR3 with an amino acid sequence as set forth in SEQ ID NO: 325, and the HFWR4 with an amino acid sequence as set forth in SEQ ID NO: 336; the HFWR1 with an amino acid sequence as set forth in SEQ ID NO: 299, the HFWR2 with an amino acid sequence as set forth in SEQ ID NO: 306, the HFWR3 with an amino acid sequence as set forth in SEQ ID NO: 318, and the HFWR4 with an amino acid sequence as set forth in SEQ ID NO: 335; or the HFWR1 with an amino acid sequence as set forth in SEQ ID NO: 6, the HFWR2 with an amino acid sequence as set forth in SEQ ID NO: 307, the HFWR3 with an amino acid sequence as set forth in SEQ ID NO: 80, and the HFWR4 with an amino acid sequence as set forth in SEQ ID NO: 119. See Table b below for details.

**Table b**

| Antibody No. | HFWR1 SEQ ID NOs: | HFWR2 SEQ ID NOs: | HFWR3 SEQ ID NOs: | HFWR4 SEQ ID NOs: |
|---|---|---|---|---|
| PR000197 | 3 | 27 | 74 | 118 |
| PR000447 | 3 | 27 | 74 | 118 |
| PR000448 | 3 | 27 | 74 | 118 |
| PR000980 | 3 | 27 | 74 | 118 |
| PR001758 | 5 | 32 | 79 | 119 |
| PR001759 | 6 | 33 | 80 | 119 |
| PR001760 | 7 | 32 | 81 | 119 |
| PR001763 | 8 | 34 | 82 | 119 |
| PR001764 | 6 | 35 | 83 | 119 |
| PR001766 | 6 | 36 | 80 | 119 |
| PR001768 | 6 | 38 | 80 | 119 |
| PR001771 | 6 | 39 | 80 | 119 |
| PR001774 | 1 | 40 | 85 | 119 |
| PR001775 | 10 | 41 | 86 | 118 |
| PR001776 | 6 | 35 | 80 | 119 |
| PR001780 | 11 | 42 | 87 | 119 |
| PR001781 | 5 | 32 | 80 | 119 |
| PR001830 | 8 | 34 | 88 | 119 |
| PR001831 | 12 | 34 | 89 | 123 |
| PR001833 | 6 | 34 | 88 | 119 |
| PR001834 | 6 | 43 | 90 | 119 |
| PR001836 | 6 | 44 | 91 | 119 |
| PR001837 | 13 | 34 | 90 | 119 |
| PR001838 | 6 | 45 | 80 | 119 |
| PR001840 | 1 | 46 | 92 | 118 |
| PR004469 | 6 | 45 | 80 | 119 |
| PR007286 | 294 | 301 | 318 | 335 |
| PR007287 | 295 | 302 | 319 | 118 |
| PR007288 | 294 | 302 | 320 | 118 |
| PR007289 | 294 | 302 | 321 | 335 |
| PR007290 | 296 | 302 | 322 | 335 |
| PR007291 | 297 | 303 | 318 | 335 |
| PR007292 | 294 | 301 | 322 | 118 |
| PR007293 | 294 | 302 | 323 | 118 |
| PR007294 | 298 | 302 | 322 | 118 |
| PR007295 | 294 | 301 | 318 | 120 |
| PR007296 | 298 | 302 | 322 | 335 |
| PR007297 | 294 | 301 | 318 | 118 |
| PR007298 | 294 | 304 | 324 | 118 |
| PR007299 | 294 | 305 | 325 | 336 |
| PR007300 | 299 | 306 | 318 | 335 |
| PR007381 | 6 | 307 | 80 | 119 |

In the present invention, the heavy chain variable region preferably comprises an amino acid sequence as set forth in any one of SEQ ID NO: 168, SEQ ID NO: 171, SEQ ID NO: 172, SEQ ID NOs: 175-180, SEQ ID NOs: 182-196, SEQ ID NOs: 337-352 and SEQ ID NO: 198 in the sequence listing.

Based on the above description, the 4-1BB-binding protein of the present invention further comprises a light chain variable region comprising LCDR1, LCDR2 and LCDR3, wherein the LCDR1 comprises a sequence as set forth in SEQ ID NO: 133, the LCDR2 comprises a sequence as set forth in SEQ ID NO: 145, and the LCDR3 comprises a sequence as set forth in SEQ ID NO: 158. Preferably, a gene encoding an FR of the light chain variable region is derived from germline V gene IGKV3-15, wherein: LFWR1 preferably comprises an amino acid sequence as set forth in SEQ ID NO: 126 or SEQ ID NO: 128, LFWR2 preferably comprises an amino acid sequence as set forth in SEQ ID NO: 140, LFWR3 preferably comprises an amino acid sequence as set forth in SEQ ID NO: 151, and LFWR4 preferably comprises an amino acid sequence as set forth in SEQ ID NO: 164; more preferably, the light chain variable region comprises a sequence as set forth in SEQ ID NO: 201 in the sequence listing or a variant thereof, wherein the variant comprises one or more amino acid residue mutations on the basis of a sequence as set forth in SEQ ID NO: 201; the sequence of resulting light chain variable region after the mutations preferably is shown in SEQ ID NO: 204.

In one specific embodiment of the present invention, the amino acid sequence of heavy chain variable region is as set forth in SEQ ID NO: 168, and the an amino acid sequence of light chain variable region is as set forth in SEQ ID NO: 201; or, the amino acid sequence of heavy chain variable region is as set forth in SEQ ID NO: 171, and the amino acid sequence of light chain variable region is s as set forth in SEQ ID NO: 204; or, the amino acid sequence of heavy chain variable region is as set forth in SEQ ID NO: 172, and the amino acid sequence of light chain variable region is as set forth in SEQ ID NO: 204. See Table c below for details.

**Table c**

| Antibody No. | VL | VH |
|---|---|---|
| PR000197 | 201 | 168 |
| PR000447 | 204 | 171 |
| PR000448 | 204 | 172 |
| PR000980 | 204 | 172 |

In addition, the 4-1BB-binding protein of the present invention may further comprise a heavy chain constant region and/or a light chain constant region; preferably, the heavy chain constant region is selected from that of hIgG1, hIgG2, hIgG3 and hIgG4 or variants thereof, and the light chain constant region is selected from that of a κ chain and a λ chain or mutations thereof.

The variant of hIgG1 comprises one or more of mutations of L234A, L235A, E345R and P329G, more preferably E345R, or a combination of L234A, L235A and E345R, or a combination of L234A, L235A and P329G; the variant of hIgG4 preferably comprises a mutation S228P.

In one preferred embodiment of the present invention, the heavy chain of 4-1BB-binding protein comprises a sequence as set forth in any one of SEQ ID NO: 209, SEQ ID NO: 212, SEQ ID NO: 213, SEQ ID NOs: 216-222, SEQ ID NOs: 224-238, SEQ ID NOs: 353-368 and SEQ ID NO: 240 in the sequence listing, and/or the light chain comprises a sequence as set forth in SEQ ID NO: 246 or SEQ ID NO: 243 in the sequence listing. For example, the amino acid sequence of heavy chain is as set forth in SEQ ID NO: 209, and the amino acid sequence of light chain is as set forth in SEQ ID NO: 243; or, the amino acid sequence of heavy chain is as set forth in SEQ ID NO: 212, and the amino acid sequence of light chain is as set forth in SEQ ID NO: 246; or, the amino acid sequence of heavy chain is as set forth in SEQ ID NO: 213, and the amino acid sequence of light chain is as set forth in SEQ ID NO: 246; or, the amino acid sequence of heavy chain is as set forth in SEQ ID NO: 216, and the amino acid sequence of light chain is as set forth in SEQ ID NO: 246, as shown in Table d below.

**Table d**

| Antibody No. | Light chain | Heavy chain |
|---|---|---|
| PR000197 | 243 | 209 |
| PR000447 | 246 | 212 |
| PR000448 | 246 | 213 |
| PR000980 | 246 | 216 |

The 4-1BB-binding protein of the present invention described above is in the form of a full-length antibody, Fab, Fab', F(ab')₂, Fv, scFv, a bispecific antibody, a multispecific antibody, a heavy-chain antibody, a single-domain antibody or a single-region antibody, or a monoclonal or polyclonal antibody prepared from an antibody as above.

When the 4-1BB-binding protein is a full-length antibody, it comprises a heavy chain comprising a sequence as set forth in SEQ ID NO: 209, and a light chain comprising a sequence as set forth in SEQ ID NO: 243; or a heavy chain comprising a sequence as set forth in SEQ ID NO: 212, and a light chain comprising a sequence as set forth in SEQ ID NO: 246; or a heavy chain comprising a sequence as set forth in SEQ ID NO: 213, and a light chain comprising a sequence as set forth in SEQ ID NO: 246; or a heavy chain comprising a sequence as set forth in SEQ ID NO: 216, and a light chain comprising a sequence as set forth in SEQ ID NO: 246.

A second aspect of the present invention provides a bispecific antibody comprising a first protein functional region and a second protein functional region, wherein the first protein functional region is the 4-1BB-binding protein described above, and the second protein functional region targets a tumor antigen, and is preferably an HER2 antibody or a PD-L1 antibody, wherein the HER2 antibody is preferably trastuzumab or pertuzumab, and the PD-L1 antibody is preferably atezolizumab or PR000265, the PR000265 comprising a heavy chain as set forth in SEQ ID NO: 211 and a light chain as set forth in SEQ ID NO: 245. See the application CN201910944996.4 for more details on the PR000265.

Specifically, the first protein functional region or the second protein functional region may be in the form of scFv, VHH, an immunoglobulin, Fab, Fab', F(ab')₂ or a heavy chain variable region; for example, the first protein functional region is Fab and the second protein functional region is VHH; or, the first protein functional region is Fab, and the second protein functional region is an immunoglobulin; or, the first protein functional region is an immunoglobulin, and the second protein functional region is a heavy chain variable region.

Preferably, the first protein functional region and the second protein functional region and/or a heavy chain variable region and a light chain variable region of the scFv are linked by a linker, the amino acid sequence of the linker is GS, or as set forth in any one of SEQ ID NOs: 273-293 in the sequence listing.

In a specific embodiment of the present invention, the bispecific antibody comprises a polypeptide chain 1 and a polypeptide chain 2, and optionally further comprises a polypeptide chain 3; preferably:
the amino acid sequence of polypeptide chain 1 is as set forth in SEQ ID NO: 244, and the amino acid sequence of polypeptide chain 2 is as set forth in any one of SEQ ID NOs: 251-265; or the amino acid sequence of polypeptide chain 1 is as set forth in SEQ ID NO: 245, and the amino acid sequence of polypeptide chain 2 is as set forth in SEQ ID NO: 271; or the amino acid sequence of polypeptide chain 1 is as set forth in SEQ ID NO: 249, and the amino acid sequence of polypeptide chain 2 is as set forth in SEQ ID NO: 272; or the amino acid sequence of polypeptide chain 1 is as set forth in SEQ ID NO: 245, and the amino acid sequence of polypeptide chain 2 is as set forth in SEQ ID NO: 270; or the amino acid sequence of polypeptide chain 1 is as set forth in in SEQ ID NO: 245, and the amino acid sequence of polypeptide chain 2 is as set forth in SEQ ID NO: 269; or the amino acid sequence of polypeptide chain 1 is as set forth in SEQ ID NO: 245, and the amino acid sequence of polypeptide chain 2 is as set forth in SEQ ID NO: 268; or the amino acid sequence of polypeptide chain 1 is as set forth in SEQ ID NO: 245, and the amino acid sequence of polypeptide chain 2 is as set forth in any one of SEQ ID NOs: 369-382; or the amino acid sequence of polypeptide chain 1 is as set forth in SEQ ID NO: 267, the amino acid sequence of polypeptide chain 2 is as set forth in SEQ ID NO: 266, and the amino acid sequence of polypeptide chain 3 is as set forth in SEQ ID NO: 246; or the amino acid sequence of polypeptide chain 1 is as set forth in SEQ ID NO: 250, the amino acid sequence of polypeptide chain 2 is as set forth in SEQ ID NO: 249, and the amino acid sequence of polypeptide chain 3 is as set forth in SEQ ID NO: 246.

A third aspect of the present invention provides an isolated nucleic acid encoding the 4-1BB-binding protein according to the first aspect of the present invention or the bispecific antibody according to the second aspect of the present invention.

A fourth aspect of the present invention provides an expression vector comprising the isolated nucleic acid according to the third aspect.

A fifth aspect of the present invention provides a host cell comprising the expression vector according to the fourth aspect of the present invention; preferably, the host cell is a prokaryotic cell or a eukaryotic cell. The eukaryotic cell is preferably a mammalian cell.

A sixth aspect of the present invention provides a method for preparing a 4-1BB-binding protein comprising culturing the host cell according to the fifth aspect and obtaining the 4-1BB-binding protein from the culture.

A seventh aspect of the present invention provides a chimeric antigen receptor comprising the 4-1BB-binding protein according to the first aspect of the present invention or the bispecific antibody according to the second aspect of the present invention.

An eighth aspect of the present invention provides an antibody-drug conjugate comprising a cytotoxic agent, and the 4-1BB-binding protein according to the first aspect of the present invention or the bispecific antibody according to the second aspect of the present invention.

The cytotoxic agent is preferably a cytotoxin, a chemotherapeutic agent, a radioisotope, a therapeutic nucleic acid, an immunomodulator, an antiangiogenesis agent, an anti-proliferative pro-apoptotic agent or a cytolytic enzyme. More preferably, the cytotoxic agent is a tubulin synthase inhibitor - methylauristatin F (MMAF), or methylauristatin E (MMAE).

The preparation method for the antibody-drug conjugate may be a conventional method in the art, and preferably the preparation method described in Doronina, 2006, Bioconjugate Chem. 17, 114-124. Preferably, the preparation method produces antibody-drug conjugates with a minimal low conjugate fraction (LCF) of less than 10%.

The antibody-drug conjugate can be present in any physical form known in the art, preferably as a clear solution.

A ninth aspect of the present invention provides a pharmaceutical composition comprising the 4-1BB-binding protein according to the first aspect of the present invention, the bispecific antibody according to the second aspect of the present invention and/or the antibody-drug conjugate according to the eighth aspect of the present invention, and a pharmaceutically acceptable carrier.

The pharmaceutical composition preferably further comprises other anti-tumor antibodies as active ingredients, and/or comprises one or more of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

The pharmaceutically acceptable carrier may be a conventional carrier in the art, and the carrier may be any suitable physiologically or pharmaceutically acceptable auxiliary material. The pharmaceutically acceptable auxiliary material is conventional one in the art, and preferably comprises a pharmaceutically acceptable excipient, a filler, a stabilizer, a diluent, and the like. More preferably, the pharmaceutical composition comprises 0.01% to 99.99% of the protein and/or the antibody-drug conjugate and 0.01% to 99.99% of a pharmaceutically acceptable carrier, the percentage is the mass percentage of the pharmaceutical composition.

Preferably, the pharmaceutical composition is an anti-tumor drug. More preferably, the pharmaceutical composition is a drug for the treatment of gastric cancer, esophageal cancer, lung cancer, ovarian cancer, melanoma, renal cancer, breast cancer, colorectal cancer, liver cancer, pancreatic cancer, bladder cancer, head and neck cancer, bronchial carcinoma, glioma and/or leukemia.

The route of administration for the pharmaceutical composition of the present invention is preferably parenteral administration, injection administration or oral administration. The injection administration preferably includes intravenous injection, intramuscular injection, intraperitoneal injection, intradermal injection or subcutaneous injection. The pharmaceutical composition is in any conventional dosage form in the art, preferably in the form of a solid, semisolid or liquid, i.e., it may be an aqueous solution, a non-aqueous solution or a suspension, more preferably a tablet, capsule, granule, injection, infusion, or the like. More preferably, it is administered intravascularly, subcutaneously, intraperitoneally or intramuscularly. Preferably, the pharmaceutical composition may also be administered as an aerosol or a coarse spray, i.e., administered nasally; or administered intrathecally, intramedullarily or intraventricularly. More preferably, the pharmaceutical composition may also be administered transdermally, percutaneously, topically, enterally, intravaginally, sublingually or rectally.

The dosage level at which the pharmaceutical composition of the present invention is administered can be adjusted depending on the amount of the composition to achieve the desired diagnostic or therapeutic outcome. The dosage regimen may also be a single injection or multiple injections, or an adjusted one. The selected dosage level and regimen is appropriately adjusted depending on a variety of factors including the activity and stability (i.e., half-life) of the pharmaceutical composition, the formulation, the route of administration, combination with other drugs or treatments, the disease or disorder to be detected and/or treated, and the health condition and past medical history of the subject to be treated.

A therapeutically effective dose for the pharmaceutical composition of the present invention may be estimated initially in cell culture experiments or animal models such as rodents, rabbits, dogs, pigs and/or primates. Animal models can also be used to determine the appropriate concentration range and route of administration, and subsequently an effective dose and a route of administration in humans. In general, the determination of and adjustment to the effective amount or dose to be administered and the assessment of when and how to make such adjustments are known to those skilled in the art.

For combination therapy, the above 4-1BB binding protein, the above antibody-drug conjugate and/or an additional therapeutic or diagnostic agent may each be used as a single agent for use within any time frame suitable for performing the intended treatment or diagnosis. Thus, these single agents may be administered substantially simultaneously (i.e., as a single formulation or within minutes or hours) or sequentially. For example, these single agents may be administered within a year, or within 10, 8, 6, 4, or 2 months, or within 4, 3, 2, or 1 week, or within 5, 4, 3, 2, or 1 day.

For additional guidance regarding formulations, doses, dosage regimens, and measurable therapeutic outcomes, see Berkow et al. (2000) The Merck Manual of Medical Information and Merck & Co. Inc., Whitehouse Station, New Jersey; Ebadi (1998) CRC Desk Reference of Clinical Pharmacology*,* etc.

A tenth aspect of the present invention provides use of the 4-1BB-binding protein according to the first aspect, the bispecific antibody according to the second aspect of the present invention and the pharmaceutical composition according to the seventh aspect in the manufacture of a medicament for the treatment and/or prevention of cancer; the cancer is preferably one associated with one or more of HER2, PD-L1 and 4-1BB, e.g., breast cancer, melanoma, lung cancer, gastric cancer, liver cancer, esophageal cancer, cervical cancer, head and neck tumor or colorectal cancer.

In some aspects, the present disclosure provides a method for treating a disease associated with one or more of HER2, PD-L1 and 4-1BB, the method comprises administering to a subject a pharmaceutically effective amount of the 4-1BB-binding protein described above, or the pharmaceutical composition described above or the nucleic acid molecule described above to treat the disease associated with one or more of HER2, PD-L1 and 4-1BB, wherein the disease is preferably a tumor or cancer.

The tumor or cancer is the conventional one in the art with abnormal expression of one or more of HER2, PD-L1 and 4-1BB, e.g., squamous cell carcinoma, myeloma, small-cell lung cancer, non-small cell lung cancer (NSCLC), head and neck squamous cell carcinoma (HNSCC), neuroglioma, Hodgkin lymphoma, non-Hodgkin lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer and skin cancer; most preferably PD-L1 positive squamous cell carcinoma, myeloma, small-cell lung cancer, non-small cell lung cancer (NSCLC), head and neck squamous cell carcinoma (HNSCC), neuroglioma, Hodgkin lymphoma, non-Hodgkin lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer and skin cancer.

An eleventh aspect of the present invention provides a kit comprising the 4-1BB-binding protein according to the first aspect of the present invention, the bispecific antibody according to the second aspect of the present invention, the chimeric antigen receptor according to the seventh aspect of the present invention, the antibody-drug conjugate according to the eighth aspect of the present invention, and/or the pharmaceutical composition according to the ninth aspect of the present invention.
preferably, the kit further comprises (i) a device for administering the antibody or an antigen-binding fragment thereof or the antibody-drug conjugate or the pharmaceutical composition; and/or (ii) instructions.

A twelfth aspect of the present invention provides a combination of kits comprising a kit A and a kit B, wherein:
the kit A comprises the 4-1BB-binding protein according to the first aspect of the present invention, the bispecific antibody according to the second aspect of the present invention, the chimeric antigen receptor according to the seventh aspect of the present invention, the antibody-drug conjugate according to the eighth aspect of the present invention, and/or the pharmaceutical composition according to the ninth aspect of the present invention;
the kit B comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

To solve the above technical problems, the 4-1BB-binding protein according to the first aspect of the present invention, the bispecific antibody according to the second aspect of the present invention, the chimeric antigen receptor according to the seventh aspect of the present invention, the antibody-drug conjugate according to the eighth aspect of the present invention, and/or the pharmaceutical composition according to the ninth aspect of the present invention may also be administered in combination with other drugs, e.g., a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, a vaccine and/or other anti-tumor antibodies (or pharmaceutical compositions comprising the other anti-tumor antibodies).

A thirteenth aspect of the present invention provides a method for diagnosing, treating and/or preventing a 4-1BB-mediated disease or disorder, the method comprises administering to a patient in need thereof a therapeutically effective amount of the 4-1BB-binding protein according to the first aspect of the present invention, the bispecific antibody according to the second aspect of the present invention, the chimeric antigen receptor according to the seventh aspect of the present invention, the antibody-drug conjugate according to the eighth aspect of the present invention, or the pharmaceutical composition according to the ninth aspect of the present invention, or using the combination of kits according to the twelfth aspect of the present invention to treat a patient in need thereof.

Preferably, the disease or disorder is a tumor, preferably a 4-1BB positive tumor, and more preferably gastric cancer, esophageal cancer, lung cancer, ovarian cancer, melanoma, renal cancer, breast cancer, colorectal cancer, liver cancer, pancreatic cancer, bladder cancer, head and neck cancer, bronchial carcinoma, glioma and/or leukemia.

A fourteenth aspect of the present invention provides a method for immuno-detection or determination of 4-1BB, comprising using the 4-1BB-binding protein according to the first aspect of the present invention, the bispecific antibody according to the second aspect of the present invention, the chimeric antigen receptor according to the seventh aspect of the present invention, the antibody-drug conjugate according to the eighth aspect of the present invention, or the pharmaceutical composition according to the ninth aspect of the present invention; preferably, the detection is not for diagnostic and/or therapeutic purposes.

A fifteenth aspect of the present invention provides a combination therapy comprising administering to a patient in need thereof the 4-1BB binding protein according to the first aspect of the present invention , the bispecific antibody according the second aspect of the present invention, the chimeric antigen receptor according to the seventh aspect of the present invention, the antibody-drug conjugate according to the eighth aspect of the present invention or the pharmaceutical composition according to the ninth aspect of the present invention, and a second therapeutic agent; the second therapeutic agent preferably comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

The above preferred conditions may be combined arbitrarily to obtain preferred embodiments of the present invention on the basis of the general knowledge in the art.

The reagents and starting materials used in the present invention are commercially available.

The beneficial effects of the present invention are as follows:
1. The present application provides a fully human antibody of an anti-4-1BB binding protein, which comprise at least one of the following properties: 1) the fully human antibody of the anti-4-1BB binding protein of the present invention comprises a full-length antibody, and a new fully human antibody comprising only a "heavy chain", and has binding activity to human 4-1BB and cynomolgus monkey 4-1BB, wherein the heavy chain of the 4-1BB-binding protein is only half the size of a conventional IgG antibody; due to the absence of a light chain, the antibody can be used for bispecific antibodies, and the problems of light chain mismatching and heterodimerization are solved; it is capable of binding to human and monkey 4-1BB protein; 2) the binding epitopes are different from those of Urelumab; 3) it can activate the 4-1BB signaling pathway, stimulate immune cells to secrete IFN-γ, IL2 and/or TNFα, and inhibit tumor growth and/or tumor cell proliferation; the activity is significantly greater than that of Utomilumab.
2. The PD-L1×4-1BB bispecific antibody provided by the present invention improves the anti-tumor effect and safety through one or more mechanisms of action. Firstly, the PD-L1×4-1BB bispecific antibody can activate T cells by blocking the PD-1/PD-L1 signaling pathway. Secondly, the PD-L1 molecules highly expressed on the tumor cell surfaces can use the bispecific antibody molecules to promote the crosslinking and trimerization of the 4-1BB molecules on the T cell surfaces and activate the downstream signaling pathway, thereby promoting T cell activation and proliferation; its T cell activation ability is even superior to that of Urelumab. Thirdly, the T cell activation effect of the PD-L1×4-1BB bispecific antibody is specifically dependent on the expression of PD-L1, and the bispecific antibody molecule-mediated T cell activation is limited to the tumor microenvironment. Compared to the crosslinking-dependent anti-4-1BB HCAb monoclonal antibodies in the art that cannot activate T cells directly, the PD-L1×4-1BB bispecific antibody of the present invention constructed using HCAb monoclonal antibodies can specifically activate T cells in the presence of cells highly expressing PD-L1, so that the toxic and side effects caused by over-activation of T cells in normal tissues by monoclonal antibodies similar to Urelumab can be avoided. Fourthly, in this example, PD-L1×4-1BB bispecific antibody molecules of a variety of structures are constructed using the antigen-binding domain Fab of the anti-PD-L1 IgG antibody and the antigen-binding domain VH of the 4-1BB HCAb antibody. This shows the flexibility of constructing the molecular structure of bispecific antibodies based on HCAbs, and the T cell function-activating activity is regulated through different structure types, relative positions, binding valences and other parameters. In one aspect, HCAb-based bispecific antibody structures, especially bispecific antibody molecules of IgG-VH tetravalent symmetric structures or bispecific antibody molecules of Fab-HCAb structures, retain the activity of the PD-L1 end and exhibit better T cell activation ability than the corresponding anti-PD-L1 parent monoclonal antibody in MLR experiments; in another aspect, the PD-L1 molecules highly expressed on target cells can mediate crosslinking and trimerization of 4-1BB to transmit T cell activation signals, and their T cell activation ability was even superior to that of Urelumab. Moreover, the bispecific antibody molecules of IgG-VH tetravalent symmetric structures or Fab-HCAb symmetric structures demonstrate better T cell activation ability than bispecific antibody molecules of FIT-Ig structures.
3. The HER2×4-1BB bispecific antibody provided by the present invention improves the anti-tumor effect and safety through one or more mechanisms of action. Firstly, the HER2×4-1BB bispecific antibody retains the original mechanism of action of the HER2 inhibitor (preventing dimerization of HER2, promoting internalization and degradation of HER2, and inhibiting downstream phosphorylation signals). Secondly, the HER2 molecules highly expressed on the tumor cell surfaces can use the bispecific antibody molecules to promote the crosslinking and trimerization of the 4-1BB molecules on the T cell surfaces and activate the downstream signaling pathway, thereby promoting T cell activation and proliferation; its T cell activation ability is even superior to that of Urelumab. Thirdly, the T cell activation effect of the HER2×4-1BB bispecific antibody is specifically dependent on the expression of HER2, and the bispecific antibody molecule-mediated T cell activation is limited to the tumor microenvironment. Different from the crosslinking-dependent anti-4-1BB HCAb monoclonal antibodies in the art that cannot activate T cells directly, the HER2×4-1BB bispecific antibody of the present invention constructed using HCAb monoclonal antibodies can specifically activate T cells in the presence of cells highly expressing HER2, so that the toxic and side effects caused by over-activation of T cells in normal tissues by monoclonal antibodies similar to Urelumab can be avoided. Fourthly, anti-HER2×4-1BB bispecific antibody molecules of an IgG-VH structure and an IgG-scFv structure were constructed in this example, and the T cell function-activating activity is regulated through different structure types, relative positions, binding valences and other parameters. Meanwhile, this shows the flexibility of constructing a bispecific antibody molecular structure based on HCAb.

### BRIEF DESCRIPTION OF THE DRAWINGS

FIGs. 1A-1B: the binding of 4-1BB H2L2 antibodies to CHO-K1 cells overexpressing human or cynomolgus monkey 4-1BB determined by FACS.
FIG. 2: the 4-1BB H2L2 antibody's blocking the binding of 4-1BB ligands to CHO-K1/human 4-1BB cells determined by FACS.
FIGs. 3A-3B: the activation of the 4-1BB signaling pathway by a 4-1BB H2L2 antibody determined using HEK293/4-1BB/NF-kb reporter gene cells.
FIG. 4: the *in vitro* activation of the 4-1BB pathway and induction of activated T cells to secrete IFN-γ by 4-1BB H2L2 antibodies.
FIGs. 5A-5B: the *in vitro* activation of the 4-1BB pathway and induction of activated CD8+T cells and CD4+T cells to secrete IFN-γ by 4-1BB H2L2 antibodies.
FIGs. 6A-6C: the inhibitory activity of a 4-1BB H2L2 antibody PR000448 against tumor growth in a B6-h4-1BB transgenic mouse model of MC38 subcutaneous colon cancer.
FIG. 7: pharmacokinetics of PR000448 in C57BL/6J mice.
FIGs. 8A-8C: the *in vitro* activation of the 4-1BB pathway and induction of activated T cells to secrete IFN-γ, TNF-α and IL-2 by antibody PR000980.
FIGs. 9A-9D: the affinities of 4-1BB H2L2 antibodies for human 4-1BB protein or monkey 4-1BB protein determined by BIACORE.
FIGs. 10A-10B: the inhibitory activity of a 4-1BB H2L2 antibody PR000980 against tumor growth in a B6-h4-1BB transgenic mouse model of MC38 subcutaneous colon cancer.
FIGs. 11A-11M: the binding of 4-1BB HCAb antibodies to CHO-K1 cells overexpressing human 4-1BB determined by FACS.
FIGs. 12A-12L: the binding of 4-1BB HCAb antibodies to CHO-K1 cells overexpressing cynomolgus monkey 4-1BB determined by FACS.
FIGs. 13A-13J: the activation of the 4-1BB signaling pathway by a 4-1BB HCAb antibody determined using HEK293/4-1BB/NF-kb reporter gene cells.
FIGs. 14A-14K: 4-1BB HCAb antibodies' blocking the binding of 4-1BB ligands to CHO-K1/human 4-1BB cells determined by FACS.
FIGs. 15A-15D: the *in vitro* activation of the 4-1BB pathway and induction of activated T cells to secrete IFN-γ by 4-1BB HCAb antibodies.
FIGs. 16A-16B: the *in vitro* activation of the 4-1BB pathway and induction of activated T cells to secrete IFN-γ by 4-1BB HCAb antibodies in the presence or absence of CHO-K1/CD32b crosslinking.
FIGs. 17A-17D: the specific binding of 4-1BB HCAb antibodies to CHO-K1/human 4-1BB cells determined by FACS.
FIGs. 18A-18C: schematic structural diagrams of HER2×4-1BB bispecific antibodies.
FIGs. 19A-19C: the binding of HER2×4-1BB bispecific antibodies to SK-BR-3 cells determined by FACS.
FIGs. 20A-20E: the binding of HER2×4-1BB bispecific antibodies to CHO-K1 cells overexpressing human 4-1BB determined by FACS.
FIGs. 21A-21B: the binding of HER2×4-1BB bispecific antibodies to CHO-K1 cells overexpressing cynomolgus monkey 4-1BB determined by FACS.
FIGs. 22A-22D: the *in vitro* activation of the 4-1BB pathway and induction of activated T cells to secrete IFN-γ by HER2×4-1BB bispecific antibodies in the presence of SK-BR-3 cell crosslinking.
FIGs. 23A-23E: schematic structural diagrams of PD-L1×4-1BB bispecific antibodies.
FIGs. 24A-24E: the binding of PD-L1×4-1BB bispecific antibodies to CHO-K1/hPD-L1 cells determined by FACS.
FIGs. 25A-25E: the binding of PD-L1×4-1BB bispecific antibodies to CHO-K1 cells overexpressing human 4-1BB determined by FACS.
FIGs. 26A-26B: the binding of PD-L1×4-1BB bispecific antibodies to CHO-K1 cells overexpressing cynomolgus monkey 4-1BB determined by FACS.
FIGs. 27A-27I : the *in vitro* activation of the 4-1BB pathway and induction of activated T cells to secrete IFN-γ by PD-L1×4-1BB bispecific antibodies in the presence of CHO-K1/hPD-L1 or MDA-MB-231 cell crosslinking.
FIGs. 28A-28K: the activation of T cells by PD-L1×4-1BB bispecific antibody molecules investigated by mixed lymphocyte reaction (MLR).
FIG. 29: pharmacokinetics of PD-L1×4-1BB bispecific antibodies in C57BL/6J mice.

### DETAILED DESCRIPTION

Some technical and scientific terms are specifically defined below. Unless otherwise specifically defined herein, all other technical and scientific terms used herein have the meanings generally understood by those of ordinary skill in the art to which the present invention belongs.

The three-letter and single-letter codes for amino acids used are described as in J. Biol. Chem, 243, p3558 (1968).

The term "antibody" refers to an immunoglobulin, which is of a tetrapeptide chain structure formed by connection between two identical heavy chains and two identical light chains by interchain disulfide bonds. Immunoglobulins differ in amino acid composition and arrangement of their heavy chain constant regions and therefore in their antigenicity. Accordingly, immunoglobulins can be classified into five classes, or isotypes of immunoglobulins, namely IgM, IgD, IgG, IgA and IgE, with their corresponding heavy chains being the µ, δ, γ, α and ε chains, respectively. The Ig of the same class can be divided into different subclasses according to the differences in amino acid composition of the hinge regions and the number and location of disulfide bonds in the heavy chains; for example, IgG can be divided into IgG1, IgG2, IgG3, and IgG4. Light chains are classified into κ or λ chains by the difference in the constant regions. Each of the five classes of Ig can have a κ chain or a λ chain.

The antibody light-chain antibody may further comprise a light chain constant region, which may comprise a human κ or λ chain or a variant thereof.

The antibody heavy-chain antibody may further comprise a heavy chain constant region, which may comprise a human IgG1, IgG2, IgG3 or IgG4 or a variant thereof.

The sequences of amino acids of the heavy and light chains of the antibody near the N-terminus vary considerably and thus are referred to as variable regions (V regions); the remaining amino acid sequences near the C-terminus are relatively stable and thus are referred to as constant regions (C regions). The light and heavy chain variable regions each consists of about 110 amino acids, and the amino acid residues in some regions, e.g., positions 24-34, 50-56 and 89-97 in the light chain and positions 31-35, 50-65 and 95-102 in the heavy chain, vary more considerably than other parts of the variable regions. These regions are called hypervariable regions (HVRs), and are also called complementary-determining regions (CDRs) because the hypervariable regions are the sites where an antibody is in direct contact with an antigenic epitope. In the non-hypervariable regions within the variable regions, the amino acid composition and the sequence vary relatively little, and these amino acid residues constitute the stable three-dimensional structure, i.e. the scaffold or framework region (FR), of the variable regions. The variable regions comprise 3 hypervariable regions (HVRs) and 4 framework regions (FRs) with relatively conservative sequences. Each of the light chain variable regions (VLs) and the heavy chain variable region (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2 and HCDR3. The present application adopts the Chothia scheme for division. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the scopes of the Kabat scheme and the Chothia scheme. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

In this application, the term "fully human antibody" generally refers to an antibody that is expressed by a genetically engineered antibody gene-deleted animal into which the entire gene that encodes an antibody in human is transferred. All parts of the antibody (including the variable and constant regions of the antibody) are encoded by genes of human origin. The fully human antibody can greatly reduce the immune side effects caused in the human body by the heterologous antibody. Methods for obtaining fully human antibodies in the art can include phage display, transgenic mice, and the like.

The term "single-chain antibody" is a single-chain recombinant protein formed by linking the heavy chain variable region (VH) and the light chain variable region (VL) of an antibody via a linker peptide. It is the smallest antibody fragment having a complete group of antigen-binding sites.

The term "epitope" refers to a site on an antigen to which an immunoglobulin or antibody specifically binds. An epitope can be formed from contiguous amino acids, or non-contiguous amino acids juxtaposed by tertiary folding of a protein. An epitope formed from contiguous amino acids are generally retained after exposure to a denaturing solvent, while an epitope formed by tertiary folding are generally lost after a denaturing solvent treatment. An epitope generally comprise, for example, at least 3 to 15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance.

The term "nucleic acid" refers to a DNA molecule and an RNA molecule. It may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

In the present application, the term "specifically bind to" generally refers to that an antibody binds to an epitope via its antigen-binding domain, and that the binding requires some complementarity between the antigen-binding domain and the epitope. According to this definition, an antibody is said to "specifically bind to" an antigen when the antibody more easily binds to an epitope via its antigen-binding domain than binds to a random, unrelated epitope.

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form another polypeptide chain, in which case an Fab (cross VH/VL) structure is formed; in certain embodiments, CH1 of the Fab is not linked to the hinge region, but rather the C-terminus of CL is linked to the hinge region of the heavy chain, in which case an Fab (cross Fd/LC) structure is formed.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced using a Harbour HCAb transgenic mouse.

### Example 1. Acquisition of Fully Human 4-1BB H2L2 Antibodies

### Example 1.1. Preparation of Monoclonal Antibodies

The Harbour H2L2 mouse (Harbour Antibodies BV) is a transgenic mouse carrying an immune repertoire of human immunoglobulins that produces antibodies with intact human antibody variable domains and rat constant domains. Harbour H2L2 mice were subjected to multiple rounds of immunization with a soluble recombinant human 4-1BB-Fc fusion protein. When the titer of the 4-1BB-specific antibody in the serum of mice was detected to reach a certain level, spleen cells of the mice were taken and fused with a myeloma cell line to obtain hybridoma cells. After multiple rounds of screening and cloning of the hybridoma cells, hybridoma cell strains 65D4G5G11 and 79B10G8D4 expressing anti-4-1BB monoclonal antibody molecules were separated. The nucleotide sequences encoding the variable domains of the antibody molecules and the corresponding amino acid sequences were obtained through conventional sequencing means for hybridomas. In this example, the sequences of the variable domains of the monoclonal antibody molecules obtained from immunized Harbour H2L2 mice were human antibody sequences. The CDR sequences of antibody variable domains can be analyzed using the Kabat scheme, the Chothia scheme, or a scheme called the Combined scheme which combines the Kabat scheme and the Chothia scheme. The CDR sequences in the examples of the present application were defined according to the Chothia scheme.

### Example 1.2. Preparation of recombinant fully human antibodies

After obtaining the sequences of light and heavy chain variable domains encoding the antibody molecules, the sequences of the light and heavy chain variable domains can be fused with the corresponding sequences of the light and heavy chain constant domains of the human antibody and expressed by conventional recombinant DNA techniques to obtain recombinant antibody molecules.

The antibody number of the 65D4G5G11 clone is PR000196, and that of the 79B10G8D4 clone is PR000197.

In addition, anti-4-1BB positive control antibodies Urelumab and Utomilumab analogs were produced in the present application. The antibody number corresponding to Urelumab is PR000628, and that corresponding to Utomilumab is PR000483. Their corresponding amino acid sequences were found in the IMGT database.

In this example, the sequence of the light chain variable domain (VL) of the antibody was genetically synthesized and cloned into a mammalian cell expression plasmid vector encoding the sequence of the κ light chain constant domain of the human antibody to encode a full-length light chain producing the antibody. In this example, the sequence of the heavy chain variable domain (VH) of the antibody was genetically synthesized and cloned into a mammalian cell expression plasmid vector encoding the sequence of the heavy chain constant domain of the human IgG4 antibody to encode a full-length heavy chain producing the IgG4 antibody. In addition, an S228P mutation (substitution of serine with proline at position 228 according the EU numbering) was introduced into the IgG4 heavy chain constant domain to increase the stability of the IgG4 antibody. In this example, the sequence of the heavy chain variable domain (VH) of the antibody was genetically synthesized and cloned into a mammalian cell expression plasmid vector encoding the sequence of the heavy chain constant domain of the human IgG1 or IgG2 antibody to encode a full-length heavy chain producing the IgG1 or IgG2 antibody. In this example, the sequences of the variable domains of the monoclonal antibody molecules obtained from immunized Harbour H2L2 mice were human antibody sequences, therefore, a fully human anti-4-1BB recombinant antibody was also obtained from this example.

The plasmid encoding the heavy chain of the antibody and the plasmid encoding the light chain of the antibody were simultaneously transfected into a mammalian host cell (e.g., human embryonic kidney cell HEK293), and a purified recombinant antibody with light and heavy chain correctly assembled in pairs can be obtained by the conventional recombinant protein expression and purification techniques. Specifically, HEK293 cells were expanded in FreeStyle^{™} F17 Expression Medium (Thermo, A1383504). Before the transient transfection, the cells were adjusted to a concentration of (6-8)×10⁵ cells/mL, and cultured in a shaker at 37 °C with 8% CO₂ for 24 h to make a concentration of 1.2×10⁶ cells/mL. 30 mL of cultured cells were taken. The plasmid encoding the heavy chain of the antibody and the plasmid encoding the light chain of the antibody described above were mixed at a ratio of 2:3. A total of 30 µg of plasmids was dissolved in 1.5 mL of Opti-MEM reduced serum medium (Thermo, 31985088), and the medium was sterilized through a 0.22 µm membrane filter. Then, 1.5 mL of Opti-MEM was dissolved in 120 µL of 1 mg/mL PEI (Polysciences Inc, 23966-2), and the mixture was left to stand for 5 min. PEI was slowly added to the plasmid, and incubated at room temperature for 10 min. The mixed solution of plasmid and PEI was slowly dropped dropwise while shaking the culture flask, and the cells were cultured in a shaker at 37 °C with 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, 7311550) containing MabSelect^{™}(GE Healthcare Life Science, 71-5020-91 AE) was equilibrated with PBS (pH 7.4) and rinsed with 2-5 column volumes of PBS. The supernatant sample was loaded onto a column. The column was rinsed with 5-10 column volumes of PBS. The target protein was eluted with 0.1 M glycine (pH 3.5). The eluate was adjusted to neutrality with Tris-HCl (pH 8.0), and concentrated and buffer exchanged into PBS buffer with an ultrafiltration tube (Millipore, UFC901024) to obtain a purified antibody solution. Finally, the purified solution was determined for concentration using NanoDrop (Thermo Scientific^{™} NanoDrop^{™} One), subpackaged and stored for later use.

### Example 1.3. Sequence analysis and optimization of antibodies

The sequences of the heavy chain variable domain of the antibody are derived from events such as gene rearrangements of germline gene V, D and J segments of heavy chain gene clusters and somatic hypermutations on chromosomes; the sequences of the light chain variable domain are derived from the events such as gene rearrangements of germline gene V and J segments of κ or λ light chain gene clusters and somatic hypermutations. Gene rearrangement and somatic hypermutation are major factors in increasing antibody diversity. Antibodies derived from the same germline V gene segment may also produce different sequences, but with relatively high similarity overall. The germline gene segments that are likely to undergo gene rearrangement can be deduced from the antibody variable domain sequences using algorithms such as IMGT/DomainGapAlign (http://imgt.org/3Dstructure-DB/cgi/DomainGapAlign.cgi) or NCBI/IgBLAST (https://www.ncbi.nlm.nih.gov/igblast/). The antibody sequences obtained in Example 1.1 were analyzed and germline gene V segments for the heavy chain variable domain (VH) and light chain variable domain (VL) are listed in Table 1.

Chemical modifications are sometimes introduced after translation and synthesis of protein or polypeptide amino acid chains in cells, known as post-translational modifications (PTMs).. For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have a greater effect on antigen binding or result in changes in the physicochemical properties of the antibody, if they are present in the variable domains, particularly in the antigen binding regions (e.g., CDRs) of an antibody. For example, glycosylation, deamidation, isomerization, oxidation, and the like may increase the instability or heterogeneity of antibody molecules, thereby increasing the difficulty and risk of antibody development. Thus, it is very important for the development of therapeutic antibodies to avoid some potential PTMs. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, it can be predicted that there is an N-linked glycosylation site from the N-x-S/T sequence pattern (asparagine at the first position, any amino acid other than non-proline at the second position, and serine or threonine at the third position). The amino acid sequence patterns leading to PTMs may be derived from germline gene sequences, e.g., the human germline gene fragment IGHV3-33 naturally has a glycosylation pattern NST in the FR3 region; or they may also be derived from somatic hypermutations. The predicted PTMs for the variable domains VH and VL of the antibody of Example 1.1 are listed in Table 1. Specifically, NHS may be a glycosylation site, and NG may be a deamidation site.

**Table 1. Germline gene analysis and post-translational modification site (PTM) analysis of sequences of hybridoma monoclonal antibodies**

| Clone No. | Recombinant antibody | VH germline V gene | VL germline V gene | VH PTM | VL PTM |
|---|---|---|---|---|---|
| 65D4G5G11 | PR000196 | IGHV3-23 | IGKV2-28 | / | NG (LCDR1) |
| 79B10G8D4 | PR000197 | IGHV4-34 | IGKV3-15 | NHS (HCDR2) | / |

The amino acid sequence patterns of PTMs may be disrupted by amino acid mutations, thereby reducing or eliminating the formation of specific PTMs. There are different methods for designing mutations depending on the antibody sequences and PTM sequence patterns. One approach is to replace a "hot spot" amino acid (e.g., N or G in the NG pattern) with an amino acid with similar physicochemical properties (e.g., mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

Antibody Fc domain-mediated effector functions such as ADCC and CDC are also very important biological functions. Different IgG subtypes have different ADCC or CDC functions; for example, IgG1 and IgG3 have strong ADCC and CDC effects, while IgG2 and IgG4 have relatively weak effects. In addition, the inherent effector functions of Fc can be modulated by altering the binding ability of Fc to Fc receptors by amino acid mutation or modification. For example, the "LALA" double mutant (L234A/L235A) in IgG1 can significantly reduce the affinity for FcyRIIIA (CD16A), thereby reducing the ADCC effect. In this example, variable regions from the same antibody may be recombined into different IgG subtypes or mutants, e.g., human IgG4 (S228P), human IgG2, human IgG1 (LALA), to modulate effector functions.

D. Zhang et al. found that the introduction of an E345R mutation at site E345 in the Fc of human IgG was effective in increasing the activation of agonistic OX40 antibodies (*The* Journal of Biological Chemistry, 291:27134-27146, 2016).

The present application also reveals that the E345R mutation is effective in increasing the activation of agonist 4-1BB antibodies. In this example, a range of recombinant antibodies or variants thereof (see Table 2) were obtained by sequence optimization of the anti-4-1BB hybridoma monoclonal antibodies of Table 1 by IgG subtype transformation, variable region mutation and Fc mutation. The amino acid sequences of the light and heavy chain variable domains, the full-length amino acid sequence of the light chain, the full-length amino acid sequence of the heavy chain and the amino acid sequences of the CDRs defined according to the Chothia scheme of the recombinant antibodies obtained in this example are listed in Table 3.1, Table 3.2 and Table a.

**Table 2. Recombinant antibodies obtained by IgG subtype transformation, variable region mutation and Fc mutation**

| **Initial antibody** | **Recombinant antibody (variant)** | **Variable region mutations** | **IgG subtype** | **Fc mutation** |
|---|---|---|---|---|
| 65D4G5G11 | PR000196 | | Human IgG4 | S228P |
| 79B10G8D4 | PR000197 | | Human IgG4 | S228P |
| PR000197 | PR000447 | VH:N52I; VL:F2I | Human IgG4 | S228P |
| PR000197 | PR000448 | VH:N52Q; VL:F2I | Human IgG4 | S228P |
| PR000448 | PR000980 | VH:N52Q; VL:F2I | Human IgG1 | L234A, L235A, E345R |

**Table 3.1. Sequence numbers of antigen-binding proteins**

| **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR000483 | 247 | 214 | 205 | 173 | 136 | 147 | 161 | 18 | 55 | 99 |
| PR000628 | 248 | 215 | 206 | 174 | 137 | 148 | 162 | 16 | 56 | 100 |
| PR000196 | 242 | 208 | 200 | 167 | 132 | 144 | 157 | 15 | 49 | 95 |
| PR000197 | 243 | 209 | 201 | 168 | 133 | 145 | 158 | 16 | 50 | 96 |
| PR000447 | 246 | 212 | 204 | 171 | 133 | 145 | 158 | 16 | 53 | 96 |
| PR000448 | 246 | 213 | 204 | 172 | 133 | 145 | 158 | 16 | 54 | 96 |
| PR000980 | 246 | 216 | 204 | 172 | 133 | 145 | 158 | 16 | 54 | 96 |

**Table 3.2. Specific sequences corresponding to CDR sequence numbers**

| HCDR 1 SEQ ID NO: | Specific sequence | HCDR 2 SEQ ID NO: | Specific sequence | HCDR3 SEQ ID NO: | Specific sequence |
|---|---|---|---|---|---|
| 15 | GFTFSSY | 49 | SGSGGN | 95 | EAYHYGSGSYYDDYY YGMDV |
| 16 | GGSFSGY | 50 | NHSGS | 96 | LTGPFDY |
| 18 | GYSFSTY | 53 | IHSGS | 99 | GYGIFDY |
| | | 54 | QHSGS | 100 | DYGPGNYDWYFDL |
| | | 55 | YPGDSY | | |
| | | 56 | NHGGY | | |
| | | | | | |

| LCDR 1 SEQ ID NO: | Specific sequence | LCDR 2 SEQ ID NO: | Specific sequence | LCDR3 SEQ ID NO: | Specific sequence |
|---|---|---|---|---|---|
| 132 | RSSQSLLHSNGYNYL D | 144 | LGSNRAS | 157 | MQALQTPLT |
| 133 | RASQSISSILA | 145 | GASTRAT | 158 | QQYYNWPLT |
| 136 | SGDNIGDQYAH | 147 | QDKNRPS | 161 | ATYTGFGSLAV |
| 137 | RASQSVSSYLA | 148 | DASNRAT | 162 | QQRSNWPPALT |

### Example 1.4. Binding of 4-1BB H2L2 antibodies to 4-1BB on cell surfaces

CHO-K1 cells expressing human 4-1BB and CHO-K1 cells expressing cynomolgus monkey 4-1BB were expanded, then washed 3 times with PBS, and plated on a 96-well plate (Corning, #3799) at 3×10⁵/well. Then 100 µL of a test antigen-binding protein or the positive control antibody Utomilumab or Urelumab was added; the highest concentration was 200 nM, and 5-fold dilution was performed. The plate was incubated at 4 °C for 1 h. The cells were washed twice with FACS buffer, and a 1:1000 diluted goat anti-human IgG AF488 conjugated antibody (Life Technologies, # A11013) was added. The plate was incubated at 4 °C for 1 h. The cells were washed twice with FACS buffer, resuspended in 100 µL of PBS, and subjected to an FACS (BD Biosciences, Canto II) assay, and the fluorescence signals were read. The half maximal effective concentration (EC₅₀) was calculated from the assay results as a basis for evaluation of relative binding activity.

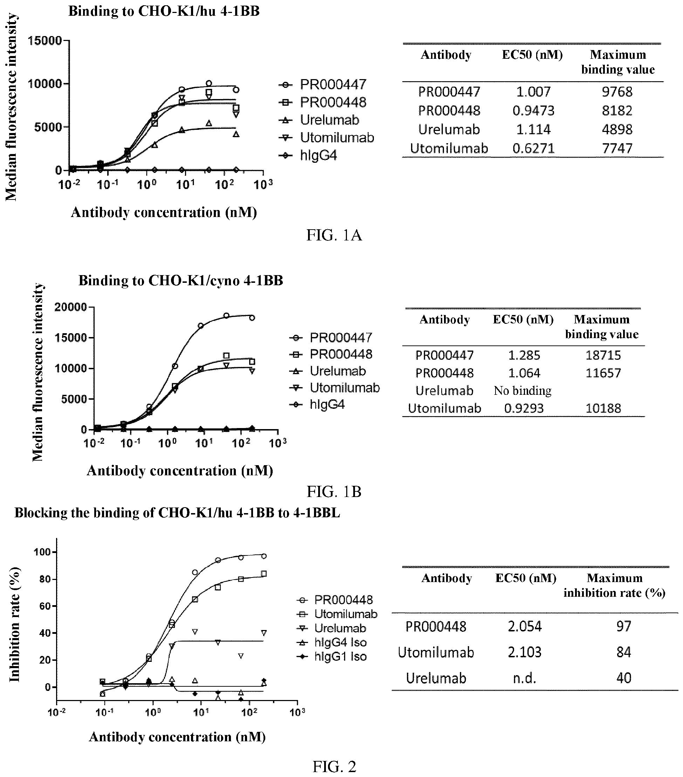

The results are shown in FIG. 1 and Table 4 below. FIG. 1A shows the binding activity of antigen-binding proteins to human 4-1BB. FIG. 1B shows the binding activity of antigen-binding proteins to monkey 4-1BB. Both PR000447 and PR000448 were able to bind to human and cynomolgus monkey 4-1BB, and their binding activities were superior to that of the positive control urelumab. Urelumab was unable to cross-bind to cynomolgus monkey 4-1BB.

**Table 4. Binding of antigen-binding proteins to cells expressing human or monkey 4-1BB**

| **EC₅₀ (nM)** | **PR000447** | **PR000448** | **Urelumab** | **Utomilumab** |
|---|---|---|---|---|
| Human 4-1BB | 1.007 | 0.9473 | 1.114 | 0.6271 |
| Cynomolgus | 1.285 | 1.064 | No binding | 0.9293 |
| monkey 4-1BB | | | | |

### Example 1.5. 4-1BB H2L2 antibody's blocking binding of 4-1BB ligand to 4-1BB

To investigate the *in vitro* activity of the human 4-1BB-binding protein in blocking the binding of human 4-1BB to human 4-1BBL, a human 4-1BB/human 4-1BBL binding blocking experiment was conducted at the cellular level using a CHO-K1 cell strain overexpressing human 4-1BB (CHO-K1/hu4-1BB). Briefly, the CHO-K1/hu4-1BB cells were digested and resuspended in F-12K complete medium, and the cell density was adjusted to 1×10⁶ cells/mL. The cells were seeded in a 96-well V-bottom plate (Corning, #3894) at 100 µL/well, followed by the addition of the test antigen-binding protein serially diluted 3-fold with concentrations that were twice the final concentrations at 100 µL/well. The mixtures were well mixed. The highest final concentration of the antigen-binding protein was 100 nM. A total of 8 concentrations were set. hIgG1 was used as a control. The cells were incubated at 4 °C for 1 h away from light. Thereafter, centrifugation was carried out at 4 °C for 5 min, and the supernatant was discarded, followed by the addition of a biotin-labeled human 4-1BBL protein (Aero, #41L-H82F9) with a concentration of 1 µg/mL at 50 µL/well. The cells were incubated at 4 °C away from light for 30 min. The cells in each well were rinsed twice with 100 µL of pre-cooled PBS, and centrifuged at 500 g at 4 °C for 5 min, and then the supernatant was discarded. A fluorescent secondary antibody (PE Streptavidin, BD, #554061, 1:200) was added at 100 µL/well, and the cells were incubated at 4 °C away from light for 30 min. The cells in each well were rinsed twice with 200 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII. The IC₅₀ was calculated. Inhibition% = 1 - MFI(4-1BB Ab)/MFI(iso).

The results are shown in FIG. 2. PR000448 could block the binding of the 4-1BB ligand to 4-1BB, and the blocking effect was similar to that of Utomilumab.

### Example 1.6. Detection of stimulation of 4-1BB signaling pathway using reporter gene cell line

CD32b-expressing CHO-K1 cells (CHO-K1/CD32b, Genscript, #M00587) or CHO-K1 cells (ATCC, #CCL-61) were plated on a 96-well plate (Perkin Elmer, #6005225) at 1.5×10⁴/well, 100 µL/well. The cells were incubated at 37 °C with 5% CO₂ overnight. The supernatant was removed, and a 2-fold dilution of the test antigen-binding protein was added at 40 µL/well. The initial final concentration was 200 nM, and 5-fold dilution was performed. hlgG1 was used as a control group. HEK293 reporter cells capable of constantly expressing the luciferase reporter genes of 4-1BB and NF-Kb response elements (HEK293/4-1BB/NF-kb reporter cells, BPS Biosciences, #79289) were added at 4.5×10⁴/well, 40 µL/well. The cells were incubated at 37 °C with 5% CO₂ for 6 h. ONE-Glo^{™} luciferase reagent (Promega, #E6110) was added. The cells were incubated at room temperature for 5 min, and the luminescence values were measured using a microplate reader.

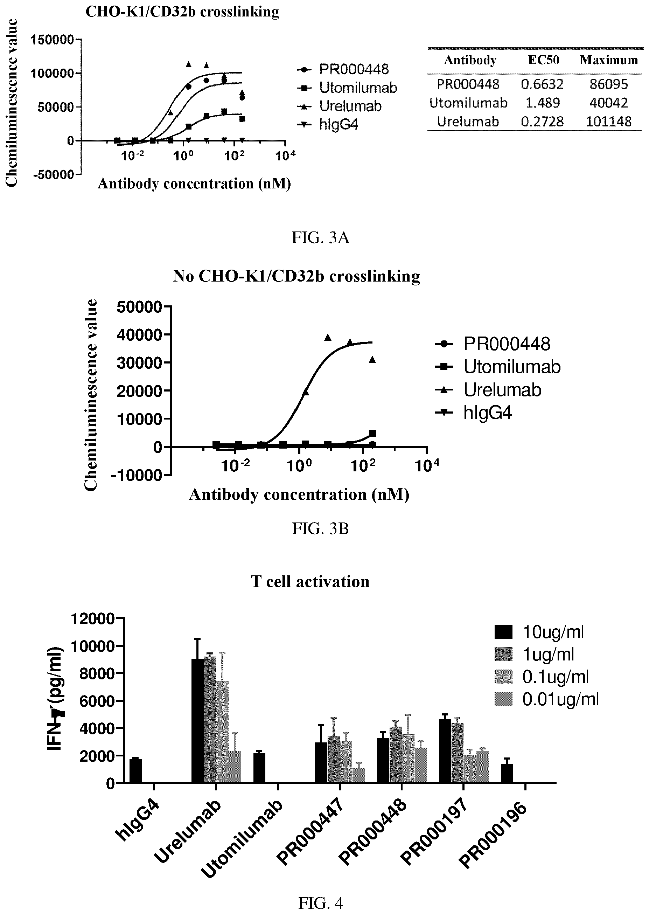

As shown in FIGs 3A and 3B and Table 5 below, the 4-1BB antigen-binding protein PR000448 of the present application was of the CHO-K1/CD32b crosslinking dependent type. As shown in FIG. 3A, the promotion of the 4-1BB-mediated NF-Kb signaling pathway by PR000448 of the present application increased with its concentration in a positive correlation under CHO-K1/CD32b crosslinking. The antibody had a lower EC50 and a higher maximum luminescence value than the control antibody (Utomilumab), which indicates that the antibody was able to promote NF-Kb activation at lower concentrations. As shown in FIG. 3B, the activation of the 4-1BB signaling pathway by the control antibody urelumab was independent of crosslinking; it could also activate the 4-1BB-mediated NF-Kb signaling pathway in the absence of CHO-K1/CD32b crosslinking. PR000448, however, failed to activate the 4-1BB-mediated NF-Kb signaling pathway in the absence of CHO-K1/CD32b crosslinking.

**Table 5. Reporter gene system detection of activation of the 4-1BB signaling pathway by the 4-1BB antibody**

| Antibody | EC₅₀ (nM) | Maximum |
|---|---|---|
| PR000448 | 0.6632 | 86095 |
| Utomilumab | 1.489 | 40042 |
| Urelumab | 0.2728 | 101148 |

### Example 1.7. In vitro functional assay of 4-1BB H2L2 antibodies

### Example 1.7.1. In vitro activation of 4-1BB pathway by 4-1BB H2L2 antibodies

CHO-K1-CD32b cells (CHO-K1 cells overexpressing human CD32b) were treated with 10 µg/mL mitomycin (Beijing Ruitaibio, #10107409001). The cells were let stand at 37 °C for 30 min and then washed 4 times with F-12K culture medium containing 10% FBS. The treated cells were placed in a 96-well plate at 1.5×10⁴ cells/well and incubated in an incubator at 37 °C overnight. The next day, human CD3 positive T cells were isolated from human PBMCs using an MACS kit (Miltenyi Biotec, #130-096-535). The number of the cells was determined firstly, and then corresponding amounts of MACS buffer and Pan-T cell biotin antibody were added according to the number of the cells. The mixture was well mixed and left to stand at 4 °C for 5 min. Then, a corresponding amount of magnetic microbeads was added, and the mixture was left to stand at 4 °C for 10 min. What passed through the LS column were CD3 positive T cells. The previous day's culture medium was washed off the 96-well plate, and purified T cells were added at 1×10⁵ cells/well. Then a 4-1BB antigen-binding protein, the control antibody Utomilumab, or PR000196 was added at a corresponding concentration, and OKT3 (eBiosciences, #16-0037-85) was added at a final concentration of 0.3 µg/mL. The wells were cultured in an incubator at 37 °C for 72 h. After 72 hours, the supernatant was collected and assayed for IFN-γ content using an ELISA kit (Invitrogen, #88-7316-88). A coating antibody was added to a 96-well flat-bottom plate, and the plate was incubated at 4 °C overnight. The next day, ELISA buffer was added, and the plate was incubated at room temperature for 1 h. The collected supernatant was added, and the plated was incubated at room temperature for 2 h. The plate was washed twice, and test antibodies were added. The plate was incubated at room temperature for 1 h. The plate was washed twice, and HRP-streptavidin was added. The plate was incubated at room temperature for 1 h. Then TMB substrate was added, and ELISA stop buffer (BBI, #E661006-0200) was added later. The absorbance values at 450 nm and 570 nm were read using a microplate reader (PerkinElemer Enspire), and the IFN-γ concentration was calculated using OD450-OD570.

The results are shown in FIG. 4. Antibodies PR000197, PR000447 and PR000448 were all able to activate the 4-1BB pathway and induce T cells to secrete IFN-γ, and the activation effects were greater than those of Utomilumab and PR000196.

### Example 1.7.2. Activity of 4-1BB H2L2 antibodies in inducing CD4+ and CD8+ T cells

CHO-K1 or CHO-K1/CD32b cells were treated through the method of Example 1.7.1, plated and incubated in an incubator at 37 °C overnight. The next day, CD8+ and CD4+ T cells were sorted using sorting reagents (Miltenyi Biotec, #130-096-495, #130-096-533). Then a 4-1BB antibody or a control antibody was added at a corresponding concentration, and OKT3 (eBiosciences, #16-0037-85) was added at a final concentration of 0.3 µg/mL. The wells were cultured in an incubator at 37 °C for 72 h. After 72 hours, the supernatant was collected and assayed for IFN-γ content using an ELISA kit (Invitrogen, #88-7316-88).

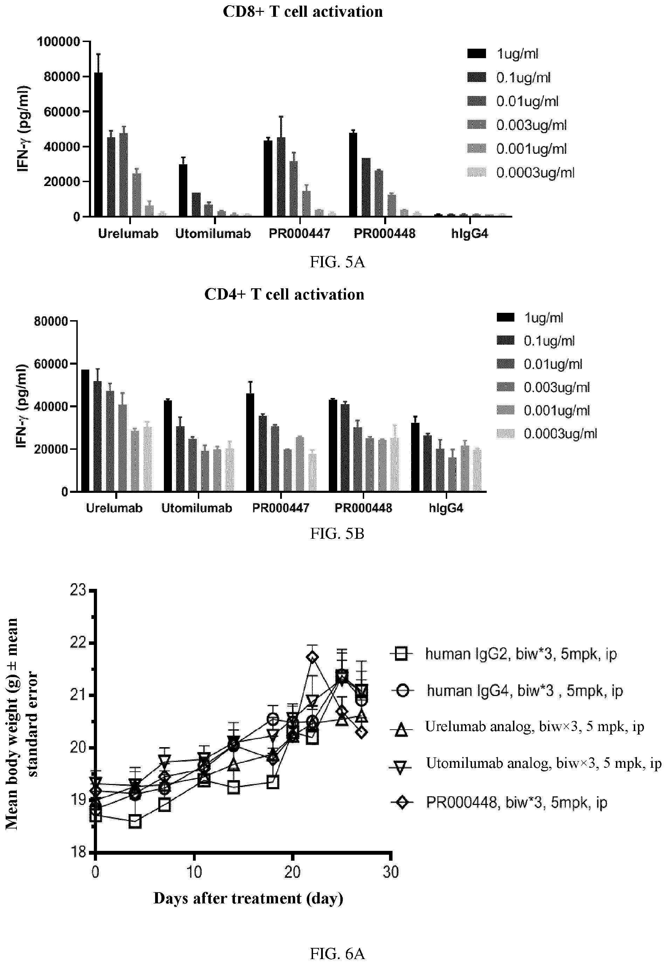

The induction of CD8+ T cells is shown in FIG. 5A, and the induction of CD4+ T cells is shown in FIG. 5B.

The results show that PR000447 and PR000448 significantly activated CD8+ T cells to secrete IFN-γ, but the activation of CD4+ T cells was not significant. This is probably because 4-1BB was expressed at a higher level on CD8+ T cells than on CD4+ T cells.

### Example 1.8. Activity of 4-1BB H2L2 antibody in inhibiting tumor growth in vivo

A B6-h4-1BB transgenic mouse (Beijing Biocytogen Pharmaceuticals Co., Ltd., #110004) model of MC38 subcutaneous colon cancer was established. First, MC38 mouse colon cancer cells (Kerafast, ENH204-FP) were thawed. The culture conditions were as follows: RPMI-1640 medium (Gibco, 22400089) + 100 U/mL penicillin (AMRESCO^{®}, #0242-100MU) + 100 µg/mL streptomycin (AMRESCO^{®}, #0382) + 10% FBS (GIBCO^{®} Invitrogen^{™}, #10099); 37 °C, saturated humidity, 5% CO₂. MC38 cells growing at log phase were collected (the number of passages was recorded), renoved the culture medium , washed twice with PBS and then used for subcutaneous inoculation (98.9% cell viability before tumor bearing and 92% cell viability after tumor bearing) on the right side in an amount of 5×10⁵/100 µL/mouse (no matrigel added). According to grouping criteria, mice were divided into 5 groups of 6 with similar means ranging from 80 to 120 mm³. Enrollment criteria: a single mouse in each group bore a tumor with a volume of no more than 150 mm³, and it would be best for the intra-group SEM not to exceed 1/10 of the mean. The day of grouping was defined as day 0, and administration was started on day 0, the day of grouping.

The test sample was formulated in PBS. The route of administration was intraperitoneal injection. The volume of a dose is 10 µL/g. The dose for administration was 5 mg/kg. The administration was performed at a frequency of twice a week for 3 weeks, making a total of 6 doses. The mouse body weight and tumor volume were measured on the days of administration, twice a week, until the mean tumor volume reached 700-800 mm³. When the mean tumor volume exceeded 700-800 mm³, the tumor volume was measured 3 times a week instead. Tumor volume calculation formula: tumor volume (mm³) = 0.5 × (long diameter of tumor × short diameter of tumor²).

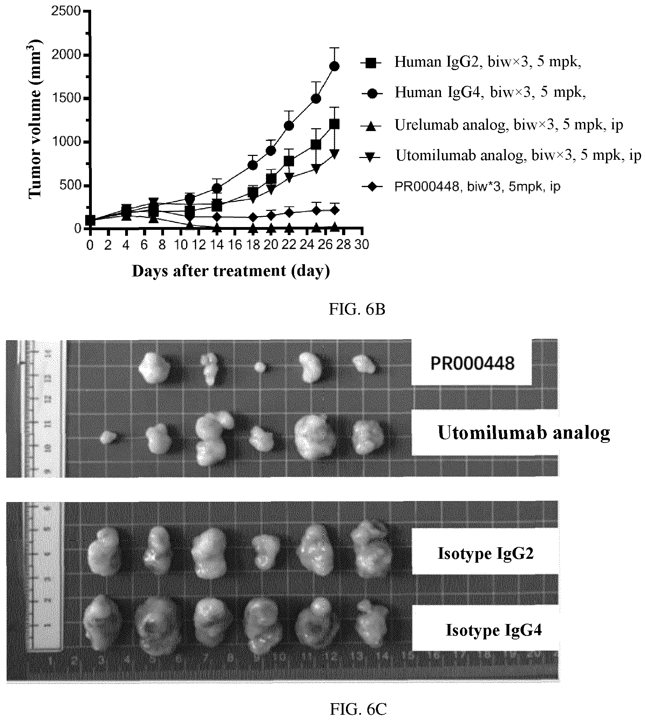

The mouse body weight was monitored as shown in FIG. 6A, and the tumor volume was monitored as shown in FIG. 6B. After 27 days, the mice were euthanized and tumors were took out and photographed. The tumor size was shown in FIG. 6C.

The results show that PR000448 produced a significant inhibitory effect against tumors, and the animals in each group gained weight during the experiment, which indicates that the test sample was well tolerable in the animals; the test sample did not produce a significant toxic effect on the animals, which indicates that it has good safety.

### Example 1.9. Pharmacokinetic study

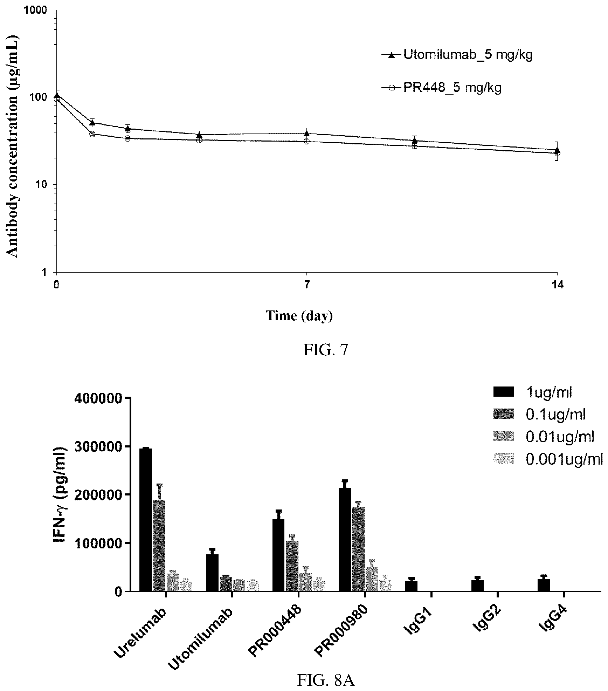

The pharmacokinetics of PR000448 in female C57BL/6J mice was evaluated. PR000448 and Utomilumab were intravenously administered to mice (n = 5) in a single dose of 5 mg/kg, and blood was collected before the injection and 1, 2, 4, 7, 10 and 14 days after the injection. The collected blood was immediately centrifuged at 15,000 rpm at 4 °C for 15 min to obtain plasma, which was stored in a refrigerator at -20 °C or lower. The concentration of the test antibody in the plasma was determined by ELISA. The changes in the plasma concentrations of PR000448 and Utomilumab are shown in FIG. 7. The changes in the plasma concentrations were analyzed in the pharmacokinetic analysis software WinNolin to calculate the half-lives (t_{1/2}) of the drugs, t_{1/2} were calculated from the last three points or the final phase plasma concentrations set automatically by the software. The resulting terminal t_{1/2} is shown in FIG. 7 and Table 6 below.

The results show that PR000448 had a longer half-life than Utomilumab in blood.

**Table 6. Pharmacokinetic parameters of antigen-binding proteins**

| | PR000448 | Utomilumab |
|---|---|---|
| Terminal t_{1/2} (days) | 16.9 | 12.1 |

### Example 1.10. In vitro activation function of 4-1BB H2L2 antibody with Fc point mutations

To evaluate the activation effect of the antibody PR000980 with Fc mutations on the 4-1BB pathway, the IFN-γ, TNF-α and IL-2 contents were determined using ELISA kits (Invitrogen #88-7316-88, Invitrogen #88-7346-88, Invitrogen #88-7025-77) according to the method in Example 1.6.1. Utomilumab, IgG1, IgG2 and IgG4 were used as controls.

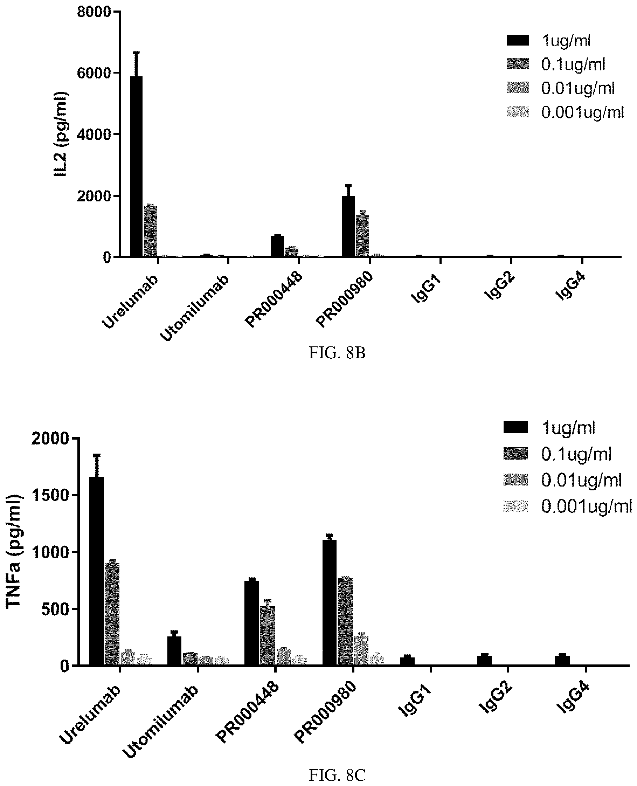

As shown in FIGs. 8A, 8B and 8C, the antigen-binding proteins tested were all able to stimulate activated T cells to secrete cytokines, and their effects were greater than that of Utomilumab; in addition, PR000980 with mutated Fc retained its parent antibody PR000448's ability to stimulate T cells to secrete cytokines (IFN-γ, TNF-α and IL-2).

### Example 1.11. Epitope identification of antigen-binding proteins

The 4-1BB H2L2 antibodies (PR000448 and PR000980) obtained in Example 1 , Utomilumab and Urelumab were subjected to epitope identification on the ForteBio Octet platform. The Urelumab was the anti-4-1BB antibody BMS-663513 (CAS: 934823-49-1) from Bristol-Myers Squibb, obtained by expression and purification according to the method in Example 1.2. As a first step, after capturing histidine-labeled 4-1BB antigens, a sensor was immersed in an antibody, and the 100% signal of the antibody was obtained. As a second step, a primary antibody was loaded onto multiple AHC tips, and a 60-second baseline was obtained in pH 7.5 assay buffer. Then the tips were exposed to histidine-labeled 4-1BB for 180 s to allow antigen binding. The tips were transferred to a well containing a secondary antibody in assay buffer and kept for 90 s, and the final signal was recorded as the signal of the secondary antibody. The inhibition rate was calculated through the following formula: inhibition (%) = (A - B ) / A × 100, where A: the 100% signal of an antibody (obtained from the first step), and B: the signal for the antibody as the secondary antibody (obtained from the second step).

If the secondary antibody exhibited significant binding (i.e., an inhibition less than 40%), it was considered a non-competitor (i.e., in an epitope interval different from that of the primary antibody). If the secondary antibody exhibited no significant binding (i.e., an inhibition greater than or equal to 40%), it was considered a competitor (i.e., in the same epitope interval as the primary antibody). The binding assay was performed by comparing the binding of the secondary antibody to 4-1BB in the presence of the primary antibody to the blocking of the primary antibody itself.

As shown in Table 7 below, the 4-1BB binding sites of PR000448 and PR000980 overlapped with those of Utomilumab to a high degree but with those of Urelumab to a low degree.

**Table 7. Epitope identification of the antigen-binding proteins of the present application and clinical antibodies**

| Inhibition rate (%) | | Secondary antibody | | | |
|---|---|---|---|---|---|
| | | PR000448 | PR000980 | Urelumab | Utomilumab |
| Primary antibody | PR000448 | 98.4 | 90.2 | 18.3 | 90.6 |
| | PR000980 | 101.6 | 98.5 | 21.0 | 95.1 |
| | Urelumab | 32.5 | 18.5 | 95.1 | 34.8 |
| | Utomilumab | 82.8 | 69.3 | 21.8 | 94.9 |

### Example 1.12. Assay for affinities of 4-1BB H2L2 antibodies for human or monkey 4-1BB

The 4-1BB H2L2 antibodies (PR000448 and PR000980) obtained in Example 1 , Utomilumab and Urelumab were subjected to affinity identification on the Biacore platform. In the assay, HBS-EP+ (10 mM HEPES, 150 mM NaCl, 3 mM EDTA and 0.05% P20, pH 7.4, GE Healthcare, BR-1006-69) was used as the running buffer, and series S CM5 (GE Healthcare, BR-1005-30) was used as the experimental chip. The flow rate was set at 10 µL/min first, and Protein A was coupled to the 4 channels of CM5 through the following procedures: 1) the injection time was set to 800 s, and a fresh mixture of 50 mM NHS and 200 mM EDC at a ratio of 1:1 was injected into the 4 channels; 2) Protein A was diluted to 20 µg/mL with pH 4.5 sodium acetate (GE Healthcare, BR-1003-50) and injected into each channel for 800 s; 3) 1 M pH 8.5 ethanolamine was injected for 800 s to block the remaining active carboxyl groups on the chip surface. After blocking, the instrument was equilibrated with 1× HBS-EP + buffer for 2 h, and the final coupling level of Protein A was about 2000 RU. Then an assay for the affinities of the antibodies for proteins was conducted by setting the instrument to the multi-cycle kinetics mode. Each cycle included antibody capture, analyte binding and chip regeneration. Each antibody was diluted to 1 µg/mL, injected into channels 2, 3 and 4 at a flow rate of 10 µL/min for 30 s, and captured by the pre-coupled Protein A at level about 120 RU. Human 4-1BB or cynomolgus monkey 4-1BB was injected into four channels in sequence according to the following concentration gradients: 0 nM, 0.391 nM, 0.781 nM, 1.5625 nM, 3.125 nM, 6.25 nM, 12.5 nM and 25 nM, at a flow rate of 30 µL/min. The dissociation time set for PR000448 and PR000980 was 600 s, and that for Uremulab and Utomilumab was 80 s. The injection time was set to 120 s. Finally, to remove the test antibody from the surface, the chip was regenerated by injecting 10 mM glycine-hydrochloric acid pH 1.5 (GE Life Sciences, BR-1003-54) at the same flow rate for 30 s. The experimental results were analyzed using the Biacore T200 analysis software 2.0, with channel 1 subtracted as a reference channel and a 1:1 kinetic fitting model selected as an analysis model.

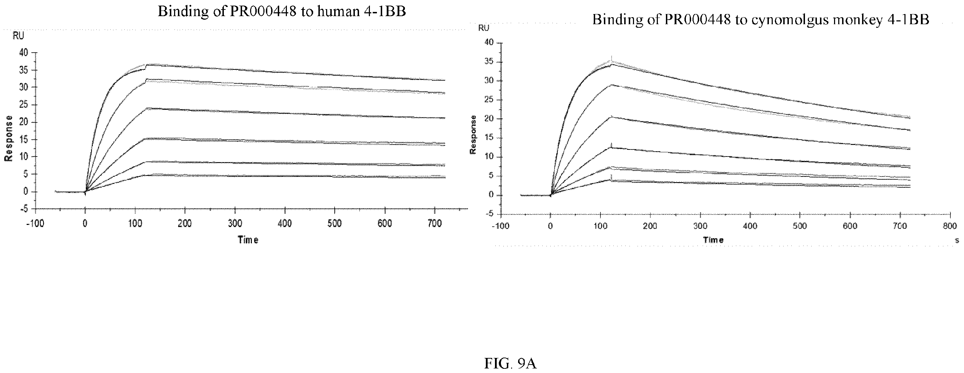

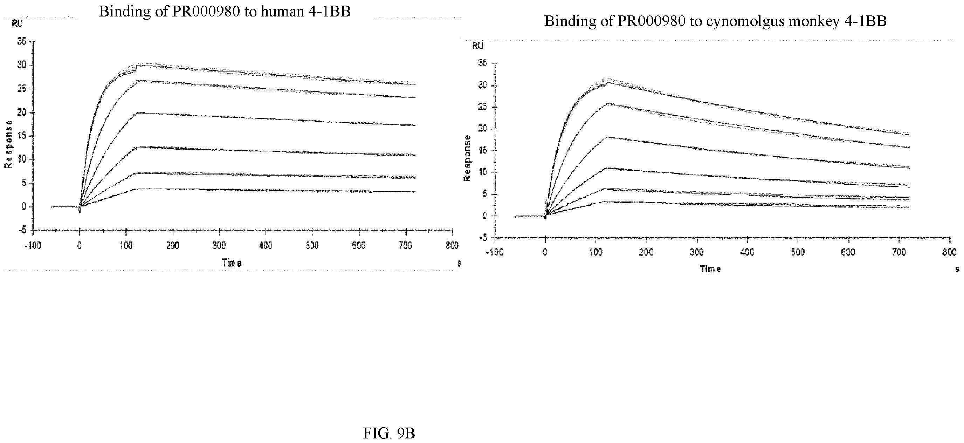

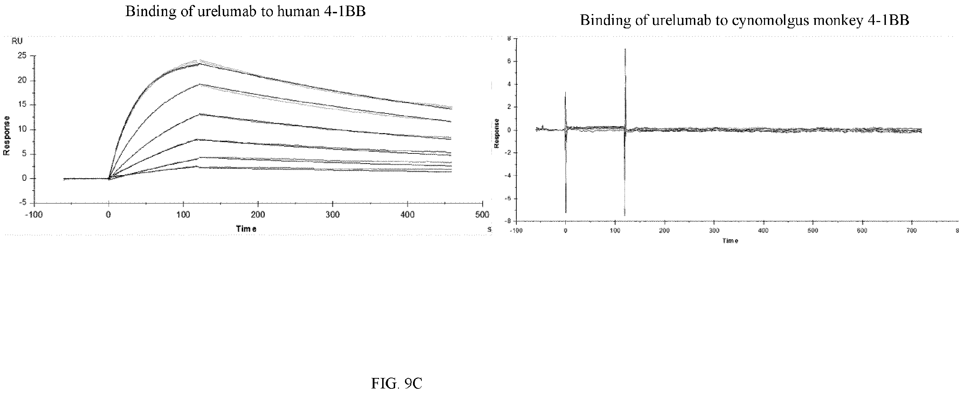

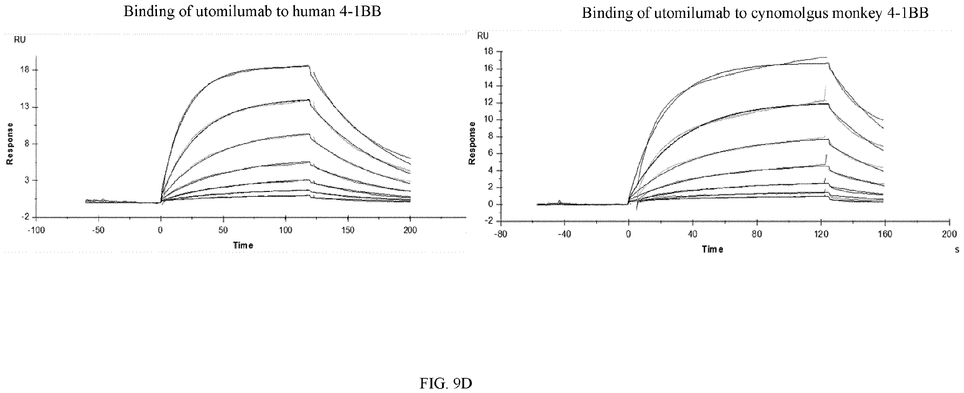

The affinities of the 4-1BB H2L2 antibodies for histidine-labeled human 4-1BB protein and histidine-labeled monkey 4-1BB protein are shown in Table 8 and FIGs. 9A-9D. The results show that the binding affinities of antibodies PR000448 and PR000980, whether for human or monkey 4-1BB protein, were higher than those of Uremulab and Utomilumab, whereas Urelumab did not bind to monkey 4-1BB protein.

**Table 8. Binding affinities of 4-1BB antibodies for human or monkey 4-1BB protein determined by BIACORE**

| Antigen-binding protein | Human 4-1BB protein antigen (histidine-labeled) | | | Monkey 4-1BB protein antigen (histidine-labeled) | | |
|---|---|---|---|---|---|---|
| | kₐ (1/Ms) | k_{d} (1/s) | K_{D} (M) | kₐ (1/Ms) | k_{d} (1/s) | K_{D} (M) |
| PR000448 | 1.42E+06 | 2.20E-04 | 1.55E-10 | 1.19E+06 | 8.92E-04 | 7.51E-10 |
| PR000980 | 1.43E+06 | 2.50E-04 | 1.74E-10 | 1.17E+06 | 8.40E-04 | 7.16E-10 |
| Uremulab | 1.09E+06 | 1.50E-03 | 1.38E-09 | NA | NA | NA |
| Utomilumab | 1.43E+06 | 1.50E-02 | 1.05E-08 | 1.09E+06 | 1.74E-02 | 1.59E-08 |

### Example 1.13. Activity of antibody with Fc point mutations in inhibiting tumor growth in vivo

A B6-h4-1BB transgenic mouse model of MC38 subcutaneous colon cancer was established according to the method in Example 5. When the mean tumor volume reached 99.14 mm³, mice were randomized into groups of 6 according to tumor volume. The day of grouping was defined as day 0, and administration was started on day 0, the day of grouping. The test sample was formulated in PBS. The route of administration was intraperitoneal injection. The dose for administration was 2 mg/kg. After the administration was started, the body weight was measured twice a week. The tumor volume was measured twice a week. The tumor volume was calculated as follows: tumor volume (mm³) = 0.5 × long diameter of tumor × short diameter of tumor².

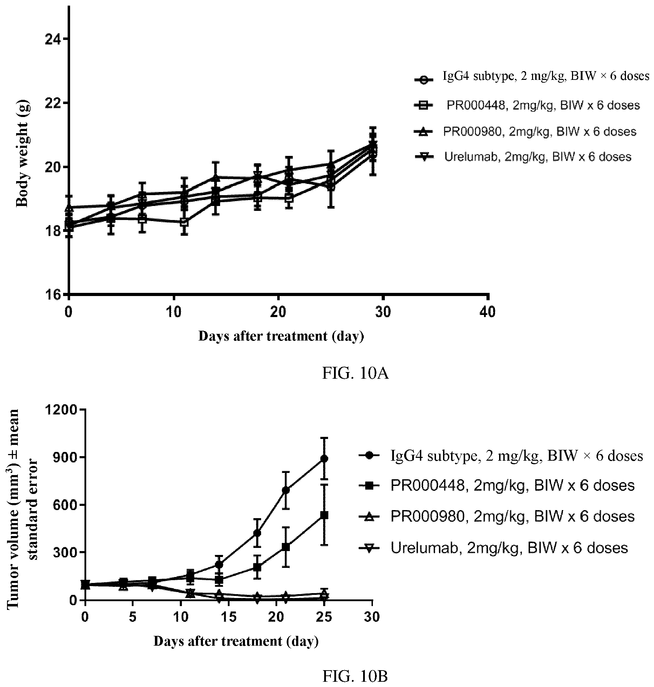

Changes in mouse body weight and tumor volume are shown in FIGs. 10A and 10B. Measurements were taken 29 days after the administration. The variant PR000980 of PR000448 with Fc mutant had greater inhibitory activity against tumor growth.

### Summary

The present application provides an anti-4-1BB antigen-binding protein having one or more of the following properties: 1) being capable of binding to human and monkey-derived 4-1BB proteins; 2) being capable of stimulating immune cells to secrete IFN-γ, IL2 and/or TNFα; 3) being capable of inhibiting tumor growth and/or tumor cell proliferation; and 4) being capable of activating the 4-1BB signaling pathway. The present application also provides use of the antigen-binding protein in the prevention and treatment of tumors.

### Example 2. Acquisition of Fully Human 4-1BB HCAB Antibodies

### Example 2.1. Immunization of fully human HCAb mice and production of 4-1BB antibodies

The Harbour HCAb mouse (Harbour Antibodies BV, WO 2002/085945 A3) is a transgenic mouse carrying an immune repertoire of human immunoglobulins, capable of producing novel "heavy chain"-only antibodies that are only half the size of conventional IgG antibodies. The antibodies produced have only human antibody "heavy chain" variable domains and mouse Fc constant domains. Due to the absence of light chain, this antibody almost solves the problems of light chain mismatch and heterodimerization, allowing the technical platform to develop products that are difficult to realize by the conventional antibody platform.

### Example 2.1.1. Immunization of HCAb mice

For 6-8 week-old Harbour human antibody transgenic mice, two immunization schemes were adopted to subject Harbour HCAb mice to multiple rounds of immunization. As immunization scheme 1, immunization was performed with recombinant human 4-1BB-ECD-Fc (ChemPartner, Shanghai) antigenic protein. In one immunization, each mouse was given a subcutaneous inguinal injection or intraperitoneal injection of 100 µL in total. In the first round of immunization, each mouse received the immunization with an immunogenic reagent prepared by mixing 50 µg of antigenic protein with complete Freund's adjuvant (Sigma, #F5881) in a 1:1 volume ratio. In each subsequent round of booster immunization, each mouse received an immunization with an immunogenic reagent prepared by mixing 25 µg of antigenic protein with Ribi adjunvant (Sigma Adjuvant System, Sigma, #S6322). As immunization scheme 2, immunization was performed with an NIH3T3-h4-1BB (ChemPartner, Shanghai) stable cell line overexpressing human 4-1BB. In one immunization, each mouse was given an intraperitoneal injection of 2×10⁶ cell suspension. The interval between rounds of booster immunization was at least two weeks. In general, there are no more than five rounds of booster immunizations. The immunization was performed at days 0, 14, 28, 42, 56 and 70; and the antibody titer in serum of mice was measured at days 49 and 77. The last round of booster immunization was performed at a dose of 25 µg of antigenic protein per mouse 5 days before the isolation of HCAb mouse splenic B cells.

### Example 2.1.2. Acquisition of anti-4-1BB HCAb antibodies

Mouse blood was collected, 10-fold diluted to obtain 6 concentrations (1:100, 1:1000, 1:10000, 1:100000 and 1:1000000), and subjected to an ELISA assay on an ELISA plate coated with human 4-1BB-ECD-Fc (Chempartner, Shanghai) for the titer of anti-human 4-1BB in the mouse blood. Two concentrations of the mouse blood (1:100 and 1:1000) were assayed by flow cytometry for the specific reactivity to CHO-K1/h4-1BB cells (Chempartner, Shanghai) highly expressing 4-1BB and CHO-K1 blast cells. Serum from pre-immunization mice was used as a blank control group (PB). When the titer of the 4-1BB-specific antibody in the serum of mice was detected to reach a certain level, spleen cells of the mice were taken, from which B cells were isolated, and the CD138-positive plasma cells and human 4-1BB antigen-positive B cell populations were sorted using a BD FACS Ariall cell sorter. The RNA of the B cells was extracted and reversely transcribed into cDNA (SuperScript IV First-Strand synthesis system, Invitrogen, 18091200), and human VH genes were amplified by PCR using specific primers. PCR primers were 5'-GGTGTCCAGTGTSAGGTGCAGCTG-3' and 5'-AATCCCTGGGCACTGAAGAGACGGTGACC-3'. The amplified VH gene fragments were constructed into a mammalian cell expression plasmid pCAG vectors encoding the sequence of the heavy chain Fc domain of the human IgG1 antibody.

Mammal host cells (e.g., human embryonic kidney cell HEK293) were transfected with the constructed plasmids and allowed to express HCAb antibodies. While the HCAb-expressing supernatant was tested for binding to a CHO-K1/hu 4-1BB (ChemPartner, Shanghai) stable cell line overexpressing human 4-1BB, Acumen screening was performed using CHO-K1 cells as a negative control. The positive monoclonal antibodies obtained were further tested for binding cross-binding activity to a CHO-K1/cyno 4-1BB (ChemPartner, Shanghai) stable cell line overexpressing cynomolgus monkey 4-1BB. While expression supernatant of 683 monoclones that bind to CHO-K1/hu 4-1BB and CHO-K1/cyno 4-1BB was obtained and subjected to NF-kb functional testing, the nucleotide sequences encoding the variable domains of the antibody molecules and the corresponding amino acid sequences were obtained using conventional sequencing means. After the removal of repeated sequences, 323 functional fully human 4-1BB monoclonal antibodies having unique sequences and binding to both CHO-K1/hu 4-1BB and CHO-K1/cyno 4-1BB were obtained. According to the ability to bind to human and monkey cells and the NF-Kb functional test results, top 38 antibodies in the overall rankings are selected for recombinant expression.

**Table 9. Germline gene analysis and post-translational modification site (PTM) analysis of sequences of HCAb antibodies**

| **No.** | **Clone No. 1** | **Recombinant antibody** | **VH germline V gene** | **VH PTM** |
|---|---|---|---|---|
| 1 | 1016P0010B11 | PR001758 | IGHV3-23 | |
| 2 | 1016P0010D7 | PR001759 | IGHV3-23 | NxS/T (HCDR3),NG (HCDR3) |
| 3 | 1016P0011G10 | PR001760 | IGHV3-23 | |
| 4 | 1016P0016E9 | PR001763 | IGHV3-23 | NxS/T (HCDR3),NG (HCDR3) |
| 5 | 1016P0022E5 | PR001764 | IGHV3-23 | |
| 6 | 1016P0034F10 | PR001766 | IGHV3-23 | |
| 7 | 1016P0038G1 | PR001767 | IGHV3-53 | DG (HCDR2) |
| 8 | 1016P0039B9 | PR001768 | IGHV3-23 | |
| 9 | 1016P0042C5 | PR001771 | IGHV3-23 | |
| 10 | 1016P0045F7 | PR001774 | IGHV3-23 | |
| 11 | 1016P0046A9 | PR001775 | IGHV3-23 | NxS/T (HFR3) |
| 12 | 1016P0049C7 | PR001776 | IGHV3-23 | DG (HCDR3) |
| 13 | 1016P0058D11 | PR001780 | IGHV3-23 | |
| 14 | 1016P007F3 | PR001781 | IGHV3-23 | |
| 15 | 1016P0014G8 | PR001830 | IGHV3-23 | |
| 16 | 1016P0015C8 | PR001831 | IGHV3-23 | NxS/T (HCDR3),NG (HCDR3) |
| 17 | 1016P0016F8 | PR001833 | IGHV3-23 | |
| 18 | 1016P0019C9 | PR001834 | IGHV3-23 | NxS/T (HCDR2) |
| 19 | 1016P0020G4 | PR001836 | IGHV3-23 | NxS/T (HCDR2),NG (HCDR2) |
| 20 | 1016P0024B10 | PR001837 | IGHV3-23 | |
| 21 | 1016P0030B2 | PR001838 | IGHV3-23 | DG (HCDR2) |
| 22 | 1016P0037D2 | PR001840 | IGHV3-74 | NS (HCDR2),DG (HCDR2) |
| 23 | 1016P0045A3 | PR001842 | IGHV3-23 | |
| 24 | R1016P142D03 | PR007286 | IGHV3-11 | |
| 25 | R1016P142F06 | PR007287 | IGHV3-21 | |
| 26 | R1016P144G12 | PR007288 | IGHV3-21 | |
| 27 | R1016P145B11 | PR007289 | IGHV3-21 | |
| 28 | R1016P145C05 | PR007290 | IGHV3-48 | |
| 29 | R1016P145C07 | PR007291 | IGHV3-11 | |
| 30 | R1016P145D10 | PR007292 | IGHV3-11 | |
| 31 | R1016P145E06 | PR007293 | IGHV3-21 | |
| 32 | R1016P147A07 | PR007294 | IGHV3-21 | |
| 33 | R1016P147A09 | PR007295 | IGHV3-11 | |
| 34 | R1016P147B10 | PR007296 | IGHV3-21 | |
| 35 | R1016P149F10 | PR007297 | IGHV3-11 | |
| 36 | R1016P149G05 | PR007298 | IGHV3-21 | |
| 37 | R1016P149H12 | PR007299 | IGHV3-21 | |
| 38 | R1016P156E05 | PR007300 | IGHV3-21 | |

New antibody molecules (referred to as PTM variants) were obtained by introducing amino acid mutations into one antibody with potential PTM sites from Example 2.1.2.

**Table 10. Mutation site designs for HCAb sequences**

| Initial antibody | Variant | Variable region mutations | Recombinant antibody subtype |
|---|---|---|---|
| PR001838 | PR004469 | G53A | Human IgG1 |
| PR001838 | PR007381 | F37V, P40A, E42G, T43K, K46E, G53A | Human IgG1 |

**Table 11. Sequence listing of HCAb antibodies**

| **Antibody No.** | **Heavy chain** | **VH** | **HCDR1** | **HCDR2** | **HCDR3** |
|---|---|---|---|---|---|
| PR001758 | 217 | 175 | 19 | 57 | 101 |
| PR001759 | 218 | 176 | 15 | 58 | 102 |
| PR001760 | 219 | 177 | 20 | 59 | 103 |
| PR001763 | 220 | 178 | 20 | 60 | 104 |
| PR001764 | 221 | 179 | 15 | 61 | 105 |
| PR001766 | 222 | 180 | 15 | 60 | 106 |
| PR001767 | 223 | 181 | 21 | 62 | 107 |
| PR001768 | 224 | 182 | 20 | 63 | 108 |
| PR001771 | 225 | 183 | 20 | 60 | 108 |
| PR001774 | 226 | 184 | 22 | 64 | 109 |
| PR001775 | 227 | 185 | 15 | 60 | 110 |
| PR001776 | 228 | 186 | 15 | 65 | 111 |
| PR001780 | 229 | 187 | 22 | 66 | 112 |
| PR001781 | 230 | 188 | 15 | 49 | 113 |
| PR001830 | 231 | 189 | 20 | 60 | 103 |
| PR001831 | 232 | 190 | 15 | 63 | 104 |
| PR001833 | 233 | 191 | 15 | 60 | 114 |
| PR001834 | 234 | 192 | 23 | 67 | 105 |
| PR001836 | 235 | 193 | 24 | 68 | 103 |
| PR001837 | 236 | 194 | 15 | 60 | 105 |
| PR001838 | 237 | 195 | 20 | 69 | 115 |
| PR001840 | 238 | 196 | 15 | 70 | 116 |
| PR001842 | 239 | 197 | 15 | 60 | 117 |
| PR004469 | 240 | 198 | 20 | 71 | 115 |
| PR007286 | 353 | 337 | 300 | 308 | 326 |
| PR007287 | 354 | 338 | 300 | 309 | 327 |
| PR007288 | 355 | 339 | 300 | 310 | 328 |
| PR007289 | 356 | 340 | 300 | 308 | 329 |
| PR007290 | 357 | 341 | 300 | 311 | 330 |
| PR007291 | 358 | 342 | 300 | 312 | 331 |
| PR007292 | 359 | 343 | 300 | 308 | 332 |
| PR007293 | 360 | 344 | 300 | 313 | 330 |
| PR007294 | 361 | 345 | 300 | 314 | 329 |
| PR007295 | 362 | 346 | 300 | 315 | 331 |
| PR007296 | 363 | 347 | 300 | 314 | 327 |
| PR007297 | 364 | 348 | 300 | 316 | 331 |
| PR007298 | 365 | 349 | 300 | 308 | 333 |
| PR007299 | 366 | 350 | 300 | 317 | 334 |
| PR007300 | 367 | 351 | 300 | 315 | 331 |
| PR007381 | 368 | 352 | 20 | 71 | 115 |

### Example 2.1.3. Preparation of anti-4-1BB fully human recombinant antibodies

Mammalian host cells (e.g., human embryonic kidney cell HEK293) were transfected with plasmids encoding HCAb antibodies, and purified anti-4-1BB recombinant heavy-chain antibodies could be obtained using conventional recombinant protein expression and purification techniques. Specifically, HEK293 cells were expanded in FreeStyle^{™} F17 Expression Medium (Thermo, A1383504). Before transient transfection, the cells were adjusted to a concentration of 6×10⁵ cells/mL, and cultured in a shaker at 37 °C with 8% CO₂ for 24 h at a concentration of 1.2×10⁶ cells/mL. 30 mL of the cultured cells were prepared, and 30 µg of the above plasmids encoding HCAb heavy chains was dissolved in 1.5 mL of Opti-MEM reduced serum medium (Thermo, #31985088). Then 120 µL of 1 mg/mL PEI (Polysciences, Inc, #23966-2) was dissolved in 1.5 mL of Opti-MEM, and the mixture was left to stand for 5 min. PEI was slowly added to the plasmids, and the mixture was incubated at room temperature for 10 min. The mixed solution of plasmids and PEI was slowly added dropwise while shaking the culture flask, and the cells were cultured in a shaker at 37 °C with 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect^{™} (GE Healthcare Life Science, #71-5020-91 AE) was equilibrated with PBS (pH 7.4) and rinsed with 2-5 column volumes of PBS. The supernatant sample was loaded onto a column. The column was rinsed with 5-10 column volumes of PBS. The target protein was eluted with 0.1 M glycine (pH 3.5). The eluate was adjusted to neutrality with Tris-HCl (pH 8.0), and concentrated and buffer exchanged into PBS buffer with an ultrafiltration tube (Millipore, UFC901024) to obtain a purified anti-4-1BB heavy-chain antibody solution. The antibody concentration was determined by measuring the absorbance at 280 nm using NanoDrop, and the antibody purity was determined by SEC-HPLC and SDS-PAGE.

### Example 2.1.4. Analysis of protein purity and polymers by HPLC-SEC

Analytical size-exclusion chromatography (SEC) was used to analyze the protein sample for purity and polymer form. An analytical chromatography column TSKgel G3000SWxl (Tosoh Bioscience, 08541, 5 µm, 7.8 mm × 30 cm) was connected to a high-pressure liquid chromatograph (HPLC, model: Agilent Technologies, Agilent 1260 Infinity II) and equilibrated with a PBS buffer at room temperature for at least 1 h. A proper amount of the protein sample (at least 10 µg, with the concentration adjusted to 1 mg/mL) was filtered through a 0.22 µm membrane filter and then injected into the system, and an HPLC program was set: the sample was passed through the chromatography column with PBS buffer (pH 7.4) at a flow rate of 1.0 mL/min for a maximum of 20 min. The detection wavelength was 280 nm. After being recorded, the chromatogram was integrated using ChemStation software and relevant data were calculated. An analysis was generated, with the retention time of the components with different molecular sizes in the sample reported.

### Example 2.1.5. Protein purity and hydrophobicity analysis by HPLC-HIC

Analytical hydrophobic interaction chromatography (HIC) was used to analyze the protein sample for purity and hydrophobicity. An analytical chromatography column TSKgel Butyl-NPR (Tosoh Bioscience, 14947, 4.6 mm × 3.5 cm) was connected to a high-pressure liquid chromatograph (HPLC, model: Agilent Technologies, Agilent 1260 Infinity II) and equilibrated with a PBS buffer at room temperature for at least 1 h. The method was set to a linear gradient from 100% mobile phase A (20 mM histidine, 1.8 M ammonium sulfate, pH 6.0) to 100% mobile phase B (20 mM histidine, pH 6.0) over 16 min. The flow rate was set at 0.7 mL/min. The protein sample concentration was 1 mg/mL. The injection volume was 20 µL. The detection wavelength was 280 nm. After being recorded, the chromatogram was integrated using ChemStation software and relevant data were calculated. An analysis was generated, with the retention time of the components with different molecular sizes in the sample reported.

### Example 2.1.6. Determination of thermostability of antibody molecules using DSF

Differential scanning fluorimetry (DSF) is a commonly used high-throughput method for determining the thermostability of proteins. In this method, changes in the fluorescence intensity of the dye that binds to unfolded protein molecules were monitored using a real-time quantitative fluorescence PCR instrument to reflect the denaturation process of the protein and thus to reflect the thermostability of the protein. In this example, the thermal denaturation temperature (Tm) of a protein molecule was measured by DSF. 10 µg of protein was added to a 96-well PCR plate (Thermo, AB-0700/W), followed by the addition of 2 µL of 100× diluted dye SYPROTM (Invitrogen, 2008138), and then the mixture in each well was brought to a final volume of 40 µL by adding buffer. The PCR plate was sealed, incubated in a real-time quantitative fluorescence PCR instrument (Bio-Rad CFX96 PCR System) at 25 °C for 5 min, then gradually warmed from 25 °C to 95 °C at a gradient of 0.2 °C/0.2 min, and cooled to 25 °C at the end of the test. The FRET scanning mode was used and data analysis was performed using Bio-Rad CFX Maestro software to calculate the Tm of the sample.

**Table 12. Physicochemical properties of anti-4-IBB HCAB antibodies**

| **Antigen-binding protein** | **SEC-HPLC (%) Purity** | **Tm (°C) by DSF*** | **HIC-HPLC retention time (min)** | **Corresponding ammonium sulfate concentration (M)** | **Yield (mg/L) after first purification** |
|---|---|---|---|---|---|
| PR001758 | 99.45 | 57 | 18.617 | 0.49 | 75.3 |
| PR001759 | 84.47 | 61.2 | 17.846 | 0.58 | 58.3 |
| PR001760 | 98.93 | 62.4 | 17.993 | 0.56 | 42 |
| PR001763 | 85.12 | 62.6 | 17.035 | 0.67 | 69.5 |
| PR001764 | 98.86 | 57.2 | 19.335 | 0.41 | 35.5 |
| PR001766 | 97.69 | 60.6 | 18.7 | 0.48 | 27.5 |
| PR001767 | 98.76 | 56 | 18.591 | 0.5 | 36.3 |
| PR001768 | 99.01 | 63 | 20.555 | 0.28 | 25 |
| PR001771 | 99.28 | 63.4 | 20.21 | 0.31 | 17.5 |
| PR001774 | 99.79 | 56.2 | 20.378 | 0.29 | 11.4 |
| PR001775 | 95.71 | 55.2 | 18.398 | 0.52 | 17 |
| PR001776 | 99.1 | 56.6 | 20.352 | 0.3 | 10 |
| PR001780 | 99.37 | 60.8 | 18.893 | 0.46 | 31 |
| PR001781 | 99.59 | 61.4 | 20.564 | 0.27 | 11.7 |
| PR001830 | 97.48 | 63.8 | 17.782 | 0.59 | 36.8 |
| PR001831 | 86.78 | 56.2 | 16.948 | 0.68 | 46.5 |
| PR001833 | 99.29 | 58.2 | 17.6 | 0.61 | 42 |
| PR001834 | 93.7 | 59 | 17.711 | 0.6 | 27.3 |
| PR001836 | 99.48 | 57.4 | 17.684 | 0.6 | 38.3 |
| PR001837 | 96.57 | 59.8 | 18.699 | 0.48 | 35.3 |
| PR001838 | 98.55 | 63.8 | 16.785 | 0.7 | 42 |
| PR001840 | 99.6 | 56.2 | 17.433 | 0.63 | 44.3 |
| PR001842 | 98.47 | 55 | 18.224 | 0.54 | 16.5 |
| PR004469 | 95.43 | - | - | - | 81.2 |
| PR007286 | - | 56.2 | 20.4 | 0.31 | 10.68 |
| PR007287 | - | 52.2 | 16.65 | 0.73 | 8.18 |
| PR007288 | - | - | - | - | 2.46 |
| PR007289 | - | 55.4 | 19.41 | 0.42 | 1.21 |
| PR007290 | - | 51.2 | 20.03 | 0.35 | 6.81 |
| PR007291 | - | 44.4 | 20.88 | 0.25 | 5.95 |
| PR007292 | - | 50.6 | 16.04 | 0.79 | 8.90 |
| PR007293 | - | - | - | - | 4.01 |
| PR007294 | - | - | - | - | 1.81 |
| PR007295 | - | 64 | 20.33 | 0.32 | 7.65 |
| PR007296 | - | 58.2 | 18.89 | 0.48 | 1.12 |
| PR007297 | - | 56.8 | 21.81 | 0.15 | 5.48 |
| PR007298 | - | 50.4 | 16.22 | 0.77 | 10.68 |
| PR007299 | - | 47.8 | 17.93 | 0.58 | 8.18 |
| PR007300 | - | 50.4 | 20.75 | 0.27 | 2.46 |
| PR007381 | 98.52% | - | - | - | 152.5 |

Table 12 shows that most 4-1BB HCABs had good physicochemical properties.

### Example 2.2. Binding of 4-1BB HCAb antibodies to 4-1BB on cell surfaces

This example is intended to investigate the *in vitro* binding activity of anti-human 4-1BB HCAb monoclonal antibodies to human and cynomolgus monkey 4-1BB. Antibody binding experiments at the cellular level were conducted using a CHO-K1 cell strain overexpressing human 4-1BB (CHO-K1/hu 4-1BB, Genescript) and a CHO-K1 cell strain overexpressing cynomolgus monkey 4-1BB (CHO-K1/cyno 4-1BB, Genescript). Briefly, the CHO-K1/hu 4-1BB and CHO-K1/cyno 4-1BB cells were digested and resuspended in F12K complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor 488-conjugated AffiniPure Goat Anti-Human IgG, Fcy Fragment Specific, Jackson, #109-545-06, 1:500 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII.

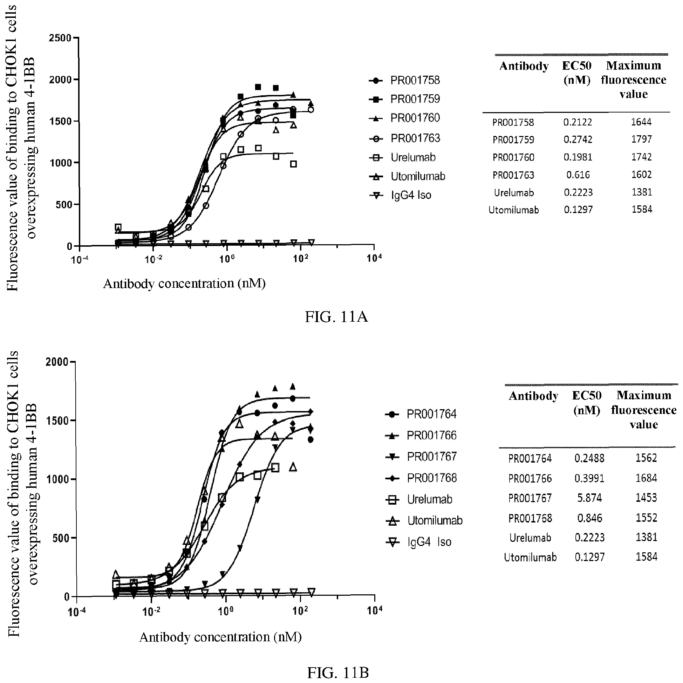

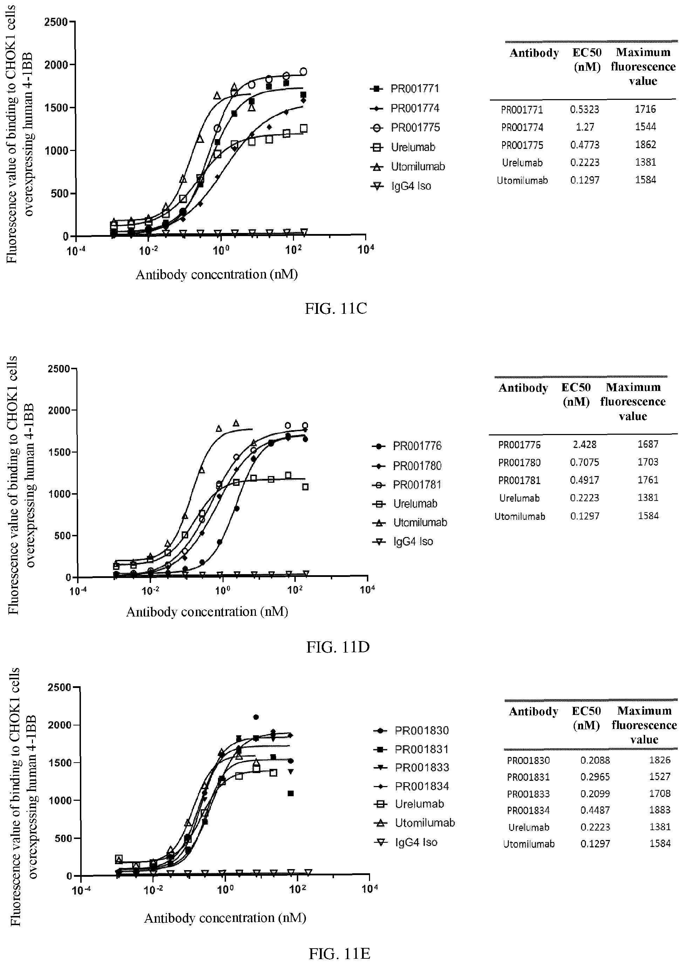

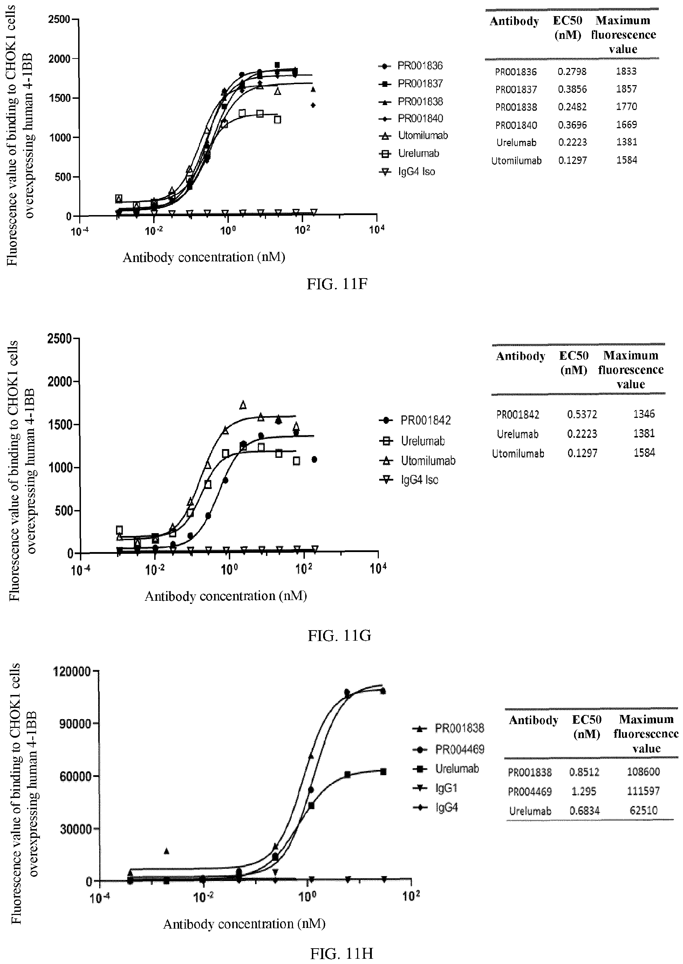

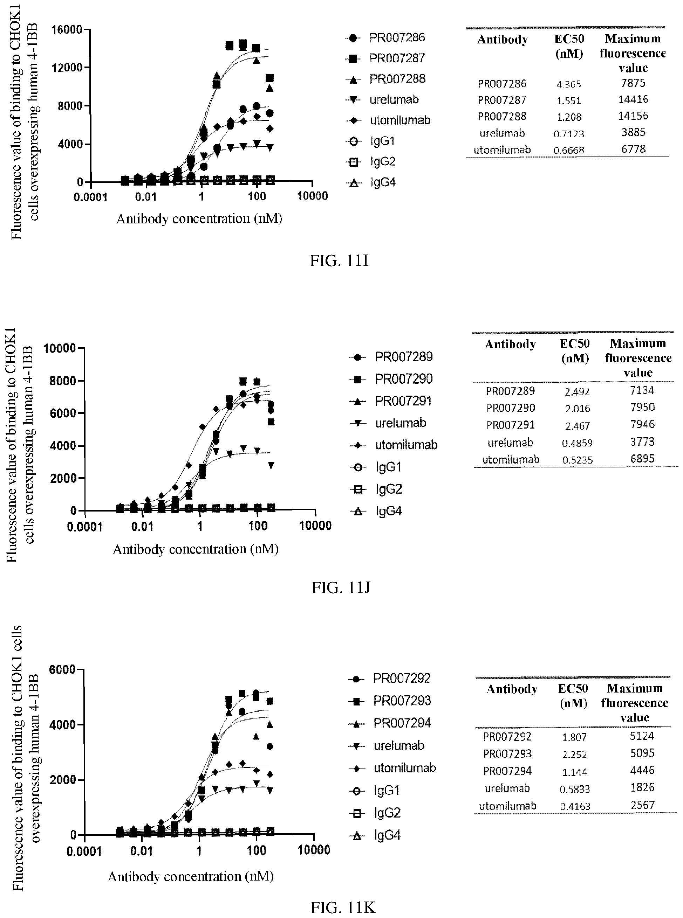

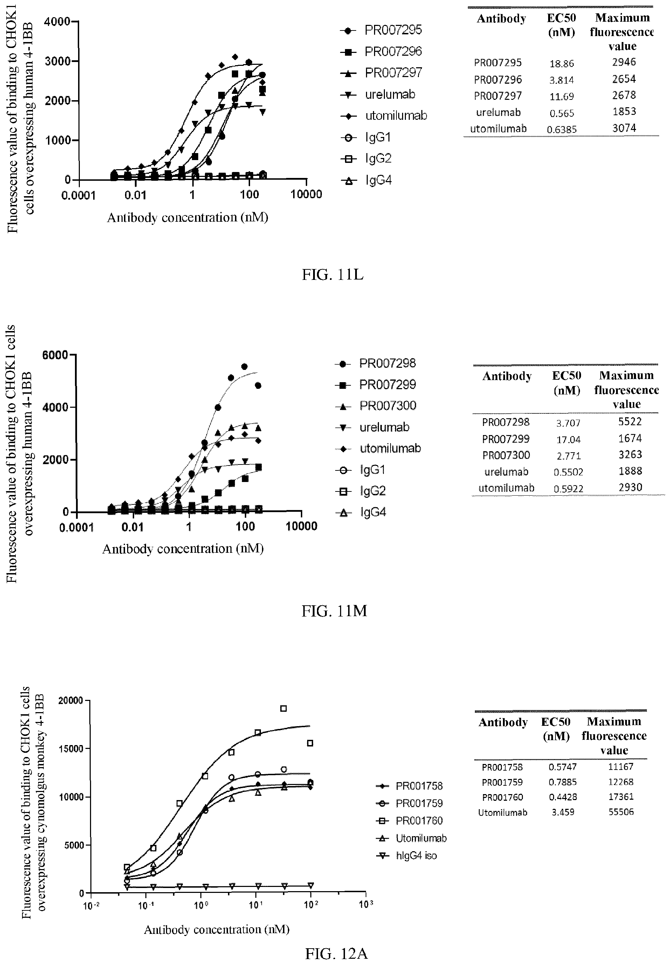

As shown in FIGs. 11A-11M, the anti-4-1BB HCAb antibodies of the present invention could all bind to human 4-1BB, and the binding abilities of the antibodies determined increased with the antibody concentrations in a positive correlation. These antibodies could more sensitively bind to human 4-1BB at lower concentrations than the control antibodies (Urelumab and Utomilumab). Among them, PR001758-PR001760, PR001764, PR001830, PR001831, PR001833, PR001836 and PR001838 were the best; their EC₅₀ values were all less than 0.3 nM, comparable to those of Urelumab and Utomilumab. The maximum fluorescence values of PR007287, PR007292, PR007293, PR007294 and PR007298 were higher than those of the control antibodies Utomilumab and Urelumab in binding to CHO-K1/hu 4-1BB cells.

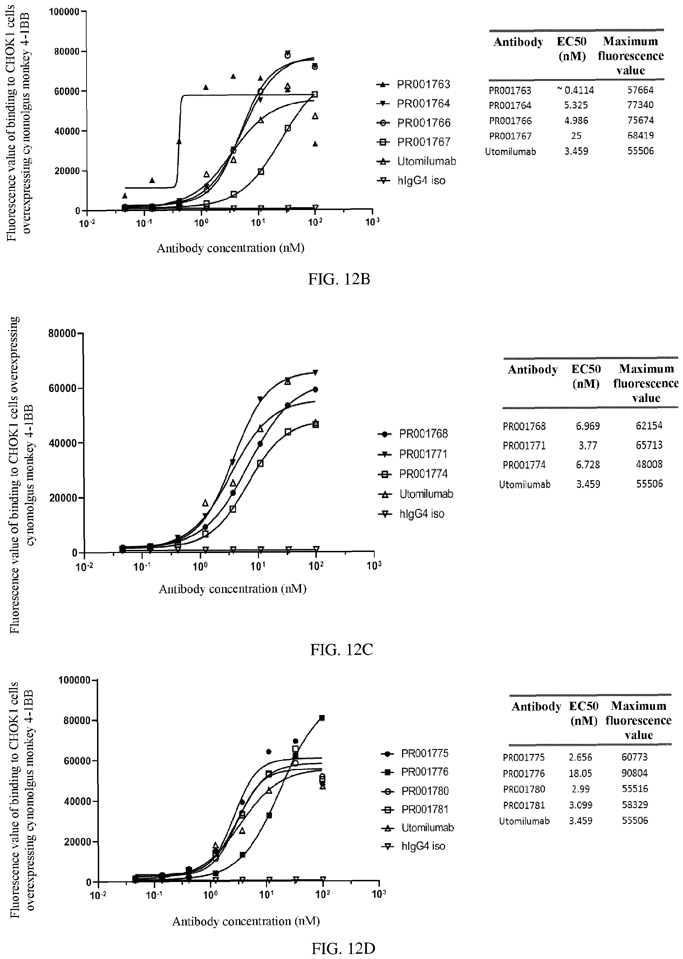

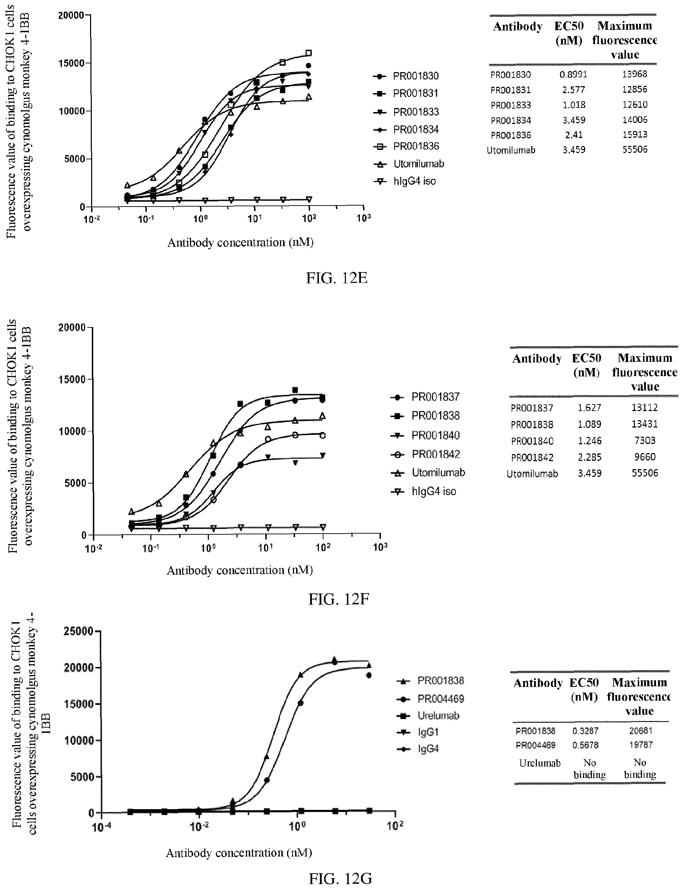

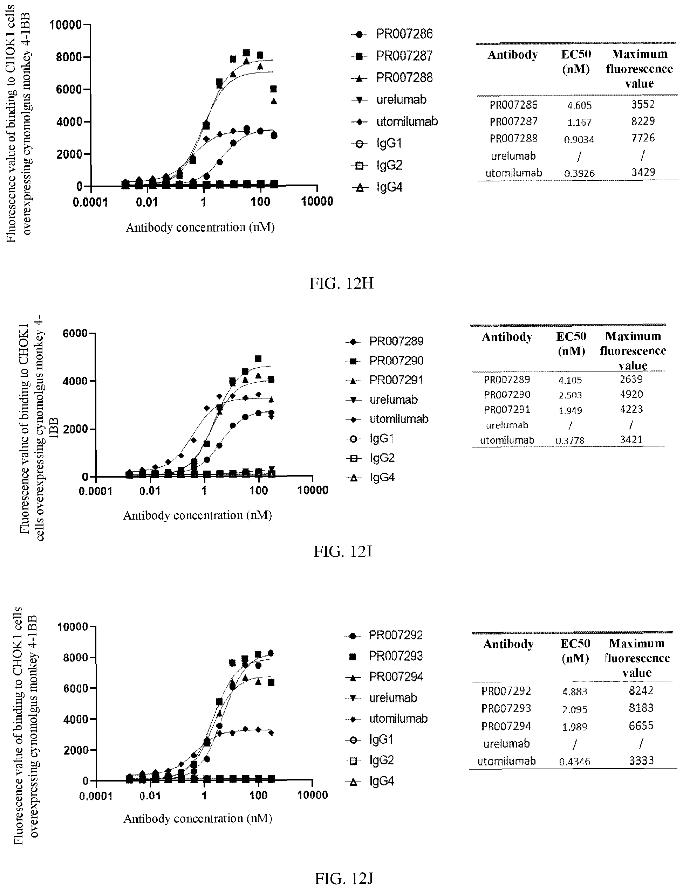

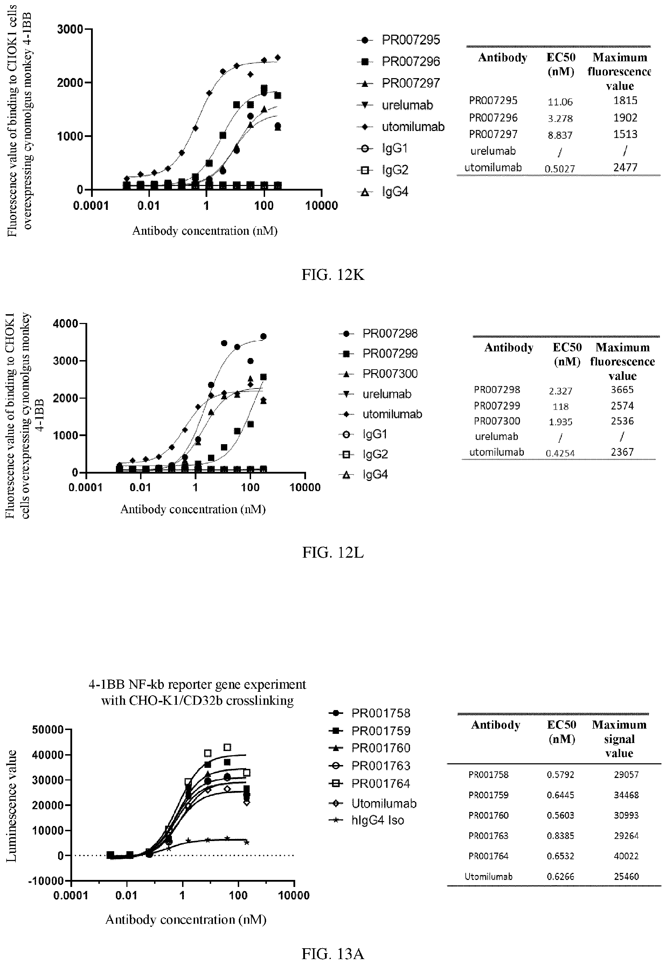

As shown in FIGs. 12A-12L, the anti-4-1BB HCAb antibodies of the present invention could all bind to monkey 4-1BB, and the binding abilities of the antibodies determined increased with the antibody concentrations in a positive correlation. These antibodies could more sensitively bind to monkey 4-1BB at lower concentrations than the control antibody Tab (Utomilumab), with EC₅₀ values comparable to or better than that of Utomilumab. The maximum fluorescence values of PR007287, PR007292, PR007293, PR007294 and PR007298 were higher than those of the control antibodies Utomilumab and Urelumab in binding to CHO-K1/hu 4-1BB cells. However, the control antibody Urelumab had no cross-binding activity to monkey 4-1BB.

### Example 2.3. Detection of stimulation of 4-1BB signaling pathway using reporter gene cell line

CD32b-expressing CHO-K1 cells (CHO-K1/CD32b) were plated on a 96-well plate (Perkin Elmer, #6005225) at 1.5×10⁴/well, 100 µL/well. The cells were incubated at 37 °C with 5% CO₂ overnight. The supernatant was removed, and a 2-fold dilution of the test antigen-binding protein was added at 40 µL/well. The initial final concentration was 200 nM, and 3-fold dilution was performed; or the initial final concentration was 30 nM, and 5-fold dilution was performed. hlgG1 was used as a control group. HEK293 reporter cells capable of constantly expressing the luciferase reporter genes of 4-1BB and NF-Kb response elements (HEK293/4-1BB/NF-kb reporter cells, BPS Biosciences, #79289) were added at 4.5×10⁴/well, 40 µL/well. The cells were incubated at 37 °C with 5% CO₂ for 6 h. ONE-Glo^{™} luciferase reagent (Promega, #E6110) was added. The cells were incubated at room temperature for 5 min, and the luminescence values were measured using a microplate reader.

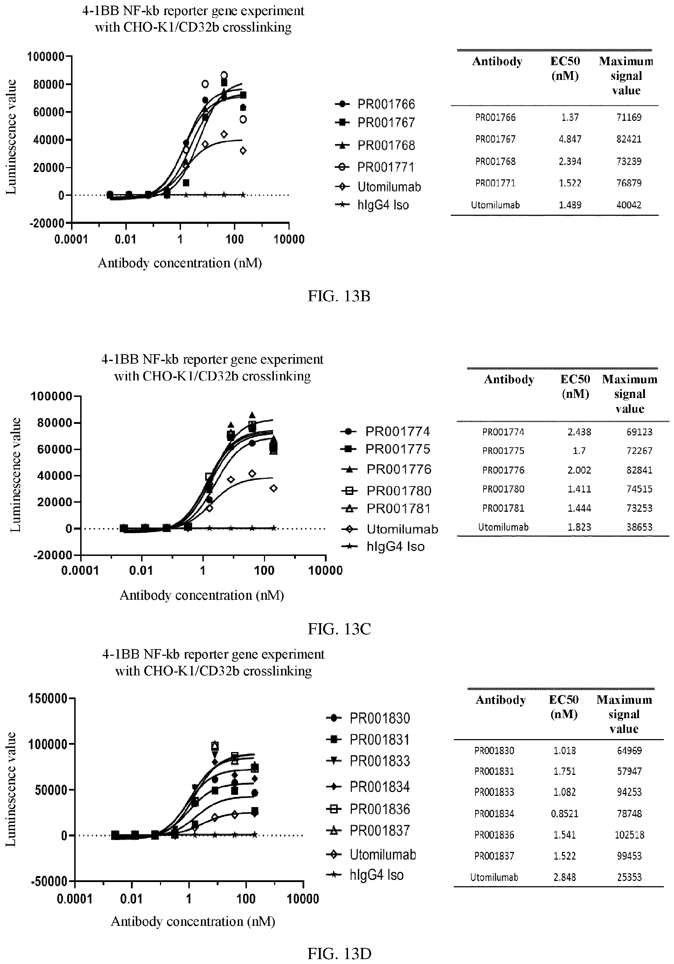

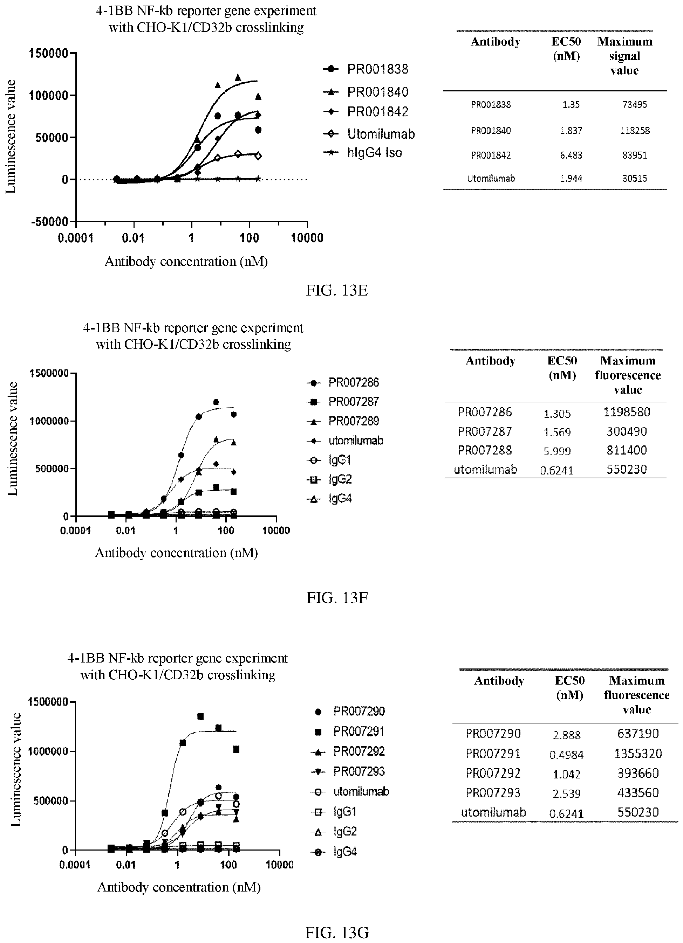

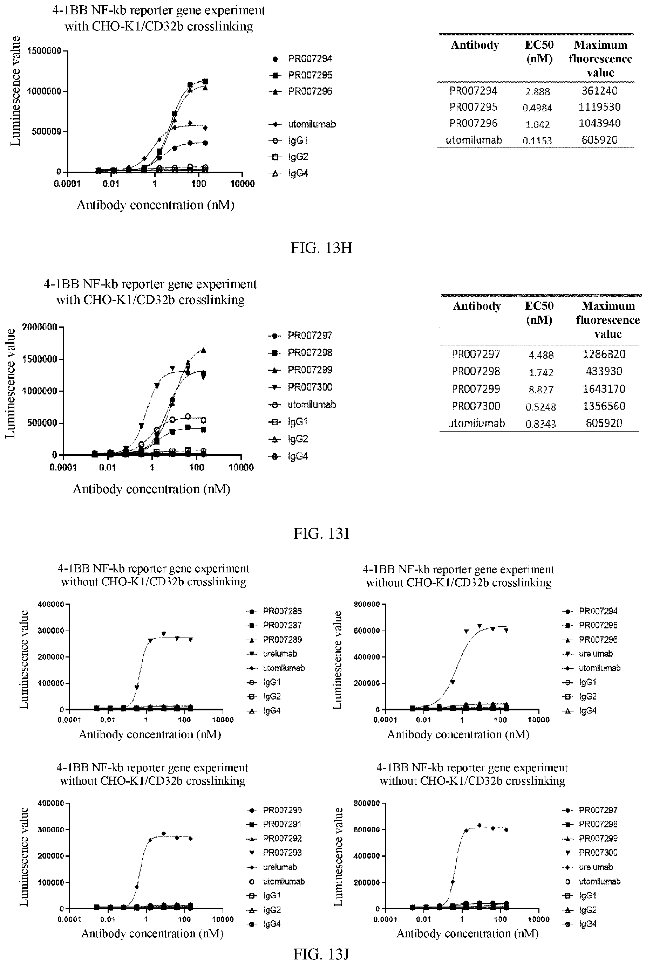

As shown in FIGs. 13A-13I, under CHO-K1/CD32b crosslinking, the promotion of the 4-1BB-mediated NF-Kb signaling pathway by most of the 4-1BB antigen-binding proteins of the present application increased with their concentrations in a positive correlation. Their EC₅₀ values were comparable to or lower than that of the control antibody Tab (Utomilumab), and their maximum fluorescence values were comparable to or higher than that of the control antibody, which indicate that these antibodies could promote NF-Kb activation at lower concentrations. Among them, PR001758, PR001759 and PR001760, PR007286, PR007291, PR007295, PR007296, PR007297, PR007299 and PR007300 were the best; their EC₅₀ values were all less than 0.8 nM, comparable to that of the control antibody.

As shown in FIG. 13J, in the absence of CHO-K1/CD32b crosslinking, all of the 4-1BB antigen-binding proteins of the present application did not activate the 4-1BB-mediated NF-Kb signaling pathway, while the control antibody Tab (Urelumab) still had a strong activating effect.

### Example 2.4. Antigen-binding proteins' blocking binding of 4-1BB ligand to 4-1BB

To investigate the *in vitro* activity of the human 4-1BB-binding protein in blocking the binding of human 4-1BB to human 4-1BBL, a human 4-1BB/human 4-1BBL binding blocking experiment was conducted at the cellular level using a CHO-K1 cell strain overexpressing human 4-1BB (CHO-K1/hu4-1BB). Briefly, the CHO-K1/hu4-1BB cells were digested and resuspended in F-12K complete medium, and the cell density was adjusted to 1×10⁶ cells/mL. The cells were seeded in a 96-well V-bottom plate (Corning, #3894) at 100 µL/well, followed by the addition of the test antigen-binding proteins serially diluted 3-fold with concentrations that were twice the final concentrations at 100 µL/well. The mixtures were well mixed. The highest final concentration of the antigen-binding proteins was 100 nM. A total of 8 concentrations were set. hIgG1 was used as a control. The cells were incubated at 4 °C for 1 h away from light. Thereafter, centrifugation was carried out at 4 °C for 5 min, and the supernatant was discarded, followed by the addition of a biotin-labeled human 4-1BBL protein (ACRO, 41L-H82F9) with a concentration of 1 µg/mL at 50 µL/well. The cells were incubated at 4 °C away from light for 30 min. The cells in each well were rinsed twice with 100 µL of pre-cooled PBS, and centrifuged at 500 g at 4 °C for 5 min, and then the supernatant was discarded. A fluorescent secondary antibody (PE Streptavidin, BD, #554061, 1:200) was added at 100 µL/well, and the cells were incubated at 4 °C away from light for 30 min. The cells in each well were rinsed twice with 200 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII. The IC₅₀ was calculated. Inhibition% = 1 - MFI_{(4-1BB Ab)}/MFI₍ᵢₛₒ₎.

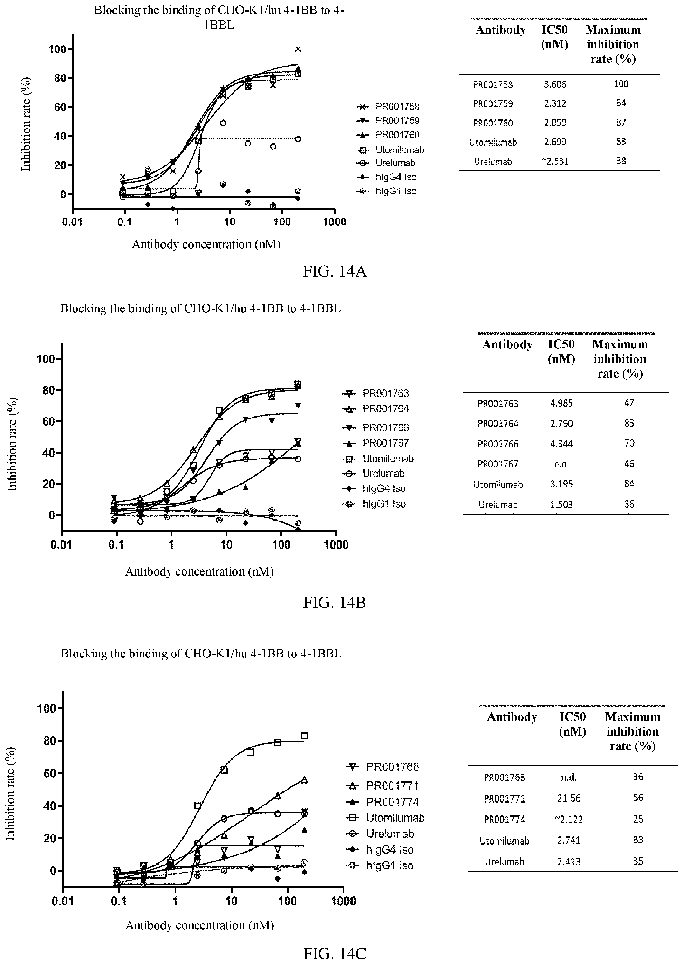

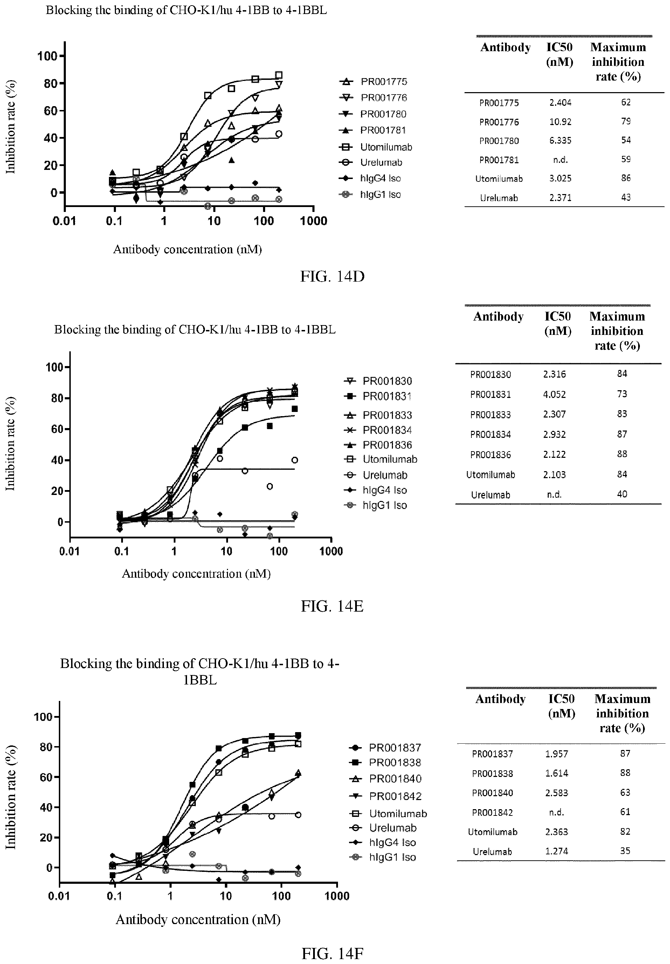

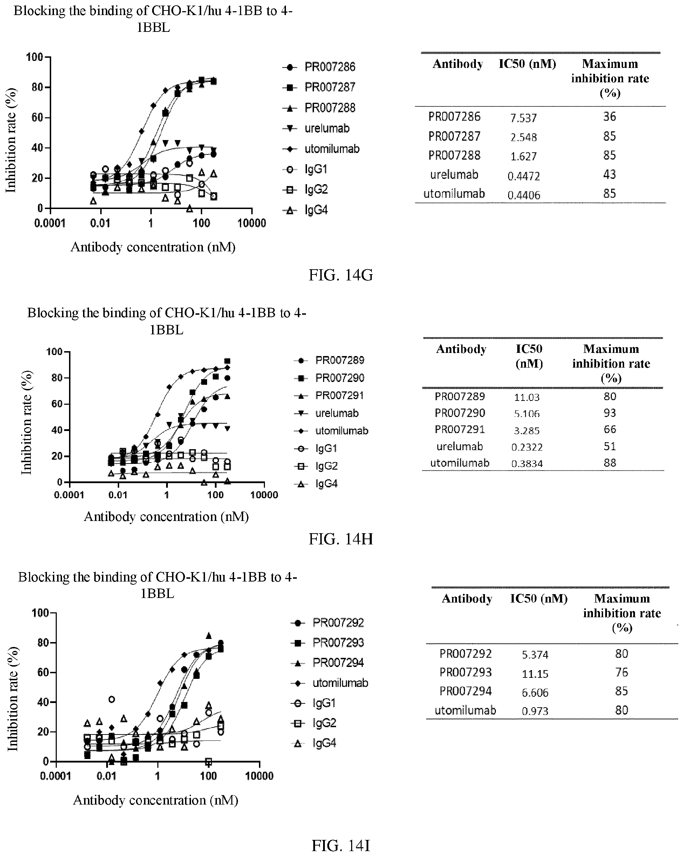

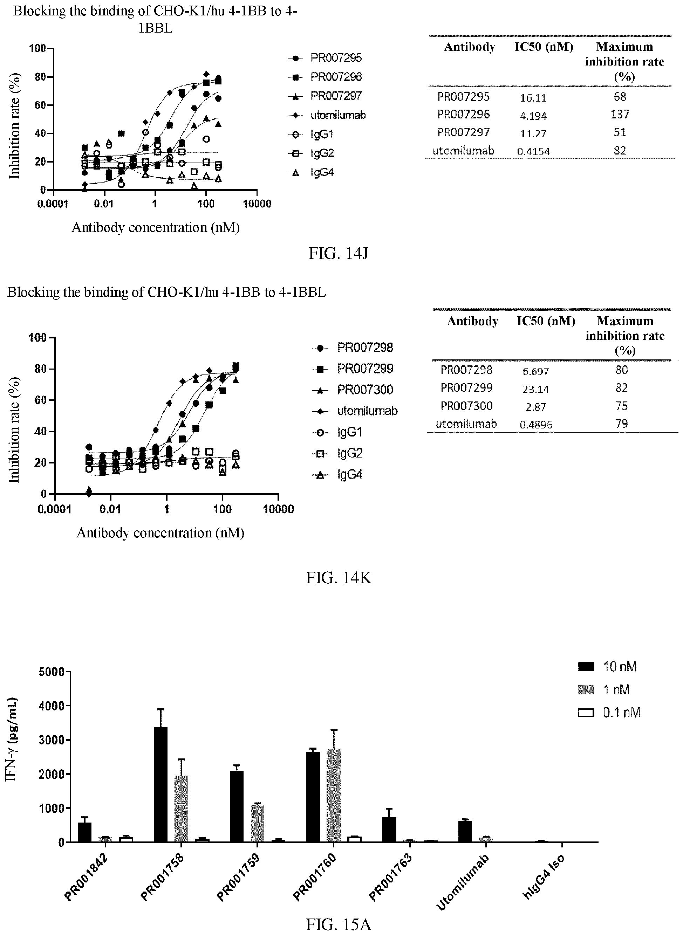

The results are shown in FIGs. 14A-14K. The 4-1BB antigen-binding proteins of the present application fall into two categories, of which one (PR001758, PR001759, PR001760, PR001764, PR001766, PR001776, PR001830, PR001831, PR001833, PR001834, PR001836, PR001837, PR001838, PR007287, PR007288, PR007289, PR007290, PR007291, PR007292, PR007293, PR007294, PR007295, PR007296, PR007298, PR007299 and PR007300) could block the binding of human 4-1BBL to human 4-1BB on cell surfaces and produce blocking effects comparable to that of the control antibody Tab (Utomilumab), and the other (PR001763, PR001767, PR001768, PR001771, PR001774, PR001780, PR001781, PR001840, PR001842, PR007286, PR007297), similar to the control antibody (Urelumab), produced weak blocking effects or no blocking effects.

### Example 2.5. Antigen-binding proteins can activate the 4-1BB pathway in vitro

CHO-K1 (ATCC, #CCL-61) or CHO-K1/CD32b (human CD32b-overexpressing CHO-K1 cells) cells were treated with 10 µg/mL mitomycin (Beijing Ruitaibio, #10107409001) at 37 °C for 30 min, then washed 4 times with F-12K culture medium containing 10% FBS. The treated cells were placed in a 96-well plate at 1.5×10⁴ cells/well and incubated in an incubator at 37 °C overnight. The next day, human CD3 positive T cells were isolated from human PBMCs using an MACS kit (Miltenyi Biotec, #130-096-535). The number of the cells was determined firstly, and then corresponding amounts of MACS buffer and Pan-T cell biotin antibody were added according to the number of the cells. The mixture was well mixed and left to stand at 4 °C for 5 min. Then, a corresponding amount of magnetic microbeads was added, and the mixture was left to stand at 4 °C for 10 min. What passed through the LS column were CD3 positive T cells. The previous day's culture medium was washed off the 96-well plate, and purified T cells were added at 1×10⁵ cells/well. Then a 4-1BB antibody or a control antibody was added at a corresponding concentration, and OKT3 (eBiosciences, #16-0037-85) was added at a final concentration of 0.3 µg/mL. The wells were cultured in an incubator at 37 °C for 72 h. After 72 hours, the supernatant was collected and assayed for IFN-γ content using an ELISA kit (Invitrogen, #88-7316-88). A coating antibody was added to a 96-well flat-bottom plate, and the plate was incubated at 4 °C overnight. The next day, ELISA buffer was added, and the plate was incubated at room temperature for 1 h. The collected supernatant was added, and the plated was incubated at room temperature for 2 h. The plate was washed twice, and test antibodies were added. The plate was incubated at room temperature for 1 h. The plate was washed twice, and HRP-streptavidin was added. The plate was incubated at room temperature for 1 h. Then TMB substrate was added, and ELISA stop buffer (BBI, E661006-0200) was added later. The absorbance value at 450 nm (OD450) was read using a microplate reader (PerkinElemer Enspire), and the IFN-γ concentration was calculated.

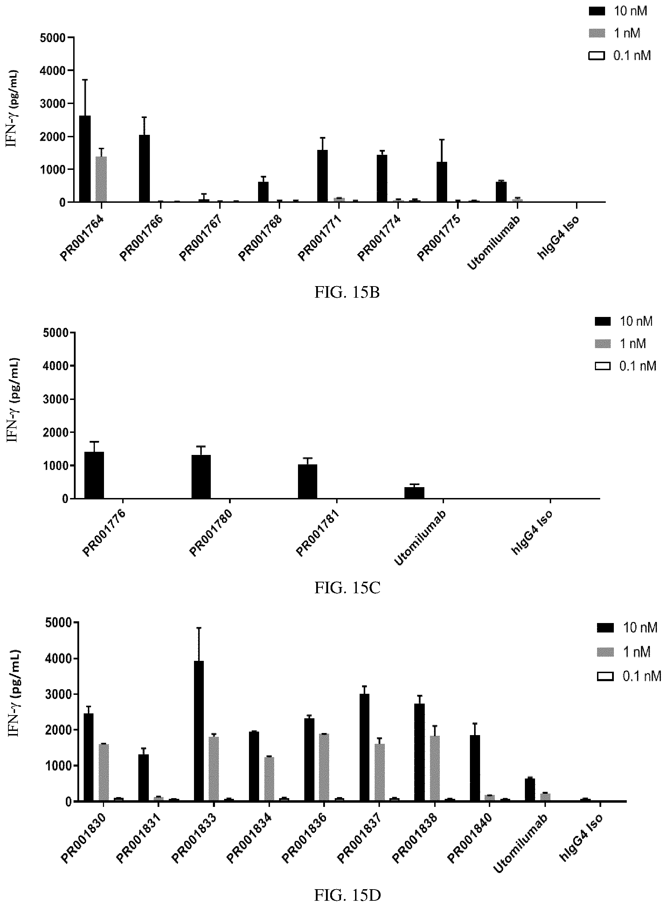

As shown in FIGs. 15A-15D, the majority of the 4-1BB HCABs in this example had stronger activating effects than Utomilumab; for example, PR001758, PR001759, PR001760, PR001764, PR001830, PR001833, PR001834, PR001836, PR001837 and PR001838 showed greater abilities to activate T cells than the control antibody Utomilumab at a low concentration of 1 nM.

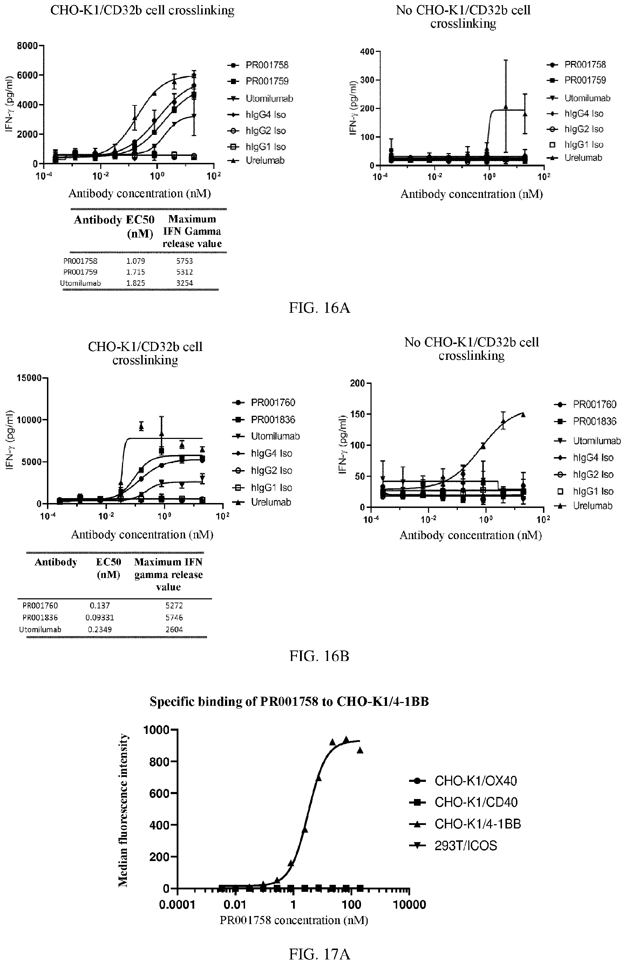

As shown in FIGs. 16A-16B, antibodies PR0001758, PR001759 and PR001760 all activated the 4-1BB pathway in a CD32b crosslinking-dependent manner and induced activation of T cell functions. In the presence of CHO-K1/CD32b cell crosslinking, their activating effects were stronger than that of Utomilumab and slightly weaker than that of Urelumab. In the absence of CHO-K1/CD32b cell crosslinking, the HCAb antibodies in this example could not activate T cell functions, while Urelumab was still able to activate T cells, which is considered the reason for Urelumab toxicity. The activation increased with the antibody concentration in a positive correlation. Their EC₅₀ values were all less than that of the control antibody Utomilumab, which indicates that they were able to activate the 4-1BB pathway at lower concentrations. In addition, their maximum cytokine interferon gamma release levels were all higher than that of Utomilumab.

### Example 2.6. Assay for binding affinity of 4-1BB antibodies for recombinant 4-1BB protein

Affinity was determined using an Octet RED96 instrument (Fortiebio) and an anti-human IgG Fc avidin sensor (AHC sensor, Pall ForteBio, #18-5060) according to the detailed procedures and methods provided by the manufacturer. Specifically, human 4-1BB protein and his tag (Acrobiosystem, #41B-H5227), or cynomolgus monkey 4-1BB protein and his tag (Acrobiosystem, #41B-C52H4), were diluted to 400 nM with PBS buffer (pH 7.4) containing 0.1% (w/w) BSA and 0.02% (v/v) Tween 20, and incubated with the AHC sensor. A 40 nM 4-1BB antibody was incubated with the AHC sensor loaded with human 4-1BB protein or monkey 4-1BB protein at 30 °C for 3 min. The reaction mixture was incubated in PBS buffer (pH 7.4) containing 0.1% (v/w) BSA and 0.02% (v/v) Tween 20 at 30 °C for another 5 min. The binding and separation signals between the 4-1BB antibody and 4-1BB protein were recorded by Octet Red 96 in real time. The affinity and association and dissociation constants were determined by Octet using software. The results are shown in Tables 13 and 14.

The results show that the KD values of the antibodies PR001836 and PR001838 among the antibodies tested were slightly low in binding to either human 4-1BB or cynomolgus monkey 4-1BB, which indicates their greater 4-1BB binding affinities.

**Table 13. Binding affinities of antibodies for human 4-1BB protein**

| **Antibody** | **Antigenic protein** | **Antibody concentration (nM)** | **KD (M)** | **kon(1/Ms)** | **kdis(1/s)** | **Response** |
|---|---|---|---|---|---|---|
| PR001758 | Human 4-1BB | 400 | 2.56E-08 | 2.26E+05 | 5.78E-03 | 0.3263 |
| PR001759 | | 400 | 1.23E-08 | 3.17E+05 | 3.89E-03 | 0.2843 |
| PR001760 | | 400 | 2.26E-08 | 2.68E+05 | 6.07E-03 | 0.2674 |
| PR001833 | | 400 | 2.34E-08 | 2.16E+05 | 5.06E-03 | 0.3112 |
| PR001830 | | 400 | 2.49E-08 | 2.66E+05 | 6.64E-03 | 0.3073 |
| PR001836 | | 400 | 1.35E-08 | 2.39E+05 | 3.22E-03 | 0.2937 |
| PR001838 | | 400 | 1.12E-08 | 2.33E+05 | 2.61E-03 | 0.3093 |
| PR001840 | | 400 | 3.27E-08 | 1.07E+05 | 3.50E-03 | 0.2236 |

**Table 14. Binding affinities of antibodies for cynomolgus monkey 4-1BB protein**

| **Antibody** | **Antigenic protein** | **Antibody concentration (nM)** | **KD (M)** | **kon(1/Ms)** | **kdis(1/s)** | **Response** |
|---|---|---|---|---|---|---|
| PR001758 | Cynomolgus monkey 4-1BB | 400 | 7.41E-09 | 1.51E+05 | 1.12E-03 | 0.274 |
| PR001759 | | 400 | 1.10E-08 | 2.15E+05 | 2.37E-03 | 0.2832 |
| PR001760 | | 400 | 2.06E-08 | 1.56E+05 | 3.21E-03 | 0.1779 |
| PR001833 | | 400 | 2.23E-08 | 1.39E+05 | 3.09E-03 | 0.2733 |
| PR001830 | | 400 | 1.81E-08 | 1.92E+05 | 3.47E-03 | 0.2222 |
| PR001836 | | 400 | 8.89E-09 | 1.65E+05 | 1.47E-03 | 0.2504 |
| PR001838 | | 400 | 1.03E-08 | 1.43E+05 | 1.46E-03 | 0.2242 |
| PR001840 | | 400 | 1.63E-08 | 6.20E+04 | 1.01E-03 | 0.2028 |

### Example 2.7. Epitope identification (epitope binning) of antigen-binding proteins

The antigen-binding proteins obtained in Example 1 , Utomilumab and Urelumab were subjected to epitope identification on the ForteBio Octet platform. Briefly, a primary antibody was loaded onto multiple AHC tips, and a 60-second baseline was obtained in pH 7.5 assay buffer. Then the tips were exposed to histidine-labeled 4-1BB for 180 s to allow antigen binding. The tips were transferred to a well containing a secondary antibody in assay buffer for 90 s. If the secondary antibody exhibited significant binding, it was considered a non-competitor (i.e., in an epitope interval different from that of the primary antibody). If the secondary antibody exhibited no significant binding, it was considered a competitor (i.e., in the same epitope interval as the primary antibody). The binding assay was performed by comparing the binding of the secondary antibody to 4-1BB in the presence of the primary antibody to the blocking of the primary antibody itself. The inhibition rate was calculated through the following formula: inhibition (%) = (A - B ) / A × 100 (note: A: the 100% signal of an antibody, and B: the signal for the antibody as the secondary antibody).

If the inhibition rate was greater than 80%, it meant that the two antibodies had very close epitopes; if the inhibition rate was between 40% and 80%, it meant that the two antibodies had epitopes that were relatively close but not completely overlapped; if the inhibition rate was less than 40%, it meant that the two antibodies had non-overlapping epitopes.

As shown in Table 15 below, the binding sites on 4-1BB of PR001760, PR001779, PR001838 and PR001840 overlapped with those of Utomilumab to a high degree but with those of Urelumab to a low degree, whereas the binding sites of PR001767 were specific, different from those of both Utomilumab and Urelumab.

**Table 15. Epitope identification of the antigen-binding proteins of the present application and clinical antibodies**

| Inhibition rate (%) | | Secondary antibody | | | | | | |
|---|---|---|---|---|---|---|---|---|
| | | Utomilumab | Urelumab | PR001760 | PR001767 | PR001779 | PR001838 | PR001840 |
| Primary antibody | Utomilumab | 93.31 | -3.7 | 101.04 | 6.51 | 96.77 | 98.5 | 94.94 |
| | Urelumab | -1.65 | 96.35 | 2.35 | 26.35 | 8.94 | 2.11 | -7.98 |
| | PR001760 | 78.72 | -0.09 | 91.61 | 15.34 | 91.78 | 89.07 | 84.56 |
| | PR001767 | -9.54 | 2.12 | -4.97 | 88.06 | -9.85 | 1.14 | 49.51 |
| | PR001779 | 42.12 | 0.52 | 65.34 | 9.28 | 89.03 | 63.36 | 69.98 |
| | PR001838 | 83.94 | -2.67 | 92.53 | 13.43 | 84.72 | 92.26 | 84.31 |
| | PR001840 | 53.97 | 1.04 | 79.78 | 65.13 | 90.58 | 78.42 | 94.97 |

### Example 2.8. Specific binding of antigen-binding proteins to 4-1BB

4-1BB belongs to the TNF tumor necrosis factor receptor superfamily, which consists of a large group of multifunctional receptors that mediate immune and non-immune cells. Six receptors including CD40, OX40, 4-1BB, CD27, GITR and CD30 have been identified as important immune co-stimulators. Similarly, the inducible T cell co-stimulatory factor (ICOS) is another receptor that plays a critical role in the function and survival of activated T cells or memory T cells.

This example is to investigate the specificity of *in vitro* binding of anti-human 4-1BB HCAb monoclonal antibodies by detecting 3 receptors of the TNF tumor necrosis factor receptor superfamily and ICOS using flow cytometry. Antibody binding experiments at the cellular level were conducted using a CHO-K1 cell strain overexpressing human 4-1BB (CHO-K1/hu 4-1BB, Genescript), a CHO-K1 cell strain overexpressing human CD40 (CHO-K1/hu CD40, Beijing KYinno, #KC-1286), a CHO-K1 cell strain overexpressing human OX40 (CHO-K1/hu OX40, Genescript, #M00561) and an HEK293 cell strain overexpressing human ICOS (HEK293T/ICOS, Genescript, #KC-0210). Briefly, these cells were digested and resuspended in F12K or DMEM complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor 488-conjugated AffiniPure Goat Anti-Human IgG, Fcγ Fragment Specific, Jackson, #109-545-06, 1:1000 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII.

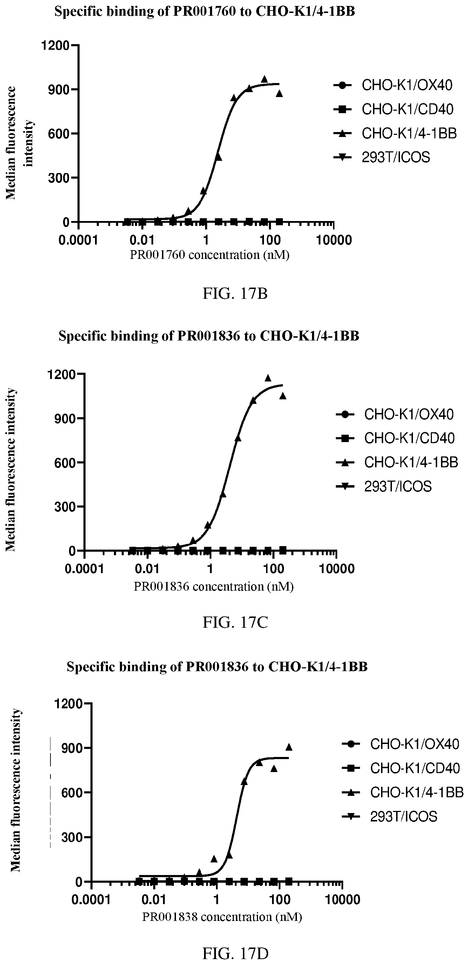

As shown in FIGs. 17A-17D, PR001758, PR001760, PR001836 and PR001838 specifically bound to the CHO-K1/hu 4-1BB cells rather than the other members of the TNF tumor necrosis factor receptor superfamily.

### Summary

The beneficial effects of the present invention are as follows:
The 4-1BB antibody of the present invention is a novel fully human antibody comprising only a "heavy chain", having specific binding activity to human 4-1BB and cynomolgus monkey 4-1BB. The 4-1BB heavy-chain antibody is only half the size of conventional IgG antibodies. Due to the absence of a light chain, the antibody can be used for bispecific antibodies, and the problems of light chain mismatching and heterodimerization are solved.

### Example 3. HER2×4-1BB Bispecific Antibodies

Human epidermal growth factor receptor 2 (HER2), also known as ERBB2, HER-2, HER-2/neu, NEU, NGL, TKR1 or c-erb B2, or ErbB2 or neu in rats, is a member of the ErbB protein family, which is commonly referred to as the epidermal growth factor receptor family. It is a highly invasive protein in breast cancer. HER2 is a tyrosine kinase bound on the cell membrane surface. It is typically involved in the signal transduction pathways leading to cell growth and differentiation. HER2 is considered an orphan receptor. No EGF ligand families can activate it. HER2 gene amplification and overexpression of its protein product are found in about 30% of breast cancer cases. The overexpression of the receptor in breast cancer is associated with disease recurrence and poor prognosis. HER2 plays a role in development, cancer, communication at neuromuscular junctions and regulation of cell growth and differentiation.

HER2×4-1BB bispecific antibodies are intended to promote 4-1BB aggregation by bridging T cells with HER2 positive tumor cells, to provide effective co-stimulatory signals for tumor antigen-specific T cells, to further enhance T cell receptor (TCR)-mediated activity and to cause tumor lysis. HER2×4-1BB-mediated activation of 4-1BB is therefore orientated toward co-localization of T cells and tumor cells *in vivo,* for example in primary tumors and tumor-infiltrating lymphocytes (TILs) or lymph nodes containing tumor metastases.

In this example, bispecific antibodies targeting both HER2 and 4-1BB were constructed for the purpose of improving the anti-tumor effect and safety through one or more mechanisms of action. Firstly, HER2×4-1BB bispecific antibodies are enriched in tumor tissues highly expressing HER2, T cells are bridged with HER2 positive tumor cells to promote 4-1BB aggregation, effective co-stimulatory signals are provided for tumor antigen-specific T cells, the T cell receptor (TCR)-mediated activity of T cells is further enhanced, and the anti-tumor activity is improved. HER2×4-1BB-mediated activation of 4-1BB is therefore orientated toward co-localization of T cells and tumor cells *in vivo,* for example in primary tumors and tumor-infiltrating lymphocytes (TILs) or lymph nodes containing tumor metastases. Secondly, the anti-4-1BB agonistic antibodies used in this example function depending on molecular crosslinking. They can only mediate T cell activation using target cells in the tumor microenvironment to avoid the toxic and side effects resulting from over-activation of T cells in normal tissues by monoclonal antibodies similar to Urelumab.

### Example 3.1. Structures and design of HER2×4-1BB bispecific antibodies

The anti-HER2 IgG antibody trastuzumab was used in this example, and its amino acid sequence was found in the IMGT database. The produced antibody was numbered PR000210.

**Table 16. Sequence listing for anti-HER2 antibody trastuzumab**

| **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR000210 | 244 | 210 | 202 | 169 | 134 | 143 | 159 | 17 | 51 | 97 |

The anti-4-1BB fully human H2L2 antibody PR000448 and derived scFv used in this example were derived from Harbour H2L2 mice. They were discovered as described in Example 1.

The anti-4-1BB fully human HCAb antibody used in this example was derived from Harbour HCAb mice. It was discovered as described in Example 2.

In this and subsequent examples, the anti-HER2 IgG monoclonal antibody PR000210 (a trastuzumab analog) was used as a positive control molecule. It was also the parent monoclonal antibody of the HER2 end of the HER2×4-1BB bispecific antibody molecules.

In this and subsequent examples, the anti-4-1BB IgG monoclonal antibodies Uremulab and Utomilumab were used as positive control molecules.

### Example 3.1.1. Construction of bispecific antibodies of IgG-VH tetravalent symmetric structure using anti-HER2 IgG antibody and anti-4-1BB HCAb antibodies

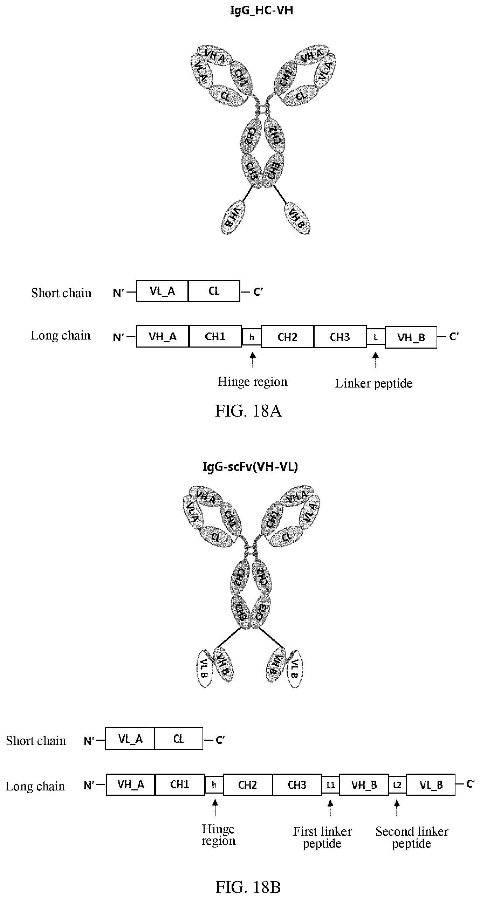

A binding protein of an IgG-VH tetravalent symmetric structure (as shown in FIG. 18A) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH 1 -h-CH2-CH3 -L-VH_B.

In one embodiment, CH3 of the polypeptide chain 2 is fusion-linked directly to VH_B, i.e., L is 0 in length. In another embodiment, CH3 of the polypeptide chain 2 is linked to VH_B via the linker peptide L; L may be the sequence listed in Table 17.

**Table 17. Linker peptides**

| **Name of linker peptide** | **Length of linker peptide** | **Sequence of linker peptide** | **Sequence No.** |
|---|---|---|---|
| GS_4 | 4 | GSGS | 273 |
| GS_5 | 5 | GGGGS | 274 |
| GS_6 | 6 | GGSGGS | 275 |
| GS_7 | 7 | GGGGSGS | 276 |
| GS_15 | 15 | GGGGSGGGGSGGGGS | 277 |
| GS_20 | 20 | GGGGSGGGGSGGGGSGGGGS | 278 |
| GS_25 | 25 | GGGGSGGGGSGGGGSGGGGS GGGGS | 279 |
| Human IgG1 hinge | 15 | EPKSCDKTHTCPPCP | 280 |
| Human IgG1 hinge (C220S) | 15 | EPKSSDKTHTCPPCP | 281 |
| H1_15 | 15 | EPKSSDK1H1PPPPP | 282 |
| G5-LH | 15 | GGGGGDKTHTCPPCP | 283 |
| H1_15-RT | 17 | EPKSSDKTHTPPPPPRT | 284 |
| L-GS_15-RT | 18 | LGGGGSGGGGSGGGGSRT | 285 |
| L-H1_15-RT | 18 | LEPKSSDKTHTPPPPPRT | 286 |
| KL-H1_15-RT | 19 | KLEPKSSDKTHTPPPPPRT | 287 |
| KL-H1_15-AS | 19 | KLEPKSSDKTHTPPPPPAS | 288 |
| RT-GS_5-KL | 9 | RTGGGGSKL | 289 |
| RT-GS_15-KL | 19 | RTGGGGSGGGGSGGGGSKL | 290 |
| RT-GS_25-KL | 29 | RTGGGGSGGGGSGGGGSGGG GSGGGGSKL | 291 |
| EPKSSD | 6 | EPKSSD | 292 |
| AS-GS_15 | 17 | ASGGGGSGGGGSGGGGS | 293 |
| GS_2 | 2 | GS | |

HER2×4-1BB bispecific antibody molecules of an IgG-VH tetravalent symmetric structure were designed using an anti-HER2 IgG antibody and anti-4-1BB HCAb heavy-chain antibodies and summarized in Table 18. The bispecific antibody molecule samples were prepared, analyzed for physicochemical properties and summarized in Table 19.

**Table 18. HER2x4-1BB bispecific antibody molecules of an IgG-VH tetravalent symmetric structure**

| **Bispecific antibody molecules** | **HER2 antibody (IgG)** | **4-1BB antibody (VH_B)** | **VH_B position relative to IgG** | **Linker peptide (between VH_B and IgG)** | **Fc type (mutation)** |
|---|---|---|---|---|---|
| PR002812 | trastuzumab | PR001758 | C-terminus of heavy chain | H1_15-RT | Human IgG1 (L234A, L235A, P329G) |
| PR002813 | trastuzumab | PR001760 | C-terminus of heavy chain | H1_15-RT | Human IgG1 (L234A, L235A, P329G) |
| PR002815 | trastuzumab | PR001764 | C-terminus of heavy chain | H1_15-RT | Human IgG1 (L234A, L235A, P329G) |
| PR002820 | trastuzumab | PR001774 | C-terminus of heavy chain | H1_15-RT | Human IgG1 (L234A, L235A, P329G) |
| PR002821 | trastuzumab | PR001775 | C-terminus of heavy chain | H1_15-RT | Human IgG1 (L234A, L235A, P329G) |
| PR002822 | trastuzumab | PR001780 | C-terminus of heavy chain | H1_15-RT | Human IgG1 (L234A, L235A, P329G) |
| PR002823 | trastuzumab | PR001781 | C-terminus of heavy chain | H1_15-RT | Human IgG1 (L234A, L235A, P329G) |
| PR002824 | trastuzumab | PR001830 | C-terminus of heavy chain | H1_15-RT | Human IgG1 (L234A, L235A, P329G) |
| PR002825 | trastuzumab | PR001831 | C-terminus of heavy chain | H1_15-RT | Human IgG1 (L234A, L235A, P329G) |
| PR002826 | trastuzumab | PR001833 | C-terminus of heavy chain | H1_15-RT | Human IgG1 (L234A, L235A, P329G) |
| PR002827 | trastuzumab | PR001836 | C-terminus of heavy chain | H1_15-RT | Human IgG1 (L234A, L235A, P329G) |
| PR002828 | trastuzumab | PR001838 | C-terminus of heavy chain | H1_15-RT | Human IgG1 (L234A, L235A, P329G) |
| PR002829 | trastuzumab | PR001840 | C-terminus of heavy chain | H1_15-RT | Human IgG1 (L234A, L235A, P329G) |

**Table 19. Physicochemical properties of HER2×4-1BB bispecific antibody molecules**

| **Bispecific antibody molecules** | **Expression system and volume** | **Plasmid transfection ratio (short chain:long chain)** | **Yield (mg/L) after first purification** | **HPLC-SE C purity (%)** | **HPLC-HIC retention time (min)** | **DSF Tm1 (°C)** |
|---|---|---|---|---|---|---|
| PR002812 | Expi293F (10ml) | 3 : 2 | 52.7 | 71.15 | 18.081 | 65.8 |
| PR002813 | Expi293F (10ml) | 3 : 2 | 37.2 | 80.50 | 18.402 | 66.2 |
| PR002815 | Expi293F (10ml) | 3 : 2 | 44.6 | 68.08 | 19.077 | 67.2 |
| PR002820 | Expi293F (10ml) | 3 : 2 | 92.5 | 70.72 | 18.082 | 67.0 |
| PR002821 | Expi293F (10ml) | 3 : 2 | 55.9 | 74.62 | 18.742 | 62.4 |
| PR002822 | Expi293F (10ml) | 3 : 2 | 29.7 | 84.14 | 19.143 | 63.2 |
| PR002823 | Expi293F (10ml) | 3 : 2 | 49.5 | 85.87 | 18.153 | 66.2 |
| PR002824 | Expi293F (10ml) | 3 : 2 | 48.0 | 79.89 | 17.734 | 67.0 |
| PR002825 | Expi293F (10ml) | 3 : 2 | 82.4 | 63.99 | 17.937 | 67.2 |
| PR002826 | Expi293F (10ml) | 3 : 2 | 61.1 | 87.09 | 17.585 | 67.4 |
| PR002827 | Expi293F (10ml) | 3 : 2 | 29.5 | 73.75 | 17.366 | 66.4 |
| PR002828 | Expi293F (10ml) | 3 : 2 | 77.7 | 86.83 | 18.932 | 67.4 |
| PR002829 | Expi293F (10ml) | 3 : 2 | 83.3 | 32.37 | 18.439 | 67.4 |

### Example 3.1.2. Construction of bispecific antibodies of IgG-scFv tetravalent symmetric structure using anti-HER2 IgG antibody and anti-4-1BB H2L2 antibody

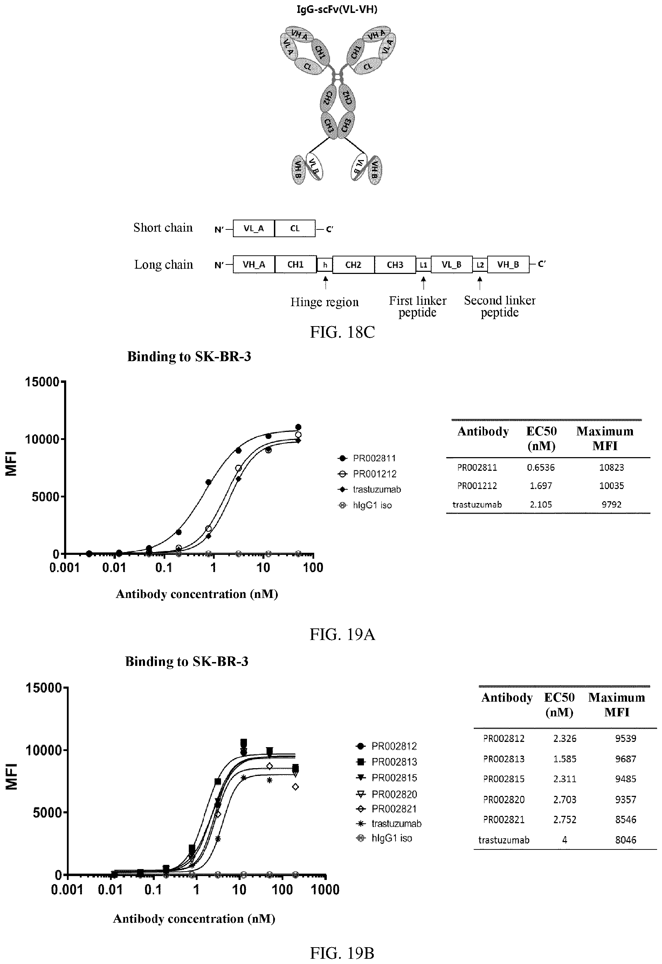

A binding protein of an IgG-scFv tetravalent symmetric structure (as shown in FIG. 18B) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-h-CH2-CH3-L1-VH_B L2-VL_B;
or alternatively,
a binding protein of an IgG-scFv tetravalent symmetric structure (as shown in FIG. 18C) comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-h-CH2-CH3-L1-VL_B L2-VH_B.

The linker peptide L1 and the linker peptide L2 of polypeptide chain 2 may be the sequences listed in Table 17.

HER2×4-1BB bispecific antibody molecules of an IgG-scFv tetravalent symmetric structure were designed using an anti-HER2 IgG antibody and an anti-4-1BB H2L2 antibody and summarized in Table 20. The bispecific antibody molecule samples were prepared, analyzed for physicochemical properties and summarized in Table 21.

**Table 20. HER2×4-1BB bispecific antibody molecules of an IgG-scFv tetravalent symmetric structure**

| **Bispecific antibody molecules** | **HER2 antibody (IgG)** | **4-1BB antibody (scFv)** | **Structure of scFv end** | **First linker peptide (between Fc and scFv)** | **Second linker peptide (between VH_B and VL_B)** | **Fc type (mutation)** |
|---|---|---|---|---|---|---|
| PR001212 | trastuzumab | PR000448 | VH-linker peptide-VL | KL-H1_15-RT | GS_15 | Human IgG1 |
| PR002811 | trastuzumab | PR000448 | VH-linker peptide-VL | H1_15-RT | GS_15 | Human IgG1 (L234A, L235A, P329G) |

**Table 21. Physicochemical properties of HER2×4-1BB bispecific antibody molecules**

| **Bispecific antibody molecules** | **Expression system and volume** | **Plasmid transfection ratio (short chain:long chain)** | **Yield (mg/L) after first purification** | **HPLC-SEC purity (%)** | **HPLC-HIC retention time (min)** | **DSF Tm1 (°C)** |
|---|---|---|---|---|---|---|
| PR001212 | HEK293F (30ml) | 3 : 2 | 22.3 | 85.05 | 17.563 | 51.4 |
| PR002811 | Expi293F (10ml) | 3 : 2 | 2.0 | | | |

**Table 22. Sequence listing for HER2×4-1BB bispecific antibody molecules**

| **Antibody No.** | **Polypeptide chain 1** | **Polypeptide chain 2** |
|---|---|---|
| PR001212 | 244 | 251 |
| PR002811 | 244 | 252 |
| PR002812 | 244 | 253 |
| PR002813 | 244 | 254 |
| PR002815 | 244 | 255 |
| PR002820 | 244 | 256 |
| PR002821 | 244 | 257 |
| PR002822 | 244 | 258 |
| PR002823 | 244 | 259 |
| PR002824 | 244 | 260 |
| PR002825 | 244 | 261 |
| PR002826 | 244 | 262 |
| PR002827 | 244 | 263 |
| PR002828 | 244 | 264 |
| PR002829 | 244 | 265 |

### Example 3.2. FACS assay for abilities of HER2×4-1BB bispecific antibodies to bind to SK-BR-3 cells

This example is intended to investigate the *in vitro* binding activity of 4-1BB bispecific antibodies to SK-BR-3. SK-BR-3 is a breast cancer cell that highly expresses Her2. Antibody binding experiments at the cellular level were conducted using SK-BR-3 cells. Briefly, SK-BR-3 cells were digested and resuspended in a complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor^{®} 647 AffiniPure Goat Anti-Human IgG, F(ab')₂ fragment specific, Jackson Immunoreserach, #109-605-006, 1:1000 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII.

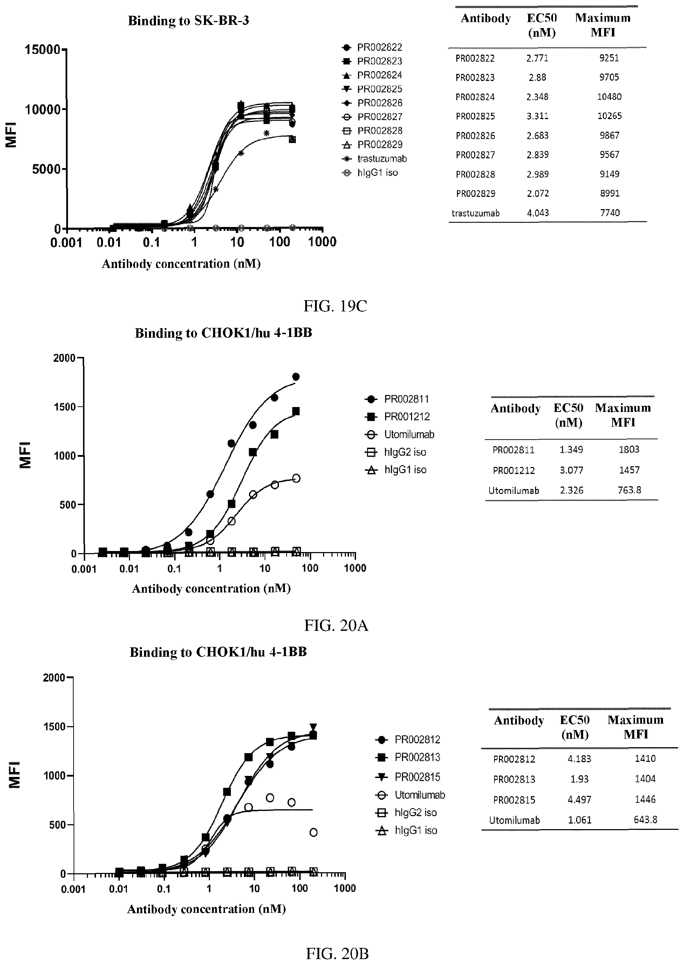

As shown in FIGs. 19A-19C, the Her2×4-1BB bispecific antibodies of the present invention could all bind to human SK-BR-3, and the binding abilities of the antibodies determined increased with the antibody concentrations in a positive correlation. PR002813 exhibited a lower EC₅₀ than the control antibody (trastuzumab) at the same concentration, while the other antibodies were comparable to the control antibody.

### Example 3.3. FACS assay for abilities of HER2×4-1BB bispecific antibodies to bind to CHO-K1/hu 4-1BB cells

This example is intended to investigate the *in vitro* binding activity of 4-1BB bispecific antibodies to 4-1BB. Antibody binding experiments at the cellular level were conducted using a CHO-K1 cell strain overexpressing human 4-1BB (CHO-K1/hu 4-1BB, Genescript). Briefly, the CHO-K1/hu 4-1BB cells were digested and resuspended in F12K complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor^{®} 647 AffiniPure Goat Anti-Human IgG, F(ab')₂ fragment specific, Jackson Immunoreserach, #109-605-006, 1:1000 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using NovoCyte flow cytometer (ACEA Biosciences).

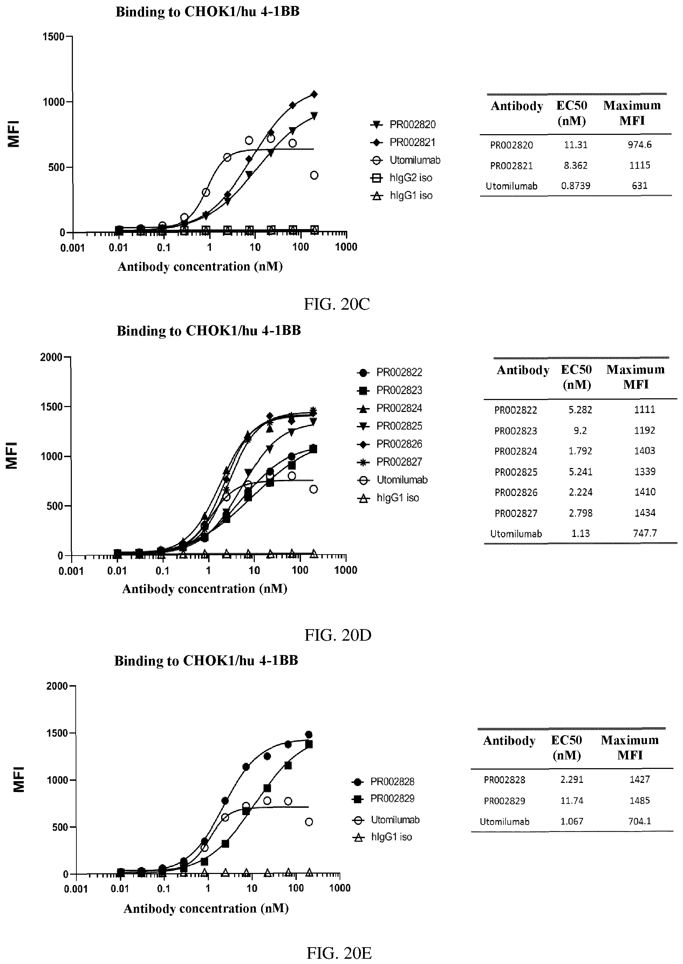

As shown in FIGs. 20A-20E, the Her2×4-1BB bispecific antibodies of the present invention could all bind well to human 4-1BB, and the binding abilities of the antibodies determined increased with the antibody concentrations in a positive correlation. These antibodies could bind to human 4-1BB more sensitively at lower concentrations. Among them, PR002811, PR001212, PR002813 and PR002824 were the best; their EC₅₀ values were superior to that of the control antibody Utomilumab. Other bispecific antibodies were comparable to or slightly weaker than Utomilumab, but their maximum MFI values were all higher than that of the control antibody Utomilumab.

### Example 3.4. FACS assay for abilities of HER2×4-1BB bispecific antibodies to bind to CHO-K1/cyno 4-1BB cells

This example is intended to investigate the *in vitro* binding activity of 4-1BB bispecific antibodies to 4-1BB. Antibody binding experiments at the cellular level were conducted using a CHO-K1 cell strain overexpressing cynomolgus monkey 4-1BB (CHO-K1/cyno 4-1BB, Genescript). Briefly, the CHO-K1/cyno 4-1BB cells were digested and resuspended in F12K complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at a concentration that was 2 times the final concentration, each at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (Alexa Fluor^{®} 647 AffiniPure Goat Anti-Human IgG, F(ab')₂ fragment specific, Jackson Immunoreserach, #109-605-006, 1:1000 diluted) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, the cells in each well were resuspended in 200 µL of pre-cooled PBS, and the fluorescence signal values were read using NovoCyte flow cytometer (ACEA Biosciences).

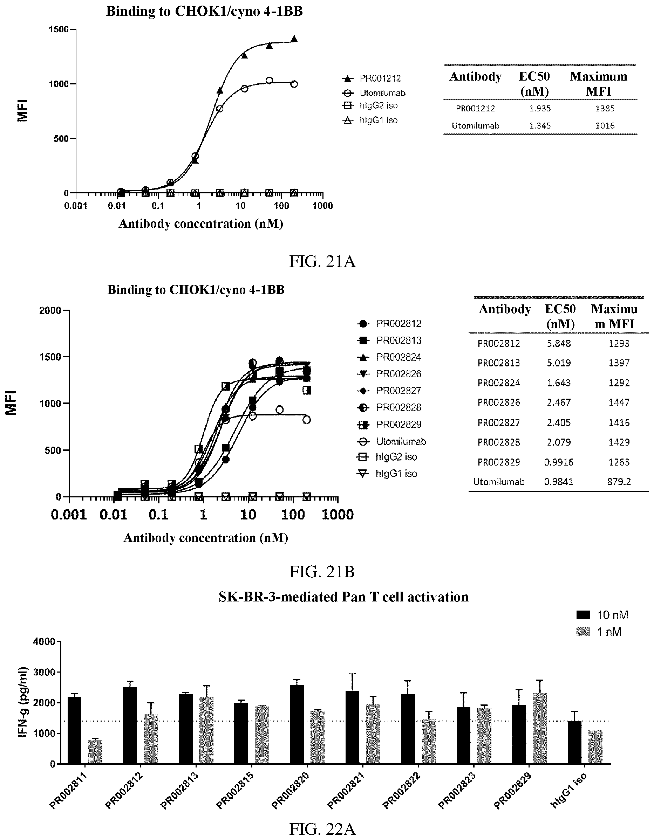

As shown in FIGs. 21A-21B, the HER2×4-1BB bispecific antibodies of the present invention could all bind well to cynomolgus monkey 4-1BB, and the binding abilities of the antibodies determined increased with the antibody concentrations in a positive correlation. PR001212, PR002824 and PR002826-PR002829 were comparable to the control antibody Utomilumab in binding to cynomolgus monkey 4-1BB.

### Example 3.5. HER2/4-1BB bispecific antibodies can activate T cell pathway in vitro

1 mg/mL OKT3 (eBiosciences, #16-0037-85) was diluted with PBS. A 96-well plate (Corning, #3599) was coated with 0.08 µg/mL OKT3 at 100 µL/well, and its final concentration was brought to 10 µg/mL. The coating was perform at 4 °C overnight. The next day, SK-BR-3 cells were digested and resuspended in a complete medium, and the cell density was adjusted to 4×10⁵ cells/mL for later use. Human CD3 positive T cells were isolated from human PBMCs using an MACS kit (Miltenyi Biotec, #130-096-535). The number of the cells was determined firstly, and then corresponding amounts of MACS buffer and Pan-T cell biotin antibody were added according to the number of the cells. The mixture was well mixed and left to stand at 4 °C for 5 min. Then, a corresponding amount of magnetic microbeads was added, and the mixture was left to stand at 4 °C for 10 min. What passed through the LS column were CD3 positive T cells. The previous day's OKT3 for coating was washed off the 96-well plate, and 50 µL of purified T cells were added at 1×10⁵ cells/well. 50 µL of SK-BR-3 cells were then added to the 96-well plate at 2×10⁴ cells/well. Then HER2/4-1BB bispecific antibodies or the control monoclonal antibody with corresponding concentrations were added. The cells were cultured in an incubator at 37 °C with 5% CO₂. After 72 h of culture, the supernatant was collected and assayed for IFN-γ content using an ELISA kit (Invitrogen, #88-7316). The ELISA assay was performed according to the supplier's instructions. Briefly, the coating antibody was added to the 96-well flat-bottom plate, and the plate was incubated at 4 °C overnight. The next day, ELISA buffer was added, and the plate was incubated at room temperature for 1 h. The collected supernatant was added, and the plated was incubated at room temperature for 2 h. The plate was washed twice, and test antibodies were added. The plate was incubated at room temperature for 1 h. The plate was washed twice, and HRP-streptavidin was added. The plate was incubated at room temperature for 1 h. Then TMB substrate was added, and ELISA stop buffer (BBI life sciences, #E661006-0200) was added 10-30 min later. The OD450-570 values were read using a plate reader (Molecular Devices, #SpectraMax Plus). The values were analyzed with Graphad 8.0 and plotted.

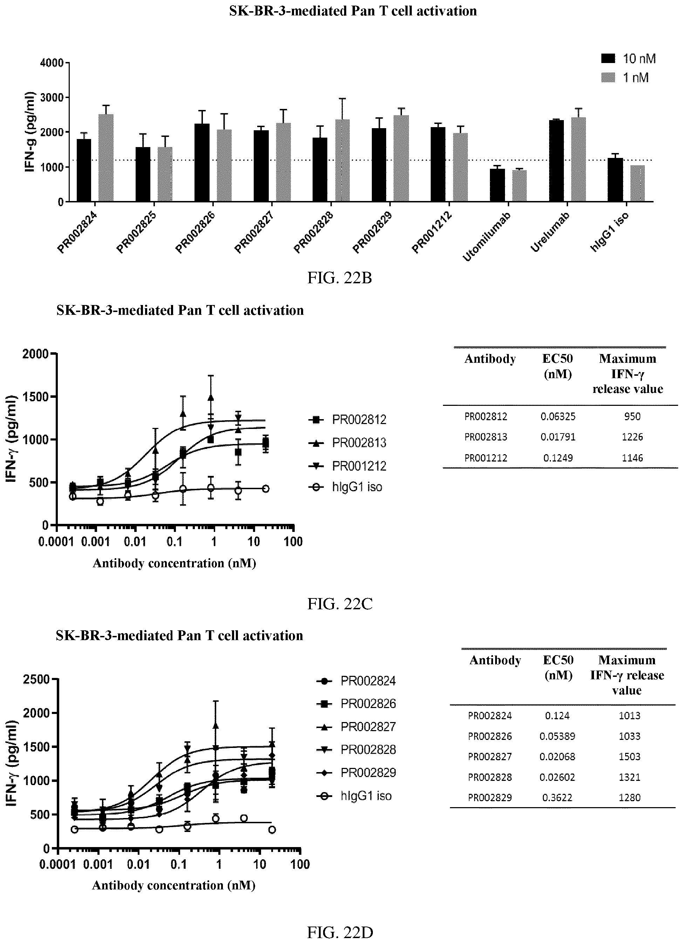

As shown in FIGs. 22A-22D, all the HER2×4-1BB bispecific antibodies of the present invention activated the 4-1BB-mediated T cell pathway under tumor cell SK-BR-3 crosslinking. Among them, PR002813 and PR002827 showed lower EC₅₀ values and higher IFN gamma release values.

### Summary

In this example, HER2×4-1BB bispecific antibody molecules of an IgG-VH tetravalent symmetric structure were constructed using the antigen-binding domain Fab of the anti-Her2 IgG antibody and the antigen-binding domain VHs of the anti-4-1BB HCAb antibodies. In addition, bispecific antibodies of an IgG-scFv tetravalent symmetric structure were constructed using the antigen-binding domain Fab of the anti-Her2 IgG antibody and the anti-4-1BB H2L2 antibody.

The anti-HER2×4-1BB bispecific antibody molecules of the two structures regulated the T cell function-activating activity through different structure types, relative positions, binding valences and other parameters. Meanwhile, this shows the flexibility of constructing a bispecific antibody molecular structure based on HCAb.

The activation of T cells by Urelumab is not target-specific, which is one of the causes for its clinical toxic and side effects. However, the activation of T cells by the HER2×4-1BB bispecific antibodies is specifically dependent on HER2 expression. The HER2×4-1BB bispecific antibodies can specifically activate T cells in the presence of cells highly expressing HER2.

In conclusion, the HER2×4-1BB bispecific antibody molecules with outstanding functional activity and good molecular stability are constructed in this example.

### Example 4. PD-L1×4-1BB Bispecific Antibodies

Programmed death receptor 1 (PD-1) is mainly expressed in immune cells such as T cells. It has two ligands, i.e., programmed death ligand 1 (PD-L1) and PD-L2. PD-L1 is mainly expressed in antigen-presenting cells and a variety of tumor cells. The interaction between PD-L1 and PD-1 can down-regulate the activity of T cells, weaken the secretion of cytokines and play a role in immunosuppression. The expression of the PD-L1 protein can be detected in many human tumor tissues. The microenvironment at the tumor site can induce the expression of PD-L1 on tumor cells, and the expressed PD-L1 facilitates the occurrence and growth of tumors, induces the apoptosis of anti-tumor T cells and further protects the tumor cells from immune attack.

4-1BB (TNFRSF9, CD137) is a transmembrane protein belonging to the TNF receptor superfamily. 4-1BB is a co-stimulatory molecule expressed on a variety of immune cells. It is a multifunctional modulator of immune activity. Its expression is induced in activated T cells, NK cells and other immune cells. 4-1BB activates T cells through trimerization mediated by its ligand 4-1BBL, promoting cell proliferation and cytokine release. Anti-4-1BB agonistic antibodies have the function of inhibiting tumors. Urelumab (BMS-663513) from Bristol-Myers Squibb (BMS) was the first anti-4-1BB fully human monoclonal antibody to be subjected to clinical trials. The initial clinical results of Urelumab were published in 2008. Although encouraging efficacy was observed in some patients, the data showed Urelumab to cause target and dose-associated hepatotoxicity. Moreover, two patients in the clinical trial died from hepatotoxicity, resulting in the discontinuation of related clinical trials.

In this example, bispecific antibodies targeting both PD-L1 and 4-1BB were constructed for the purpose of improving the anti-tumor effect and safety through one or more mechanisms of action. Firstly, the PD-L1×4-1BB bispecific antibody can activate T cells by blocking the PD-1/PD-L1 signaling pathway. Secondly, the PD-L1 molecules highly expressed on the tumor cell surfaces can use the bispecific antibody molecules to promote the crosslinking and trimerization of the 4-1BB molecules on the T cell surfaces and activate the downstream signaling pathway, thereby promoting T cell activation and proliferation. Thirdly, the bispecific antibody molecule-mediated T cell activation is limited to the tumor microenvironment, so that the toxic and side effects caused by over-activation of T cells in normal tissues by monoclonal antibodies similar to Urelumab can be avoided.

### Example 4.1. Structures and design of PD-L1×4-1BB bispecific antibodies

The anti-PD-L1 IgG antibody PR000151 (Atezolizumab analog) was used in this example, and its corresponding amino acid sequence was found in the IMGT database.

The anti-PD-L1 fully human IgG antibody PR000265 (Table 4.2) used in this example was derived from Harbour H2L2 mice. It was discovered as follows.

The Harbour H2L2 mouse (Harbour Antibodies BV) is a transgenic mouse carrying an immune repertoire of human immunoglobulins that produces antibodies with intact human antibody variable domains and rat constant domains. Harbour H2L2 mice were subjected to multiple rounds of immunization with a soluble recombinant human PD-L1 protein (NovoProtein, #C764). When the titer of the PD-L1-specific antibody in the serum of mice was detected to reach a certain level, spleen cells of the mice were taken and fused with a myeloma cell line to obtain hybridoma cells. After multiple rounds of screening and cloning of the hybridoma cells, several monoclonal antibody molecules specifically recognizing PD-L1 were identified. Those monoclonal antibodies were further identified, and several candidate antibody molecules were preferentially selected according to parameters such as the binding ability to human PD-L1, the binding ability to cynomolgus monkey PD-L1, and the ability to inhibit the binding of PD-L1 to PD-1. The candidate antibody molecules were then subjected to sequence analysis and optimization to obtain several variant sequences. The VL and VH sequences of the antibody were fused to the corresponding human κ light chain constant region and IgG1 heavy chain constant region sequences and expressed to obtain recombinant fully human antibody molecules. Anti-PD-L1 recombinant fully human IgG antibodies are listed in Table 23.

**Table 23. Anti-PD-L1 recombinant fully human IgG antibodies**

| **Antibody No.** | **Light chain** | **Heavy chain** | **VL** | **VH** | **LCDR1** | **LCDR2** | **LCDR3** | **HCDR1** | **HCDR2** | **HCDR3** |
|---|---|---|---|---|---|---|---|---|---|---|
| PR000265 | 245 | 211 | 203 | 170 | 135 | 146 | 160 | 15 | 52 | 98 |
| PR000151 | 241 | 207 | 199 | 166 | 131 | 143 | 156 | 14 | 48 | 94 |

The anti-4-1BB fully human IgG antibodies PR000197 and PR000448 used in this example were derived from Harbour H2L2 mice. They were discovered as described in Example 1.

The anti-4-1BB fully human HCAb antibodies PR001758, PR001760 and PR001836 used in this example were derived from Harbour HCAB mice. They were discovered as described in Example 2.

In this and subsequent examples, the anti-PD-L1 IgG monoclonal antibody PR000265 was used as a positive control molecule.

In this and subsequent examples, the anti-4-1BB IgG monoclonal antibodies Uremulab and Utomilumab were used as positive control molecules.

### Example 4.1.1. Construction of bispecific antibody molecules of FIT-Ig structure using anti-PD-L1 IgG antibodies and anti-4-1BB IgG antibodies

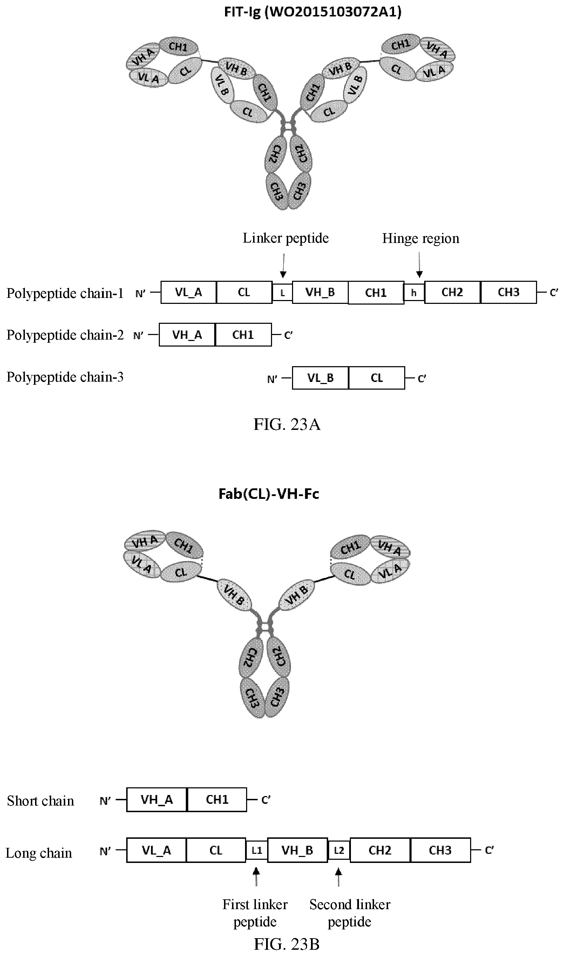

In this example, anti-PD-L1×4-1BB bispecific antibody molecules of an FIT-Ig structure were constructed using the antigen-binding domain Fab of the anti-PD-L1 IgG antibody PR000265 or PR000151 (atezolizumab analog) and the antigen-binding domain Fab of the anti-4-1BB IgG antibody PR000197 or PR000448. The FIT-Ig structure can be designed as described in reference patent WO2015/103072A1. The structure is shown in FIG. 23A.

The constructed molecules are summarized in Table 24; antibody molecule samples were prepared and analyzed as described in Examples 1 and 2, and summarized in Table 25. The sequence numbers corresponding to the polypeptide chains of the bispecific antibody molecules of the FIT-Ig structure are listed in Table 26.

**Table 24. PD-L1x4-1BB bispecific antibody molecules of FIT-Ig structure**

| **Bispecific antibody molecules** | **PD-L1 antibody (Fab A)** | **4-1BB antibody (Fab B)** | **4-1BB Fab position** | **Linker peptide (between CL and VH_B)** | **Fc type (mutation)** |
|---|---|---|---|---|---|
| PR000701 | PR000265 | PR000197 | Close to Fc | None | Human IgG4 |
| PR003052 | atezolizumab | PR000448 | Close to Fc | None | Human IgG4 |

**Table 25. Expression of PD-L1x4-1BB bispecific antibody molecule proteins of FIT-Ig structure**

| **Bispecific antibody molecules** | **Expression system and volume** | **Yield (mg/L) after first purification** | **SEC-HPLC purity (%)** |
|---|---|---|---|
| PR000701 | HEK293-F (30ml) | 198 | 79.88 |
| PR003052 | HEK293-6E (40ml) | 47.5 | 87.76 |

**Table 26. Sequence numbers of PD-L1×4-1BB bispecific antibody molecules of FIT-Ig structure**

| **Antibody No.** | **Polypeptide chain 1** | **Polypeptide chain 2** | **Polypeptide chain 3** |
|---|---|---|---|
| PR000701 | 250 | 249 | 246 |
| PR003052 | 267 | 266 | 246 |

### Example 4.1.2. Construction of bispecific antibody molecules of Fab-HCAb structure using anti-PD-L1 IgG antibody and anti-4-1BB HCAb antibody

In this example, PD-L1×4-1BB bispecific antibody molecules of a variety of structures were constructed using the antigen-binding domain Fab of the anti-PD-L1 IgG antibody PR000265 and the antigen-binding domain VH of the anti-4-1BB HCAb antibody PR001758, PR001760, or PR001836.

In this and subsequent examples, the anti-PD-L1 IgG monoclonal antibody PR000265 was used as a positive control molecule. It was also the parent monoclonal antibody of the PD-L1 end of the PD-L1×4-1BB bispecific antibody molecules.

In this and subsequent examples, the anti-4-1BB IgG monoclonal antibody urelumab (IgG4) or utomilumab (IgG2) was used as a positive control molecule.

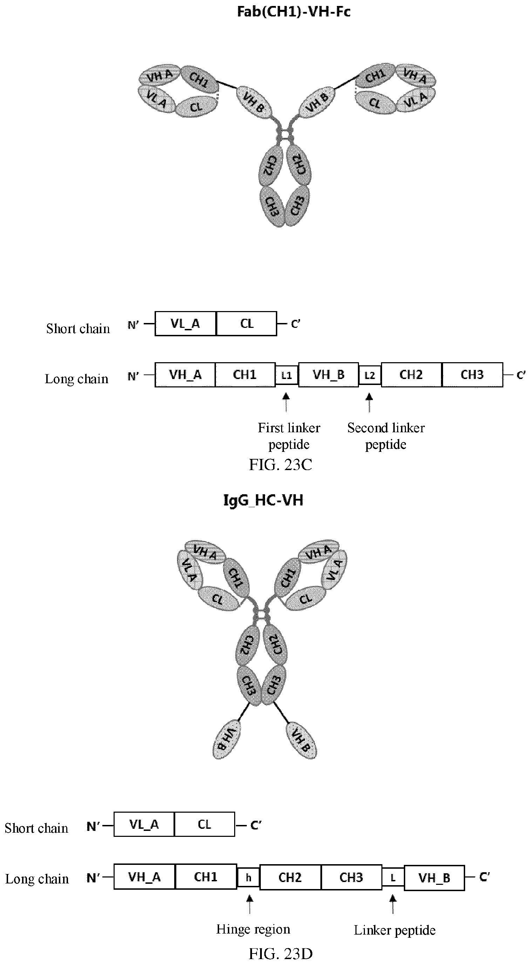

As shown in FIG. 23B and 23C, the Fab end is derived from the anti-PD-L1 IgG antibody used in this example. The VH end is derived from the antigen-binding domain VH of the anti-4-1BB HCAb antibody PR001758, PR001760 or PR001836. CL is the light chain constant region domain. CH1, CH2 and CH3 are the first, second and third domains of the heavy chain constant region, respectively. L1 and L2 are the first and second linker peptides, respectively.

### Fab(CL)-VH-Fc

A binding protein of the structure shown in FIG. 23B comprises two polypeptide chains: polypeptide chain 1, also known as short chain, from amino-terminus to carboxyl-terminus, comprising VH_A-CH1; and polypeptide chain 2, also known as long chain, from amino-terminus to carboxyl-terminus, comprising VL_A-CL-L1-VH_B-L2-CH2-CH3. In the structure Fab(CL)-VH-Fc, VL_A of the antibody A and VH_B of the heavy-chain antibody B are fused on the same polypeptide chain, so that the mismatched byproducts generated by the association of VL_A and VH_B can be avoided.

VH_B of the polypeptide chain 2 is linked to CH2 via a linker peptide L2; L2 may be a hinge region or a hinge region-derived linker peptide sequence or the sequence listed in Table 17, preferably the sequence of human IgG1 hinge region, human IgG1 hinge (C220S) or G5-LH.

In one embodiment, CL of the polypeptide chain 2 is fusion-linked directly to VH_B, i.e., L1 is 0 in length. In another embodiment, CL of the polypeptide chain 2 is linked to VH_B via the linker peptide L1; L1 may be the sequence listed in Table 17.

### Fab(CH1)-VH-Fc

A binding protein of the structure shown in FIG. 23C comprises two polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_A-CH1-L1-VH_B-L2-CH2-CH3.

VH_B of the polypeptide chain 2 is linked to CH2 via a linker peptide L2; L2 may be a hinge region or a hinge region-derived linker peptide sequence or the sequence listed in Table 17, preferably the sequence of human IgG1 hinge region, human IgG1 hinge (C220S) or G5-LH.

In one embodiment, CH1 of the polypeptide chain 2 is fusion-linked directly to VH_B, i.e., L1 is 0 in length. In another embodiment, CH1 of the polypeptide chain 2 is linked to VH_B via the linker peptide L1; L1 may be the sequence listed in Table 17.

A PD-L1×4-1BB bispecific antibody molecule of an Fab-HCAb symmetric structure was designed using an anti-PD-L1 IgG antibody and an anti-4-1BB heavy-chain antibody and summarized in Table 27. The bispecific antibody molecule was prepared, analyzed for physicochemical properties and summarized in Table 28.

**Table 27. PD-L1×4-1BB bispecific antibody molecule of Fab-HCAb symmetric structure**

| **Bispecific antibody molecules** | **PD-L1 antibody (Fab)** | **4-1BB antibody (VH_B)** | **First linker peptide (between** | **Second linker peptide (between VH_B and CH2)** | **Fc type (mutation)** |
|---|---|---|---|---|---|
| | | | **Fab and VH_B**) | | |
| PR004270 | PR000265 | PR001760 | H1_15 | Human IgG1 hinge (C220S) | Human IgG1 (L234A, L235A) |

**Table 28. Expression of PD-L1×4-1BB bispecific antibody molecule protein of Fab-HCAb symmetric structure**

| **Bispecific antibody molecules** | **Expression system and volume** | **Plasmid transfection ratio (short chain:long chain)** | **Yield (mg/L) after first purification** | **SEC-HPLC purity (%)** |
|---|---|---|---|---|
| PR004270 | HEK293-6E (40ml) | 3 : 2 | 77.50 | 92.33 |

### Example 4.1.3. Construction of molecules of IgG-VH tetravalent symmetric structure

PD-L1×4-1BB bispecific antibody molecules of an IgG-VH tetravalent symmetric structure (FIG. 23D) were designed according to the structure described in Example 3.1.1 using an anti-PD-L1 IgG antibody and anti-4-1BB heavy-chain antibodies.

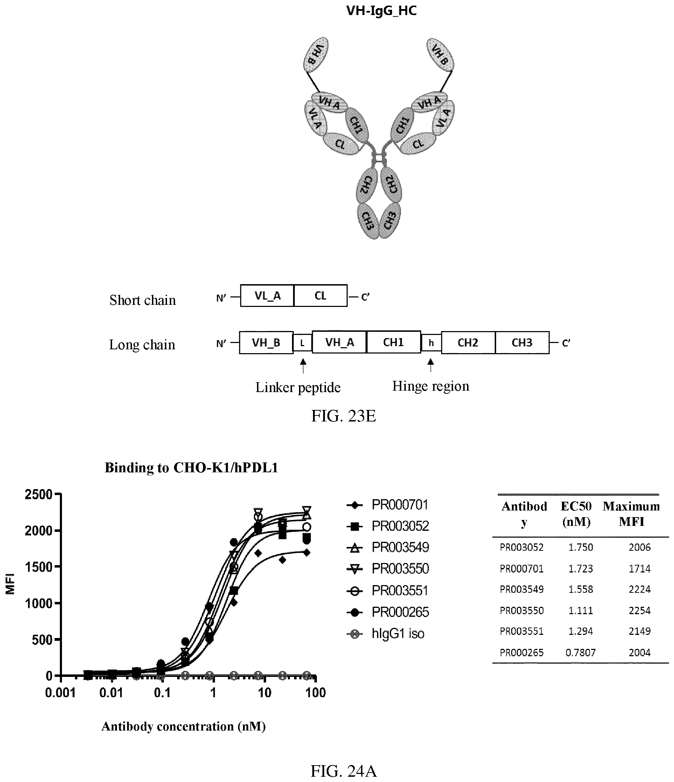

Alternatively, the VH of the anti-4-1BB HCAb antibody was linked to the N-terminus of the heavy chain of the PD-L1 IgG antibody via a linker peptide (shown in FIG. 23E). A binding protein of the structure shown in FIG. 23E comprises two different polypeptide chains: polypeptide chain 1, also known as short chain, from amino-terminus to carboxyl-terminus, comprising VL_A-CL; and polypeptide chain 2, also known as long chain, from amino-terminus to carboxyl-terminus, comprising VH_B-L-VH_A-CH1-h-CH2-CH3. In one embodiment, VH_B of the polypeptide chain 2 is fusion-linked directly to VH_A, i.e., L is 0 in length. In another embodiment, VH_B of the polypeptide chain 2 is linked to VH_A via the linker peptide L; L may be the sequence listed in Table 17.

PD-L1×4-1BB bispecific antibody molecules of an IgG-VH symmetric structure were designed using an anti-PD-L1 IgG antibody and anti-4-1BB heavy-chain antibodies and summarized in Table 29. The bispecific antibody molecules were prepared, analyzed for physicochemical properties and summarized in Table 30.

**Table 29. PD-L1×4-1BB bispecific antibody molecules of IgG-VH tetravalent symmetric structure**

| **Bispecific antibody molecules** | **PD-L1 antibody (IgG)** | **4-1BB antibody (VH_B)** | **VH_B position relative to IgG** | **Linker peptide (between VH_B and IgG)** | **Fc type (mutation)** |
|---|---|---|---|---|---|
| PR003549 | PR000265 | PR001758 | C-terminus of heavy chain | H1_15-RT | Human IgG1 (L234A, L235A, P329G) |
| PR003550 | PR000265 | PR001760 | C-terminus of heavy chain | H1_15-RT | Human IgG1 (L234A, L235A, P329G) |
| PR003551 | PR000265 | PR001836 | C-terminus of heavy chain | H1_15-RT | Human IgG1 (L234A, L235A, P329G) |
| PR004268 | PR000265 | PR001760 | N-terminus of heavy chain | GS_15 | Human IgG1 (L234A, L235A) |
| PR007130 | PR000265 | PR004469 | C-terminus of heavy chain | GS_6 | Human IgG1 (L234A, L235A, G237A) |
| PR007132 | PR000265 | PR007286 | C-terminus of heavy chain | GS_6 | Human IgG1 (L234A, L235A, G237A) |
| PR007133 | PR000265 | PR007287 | C-terminus of heavy chain | GS_6 | Human IgG1 (L234A, L235A, G237A) |
| PR007135 | PR000265 | PR007288 | C-terminus of heavy chain | GS_6 | Human IgG1 (L234A, L235A, G237A) |
| PR007136 | PR000265 | PR007289 | C-terminus of heavy chain | GS_6 | Human IgG1 (L234A, L235A, G237A) |
| PR007137 | PR000265 | PR007290 | C-terminus of heavy chain | GS_6 | Human IgG1 (L234A, L235A, G237A) |
| PR00713 8 | PR000265 | PR007291 | C-terminus of heavy chain | GS_6 | Human IgG1 (L234A, L235A, G237A) |
| PR007139 | PR000265 | PR007292 | C-terminus of heavy chain | GS_6 | Human IgG1 (L234A, L235A, G237A) |
| PR007141 | PR000265 | PR007294 | C-terminus of heavy chain | GS_6 | Human IgG1 (L234A, L235A, G237A) |
| PR007142 | PR000265 | PR007295 | C-terminus of heavy chain | GS_6 | Human IgG1 (L234A, L235A, G237A) |
| PR007143 | PR000265 | PR007296 | C-terminus of heavy chain | GS_6 | Human IgG1 (L234A, L235A, G237A) |
| PR007145 | PR000265 | PR007297 | C-terminus of heavy chain | GS_6 | Human IgG1 (L234A, L235A, G237A) |
| PR007146 | PR000265 | PR007298 | C-terminus of heavy chain | GS_6 | Human IgG1 (L234A, L235A, G237A) |
| PR007149 | PR000265 | PR007300 | C-terminus of heavy chain | GS_6 | Human IgG1 (L234A, L235A, G237A) |

**Table 30. Expression of PD-L1×4-1BB bispecific antibody molecule proteins of IgG-VH tetravalent symmetric structure**

| **Bispecific antibody molecules** | **Expression system and volume** | **Yield (mg/L) after first purification** | **SEC-HPLC purity (%)** |
|---|---|---|---|
| PR003549 | HEK293-F (30ml) | 24.51 | 98.31 |
| PR003550 | HEK293-F (30ml) | 42.16 | 95.41 |
| PR003551 | HEK293-F (30ml) | 2.28 | 99.39 |
| PR004268 | HEK293-F (30ml) | 31.4 | 97.94 |
| PR007130 | HEK293-F (40ml) | 37 | 95.957 |
| PR007132 | HEK293-F (40ml) | 36 | 94.96 |
| PR007133 | HEK293-F (40ml) | 34 | 94.452 |
| PR007135 | HEK293-F (40ml) | 43.8 | 94.557 |
| PR007136 | HEK293-F (40ml) | 41.5 | 88.662 |
| PR007137 | HEK293-F (40ml) | 23.6 | 93.763 |
| PR007138 | HEK293-F (40ml) | 18.8 | 98.629 |
| PR007139 | HEK293-F (40ml) | 31 | 96.865 |
| PR007141 | HEK293-F (40ml) | 56.5 | 95.219 |
| PR007142 | HEK293-F (40ml) | 62 | 96.848 |
| PR007143 | HEK293-F (40ml) | 56 | 95.094 |
| PR007145 | HEK293-F (40ml) | 64 | 97.083 |
| PR007146 | HEK293-F (40ml) | 60 | 97.014 |
| PR007149 | HEK293-F (40ml) | 66.5 | 98.657 |

### Example 4.1.4. Sequence listing for PD-L1×4-1BB bispecific antibody molecules and control molecules

The corresponding sequence numbers of the PD-L1×4-1BB bispecific antibody molecules constructed in this example are listed in Table 31.

**Table 31. Sequence numbers of PD-L1×4-1BB bispecific antibody molecules of this example**

| **Antibody No.** | **Polypeptide chain 1** | **Polypeptide chain 2** | **Polypeptide chain 3** |
|---|---|---|---|
| PR000701 | 250 | 249 | 246 |
| PR003052 | 267 | 266 | 246 |
| PR003549 | 245 | 268 | |
| PR003550 | 245 | 269 | |
| PR003551 | 245 | 270 | |
| PR004270 | 249 | 272 | |
| PR004268 | 245 | 271 | |
| PR007130 | 245 | 369 | |
| PR007132 | 245 | 370 | |
| PR007133 | 245 | 371 | |
| PR007135 | 245 | 372 | |
| PR007136 | 245 | 373 | |
| PR007137 | 245 | 374 | |
| PR007138 | 245 | 375 | |
| PR007139 | 245 | 376 | |
| PR007141 | 245 | 377 | |
| PR007142 | 245 | 378 | |
| PR007143 | 245 | 379 | |
| PR007145 | 245 | 380 | |
| PR007146 | 245 | 381 | |
| PR007149 | 245 | 382 | |

### Example 4.2. Binding to cells highly expressing human PD-L1

This example is intended to investigate the binding activity of the PD-L1×4-1BB bispecific antibody molecules to PD-L1.

The binding abilities of the antibody molecules to a CHO-K1 cell strain highly expressing human PD-L1 (CHO-K1/hPDL1, GenScript, M00543) or MDA-MB-231 (ATCC, HTB-26) highly expressing human PD-L1 were determined by flow cytometry FACS. Specifically, the CHO-K1/hPDL1 cells or MDA-MB-231 were digested and resuspended in a complete medium, and the cell density adjusted to 1×10⁶ cells/mL. Thereafter, the cells were seeded in a 96-well V-bottom plate (Corning, #3894) at 100 µL/well and centrifuged at 4 °C for 5 min, and the supernatant was discarded. Then, the serially diluted antibody molecules were added to the 96-well plate at 100 µL/well, and the mixtures were well mixed. The antibody molecules might serially diluted 3-fold from the highest final concentration 200 nM to obtain a total of 12 concentrations. hIgG1 iso (CrownBio, #C0001) was used as an isotype control. The cells were incubated at 4 °C for 1 h away from light. Then, the cells in each well were rinsed twice with 100 µL of pre-cooled FACS buffer (PBS buffer containing 0.5% BSA) and centrifuged at 500 g at 4 °C for 5 min, and then the supernatant was discarded. Then, a fluorescent secondary antibody (Goat human IgG (H+L) Alexa Fluor 488 conjugation, Thermo, #A11013, 1:1000 diluted) was added at 100 µL/well, and the plate was incubated at 4 °C for 1 h away from light. Then, the cells in each well were then rinsed twice with 200 µL of pre-cooled FACS buffer and centrifuged at 500 g at 4 °C for 5 min, and then the supernatant was discarded. Finally, a pre-cooled FACS buffer was added at 200 µL/well to resuspend the cells. The fluorescence signal values were read using a BD FACS CANTOII flow cytometer.

The data were processed and analyzed by plotting using GraphPad Prism 8 software, and binding curves of the antibodies to target cells, EC₅₀ values and other parameters were obtained through four-parameter nonlinear fitting.

In this examples, the anti-PD-L1 monoclonal antibody PR000265 was used as a positive control molecule. It was also the parent monoclonal antibody of the PD-L1 end of the PD-L1×4-1BB bispecific antibody molecules.

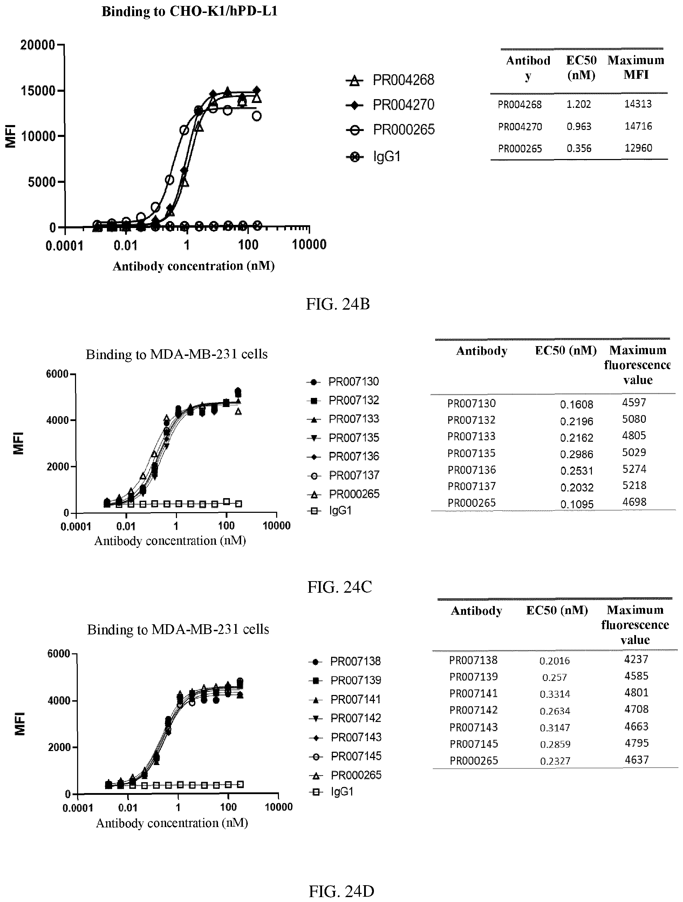

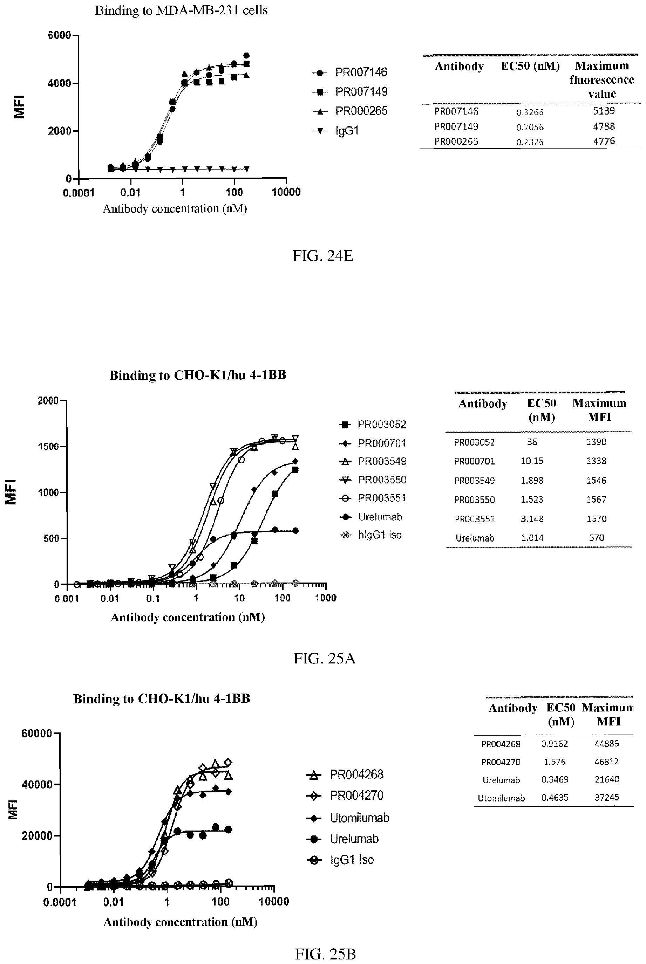

As shown in FIG. 24A, the bispecific antibody molecules of the IgG-VH (C-terminus) tetravalent symmetric structure (PR003549, PR003550 and PR003551) had similar binding ability to PD-L1 to the parent monoclonal antibody PR000265, and had slightly superior EC₅₀ values and maximum MFI values for binding to PD-L1 than the bispecific antibody molecules of the FIT-Ig structure (PR000701 and PR003052).

As shown in FIG. 24B, the abilities of the bispecific antibody molecule PR004270 of the Fab-HCAb symmetric structure and the bispecific antibody molecule PR004268 of the IgG-VH (N-terminus) tetravalent symmetric structure to bind to PD-L1 were similar to that of the parent monoclonal antibody, and their EC₅₀ values for binding to PD-L1 were slightly poorer than that of the parent monoclonal antibody, but their maximum MFI values for binding to PD-L1 were higher than that of the parent monoclonal antibody.

As shown in FIGs. 24C-24E, the abilities of the bispecific antibody molecules of the IgG-VH (C-terminus) tetravalent symmetric structure (PR007130, PR007132, PR007133, PR007135, PR007136, PR007137, PR007138, PR007139, PR007141, PR007142, PR007143, PR007145, PR007146 and PR007149) to bind to PD-L1 were similar to that of the parent monoclonal antibody PR000265.

### Example 4.3. Binding to CHO-K1 cells overexpressing 4-1BB

This example is intended to investigate the binding activity of the PD-L1×4-1BB bispecific antibody molecules to 4-1BB.

The binding abilities of the antibody molecules to a CHO-K1 cell strain highly expressing human 4-1BB (CHO-K1/hu4-1BB, GenScript, M00538) and a CHO-K1 cell strain highly expressing cynomolgus monkey 4-1BB (CHO-K1/cyno4-1BB, GenScript, M00569) were determined by flow cytometry FACS. Specifically, the cells were digested and resuspended in a complete medium, and the cell density was adjusted to 2×10⁶ cells/mL. Then, the cells were seeded in a 96-well V-bottom plate (Corning, #3894) at 100 µL/well (2×10⁵ cells/well) and centrifuged at 4 °C for 5 min, and the supernatant was discarded. Then, the serially diluted antibody molecules were added to the 96-well plate at 100 µL/well, and the mixtures were well mixed. The antibody molecules might serially diluted 3-fold from the highest final concentration 200 nM to obtain a total of 12 concentrations. hIgG1 iso (CrownBio, #C0001) was used as an isotype control. The cells were incubated at 4 °C for 1 h away from light. Then, the cells in each well were rinsed twice with 100 µL of pre-cooled FACS buffer (PBS buffer containing 0.5% BSA) and centrifuged at 500 g at 4 °C for 5 min, and then the supernatant was discarded. Then, a fluorescent secondary antibody (Goat human IgG (H+L) Alexa Fluor 488 conjugation, Thermo, #A11013, 1:1000 diluted) was added at 100 µL/well, and the plate was incubated at 4 °C for 1 h away from light. Then, the cells in each well were then rinsed twice with 200 µL of pre-cooled FACS buffer and centrifuged at 500 g at 4 °C for 5 min, and then the supernatant was discarded. Finally, a pre-cooled FACS buffer was added at 200 µL/well to resuspend the cells. The fluorescence signal values were read using a BD FACS CANTOII flow cytometer.

The data were processed and analyzed by plotting using GraphPad Prism 8 software, and binding curves of the antibodies to target cells, EC₅₀ values and other parameters were obtained through four-parameter nonlinear fitting.

In this example, the anti-4-1BB monoclonal antibody Urelumab or Utomilumab was used as a positive control molecule.

### Example 4.3.1. Binding to CHO-K1 cells CHO-K1/hu4-1BB highly expressing human 4-1BB

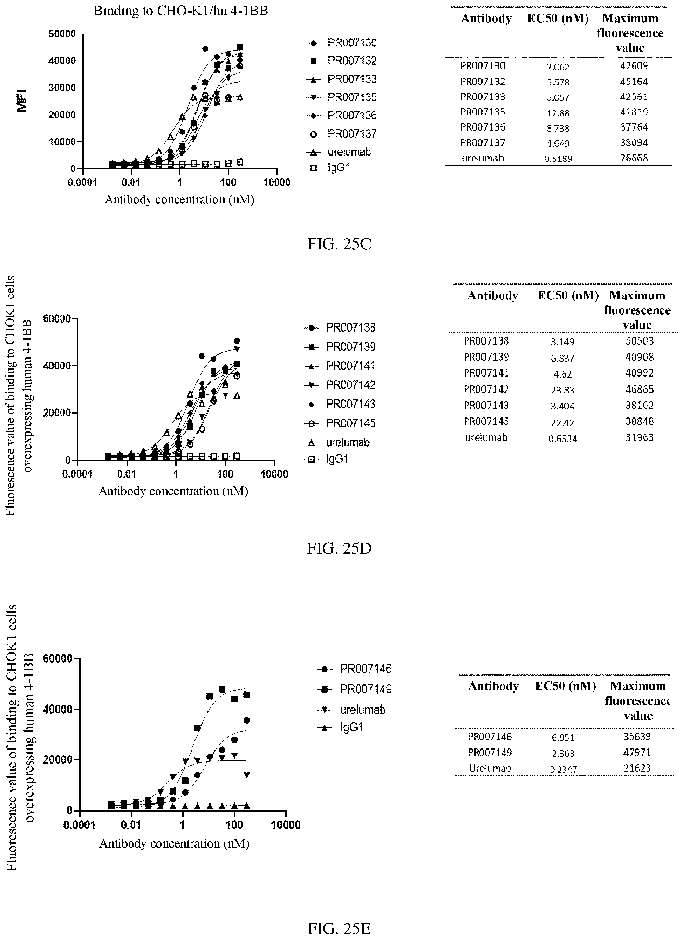

As shown in FIG. 25A, the abilities of the bispecific antibody molecules of the IgG-VH (C-terminus) tetravalent symmetric structure (PR003549, PR003550 and PR003551) to bind to human 4-1BB were superior to that of the bispecific antibody molecules of the FIT-Ig structure (PR000701 and PR003052), and their maximum MFI values were superior to that of the positive control antibody Urelumab.

As shown in FIG. 25B, with respect to the ability to bind to human 4-1BB, the bispecific antibody molecule PR004270 of the Fab-HCAb symmetric structure and the bispecific antibody molecule PR004268 of the IgG-VH (N-terminus) tetravalent symmetric structure were superior in the maximum MFI value to the positive control antibodies Urelumab and Utomilumab.

As shown in FIGs. 25C-25E, with respect to binding to human 4-1BB, the bispecific antibody molecules of the IgG-VH (C-terminus) tetravalent symmetric structure (PR007130, PR007132, PR007133, PR007135, PR007136, PR007137, PR007138, PR007139, PR007141, PR007142, PR007143, PR007145, PR007146 and PR007149) were superior in the maximum MFI value to the positive control Urelumab.

### Example 4.3.2. Binding to CHO-K1 cell CHO-K1/cyno 4-1BB highly expressing cynomolgus monkey 4-1BB

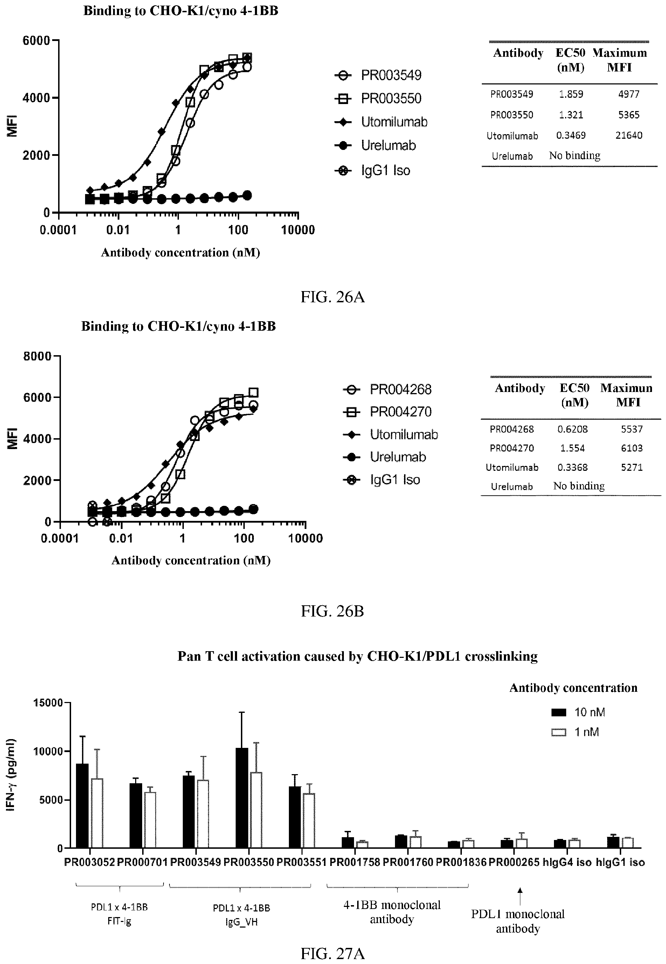

As shown in FIGs. 26A and 26B, the bispecific antibody molecules of this example were able to bind to cynomolgus monkey 4-1BB, whereas Urelumab was not. With respect to the ability to bind to cynomolgus monkey 4-1BB, PR004270 was slightly superior in the maximum MFI value to Utomilumab.

### Example 4.4. T cell specific activation mediated by target cells highly expressing PD-L1

This example is intended to investigate the activity of the PD-L1×4-1BB bispecific antibody molecules to activate T cells by binding to 4-1BB in the presence of target cells. The target cells may be cells expressing PD-L1 at different levels, e.g., CHO-K1/hPDL1 (GenScript, M00543) highly expressing human PD-L1 or MDA-MB-231 (ATCC, HTB-26) highly expressing human PD-L1. The effector cells may be isolated human PBMCs or T cells.

Specifically, a 96-well plate (Corning, #3599) was coated first with 0.3 µg/mL anti-CD3 antibody OKT3 (Thermo, #16-0037-81) at 100 µL/well. Then, the density of human T cells (isolated from human PBMCs with a T cell isolation kit (Miltenyi, #130-096-535)) was adjusted to 2×10⁶ cells/mL, and the density of target cells was adjusted to 3×10⁵ cells/mL. The two cell suspensions were each seeded into a 96-well plate at 50 µL/well, with a final effector-to-target ratio of 20:3. Then, antibody molecules at different concentrations were added at 100 µL/well, wherein the antibody concentration may be the final concentration of (10 nM, 1 nM) or 20 nM, or a total of 8 concentrations obtained by a 5-fold gradient dilution from the highest final concentration of 20 nM; and two duplicate wells were set for each concentration. hIgG1 iso (CrownBio, #C0001) and hIgG4 iso (CrownBio, #C0045) were used as controls. The 96-well plate was incubated in an incubator at 37 °C with 5% CO₂ for 3 days. Supernatants after 48 h and 72 h of culture were each collected. The IL-2 concentration in the 48-hour supernatant was determined using an IL-2 ELISA kit (Thermo, #88-7025-88), and the IFN-γ concentration in the 72-hour supernatant was determined using an IFN-γ ELISA kit (Thermo, #88-7316-77). The ELISA assay was performed by referring to the instructions of relevant kit.

The data were processed and analyzed by plotting using a GraphPad Prism 8 software.

In this example, the anti-4-1BB monoclonal antibody Urelumab was used as a positive control molecule.

### Example 4.4.1. CHO-K1/hPDL1-mediated T cell specific activation

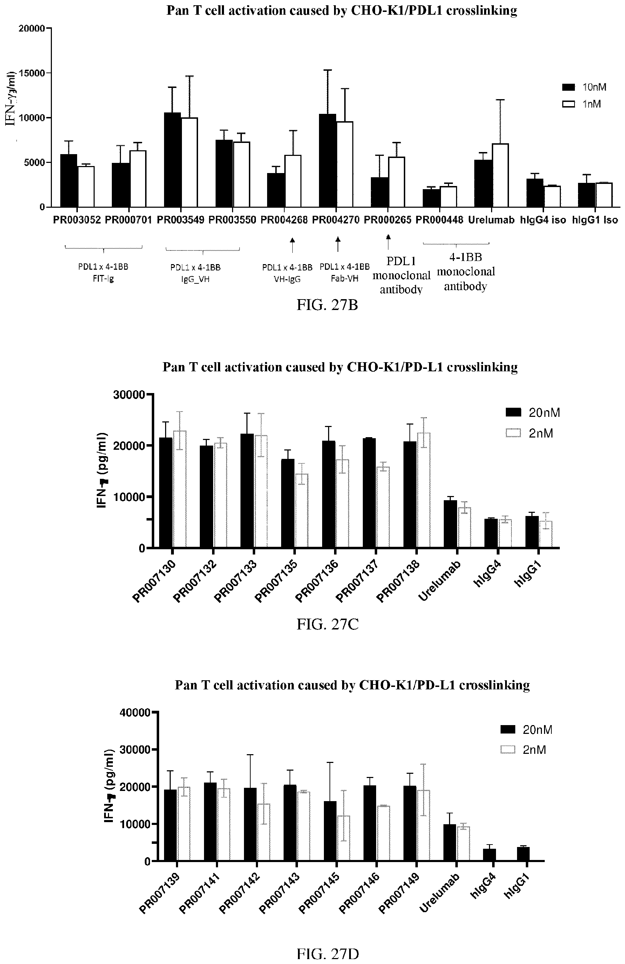

As shown in FIGs. 27A-27D, in a system where the CHO-K1/hPDL1 target cells and T cells were mixed, non-crosslinking-dependent anti-4-1BB monoclonal antibody Urelumab was able to activate T cells to release IFN-γ, while crosslinking-dependent anti-4-1BB monoclonal antibodies (PR000448, PR001758, PR001760 and PR001836) were hardly able to activate T cells. The bispecific antibody molecules of the FIT-Ig structure (PR003502 and PR000701), the bispecific antibody molecules of the IgG-VH tetravalent symmetric structure (PR003549, PR003550, PR003551, PR004268, PR007130, PR007132, PR007133, PR007135, PR007136, PR007137, PR007138, PR007139, PR007141, PR007142, PR007143, PR007145, PR007146 and PR007149), and the bispecific antibody molecule of the Fab-HCAb structure (PR004270) were all able to activate T cells to release cytokines. This indicates that the activation of T cells by the bispecific antibody molecules is dependent on the specific activation of target cells. Moreover, the bispecific antibody molecules of the IgG-VH tetravalent symmetric structure (PR003549, PR003550, PR007130, PR007132, PR007133, PR007135, PR007136, PR007137, PR007138, PR007139, PR007141, PR007142, PR007143, PR007145, PR007146 and PR007149) and the bispecific antibody molecule of the Fab-HCAb structure (PR004270) were able to cause higher release levels of cytokines and show stronger T cell activation ability than the bispecific antibody molecules of the FIT-Ig structure (PR003502 and PR000701); and those bispecific antibody molecules were superior to Urelumab.

### Example 4.4.2. MDA-MB-231-mediated T cell specific activation

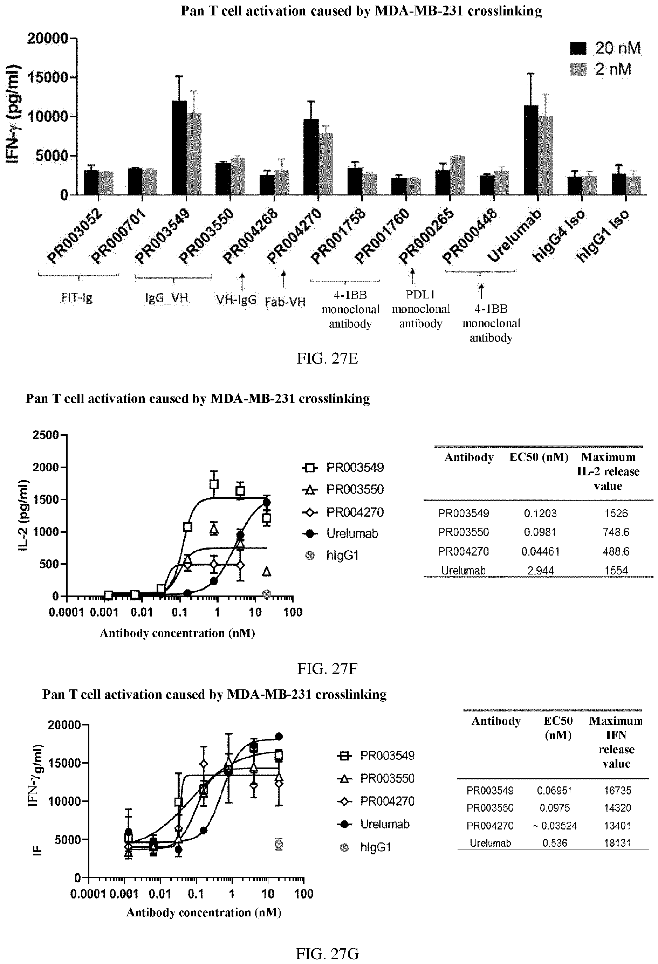

As shown in FIG. 27E, in a system where the MDA-MB-231 target cells and T cells were mixed, the bispecific antibody molecule of the IgG-VH tetravalent symmetric structure (PR003549) and the bispecific antibody molecule of the Fab-HCAb structure (PR004270) had similar T cell activation abilities, and they were superior to the bispecific antibody molecules of the FIT-Ig structure (PR003502 and PR000701).

As shown in FIGs. 27F and 27G, in a system where the MDA-MB-231 target cells and T cells were mixed, the bispecific antibody molecule of the IgG-VH tetravalent symmetric structure (PR003549) had stronger T cell activation ability than Urelumab, and was able to better promote the production of IFN-γ and IL-2.

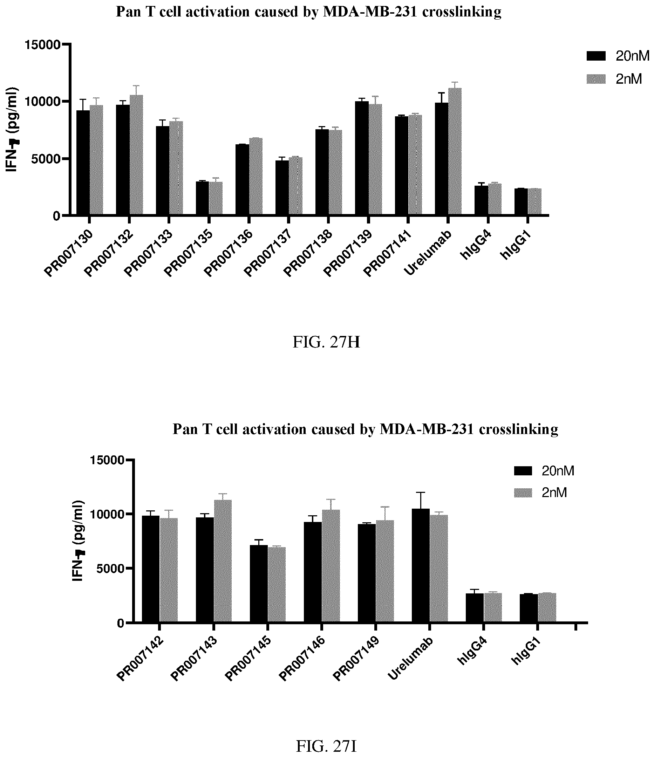

As shown in FIGs. 27H and 27I, in a system where the MDA-MB-231 target cells and T cells were mixed, the bispecific antibody molecules of the IgG-VH tetravalent symmetric structure (PR007130, PR007132, PR007133, PR007138, PR007139, PR007141, PR007142, PR007143, PR007145, PR007146 and PR007149) had a T cell activation ability comparable to that of Urelumab.

### Example 4.5. Mixed Lymphocyte Reaction (MLR)

This example is intended to investigate the T cell activation effects of PD-L1×4-1BB bispecific antibody molecules by the mixed lymphocyte reaction (MLR).

In the first step, monocytes were isolated from PBMCs (MT-Bio) of a first donor using CD14 magnetic beads (Meltenyi, #130-050-201) according to the instructions of the relevant kit. Then, 50 ng/mL of recombinant human IL-4 (PeproTech, #200-02-A) and 100 ng/mL of recombinant human GM-CSF (PeproTech, #300-03-A) were added, and after 7 days of induction at 37 °C, immature dendritic cells (iDC cells) were obtained. 1 µg/mL lipopolysaccharide (LPS, Sigma, #L6529) was then added, and after 24 h of induction, mature dendritic cells (mDC cells) were obtained. In the second step, T lymphocytes were isolated from PBMCs (MT-Bio) of a second donor using a T cell isolation kit (Meltenyi, #130-096-535). In the third step, the obtained T cells and mDC cells were seeded in a 96-well plate (T cells at 1×10⁵/well and mDC cells at 2×10⁴/well) at a ratio of 5:1. Then, antibody molecules at different concentrations were added at 50 µL/well, wherein the antibody concentration may be the final concentration of (10 nM, 1 nM), or a total of 8 concentrations obtained by a 3-fold gradient dilution from the highest final concentration of 50 nM; and two duplicate wells were set for each concentration. hIgG 1 iso (CrownBio, #C0001) or a blank well was used as a control. The cells were incubated in an incubator at 37 °C with 5% CO₂ for 5 days. In the fourth step, supernatants on day 3 and on day 5 were each collected. The IL-2 concentration in the 3-day supernatant was determined using an IL-2 ELISA kit (Thermo, #88-7025-88), and the IFN-γ concentration in the 5-day supernatant was determined using an IFN-γ ELISA kit (Thermo, #88-7316-77). The ELISA assay was performed by referring to the instructions of relevant kit.

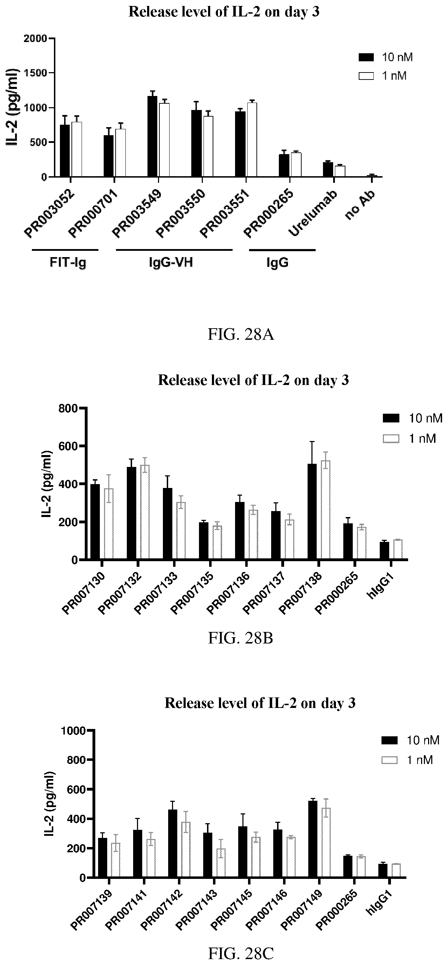

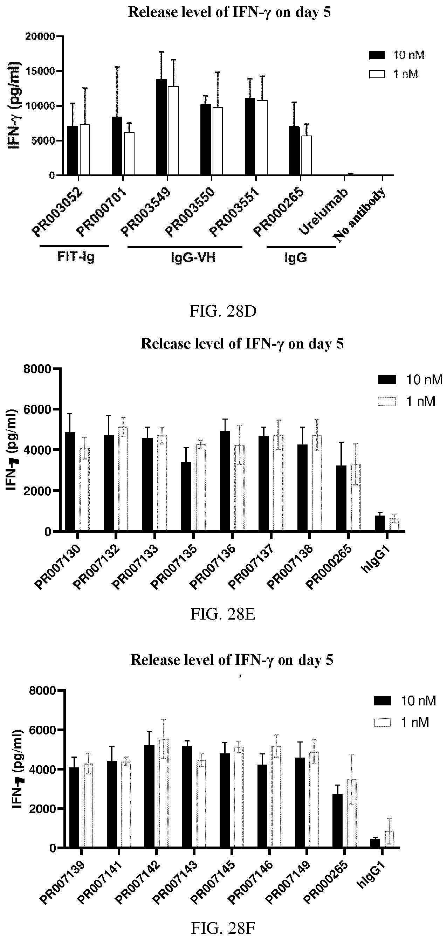

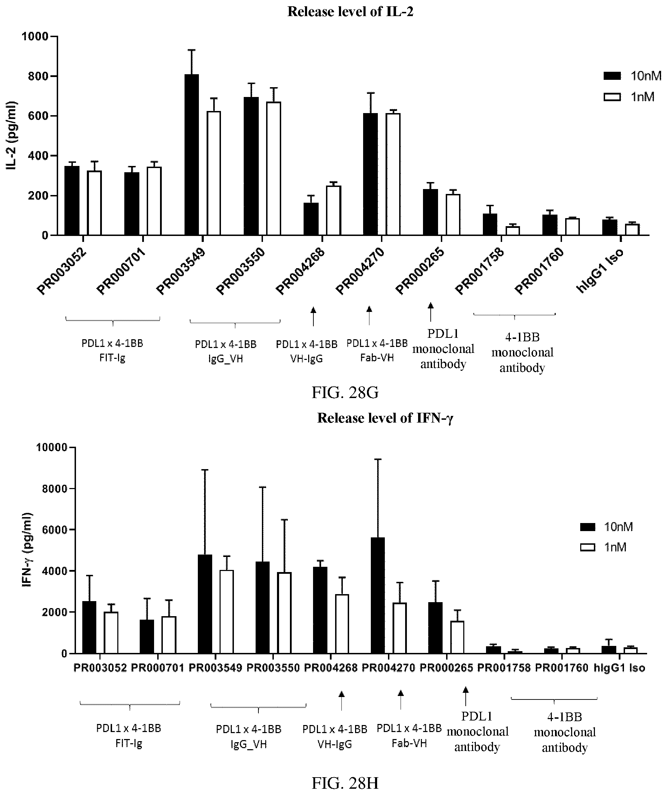

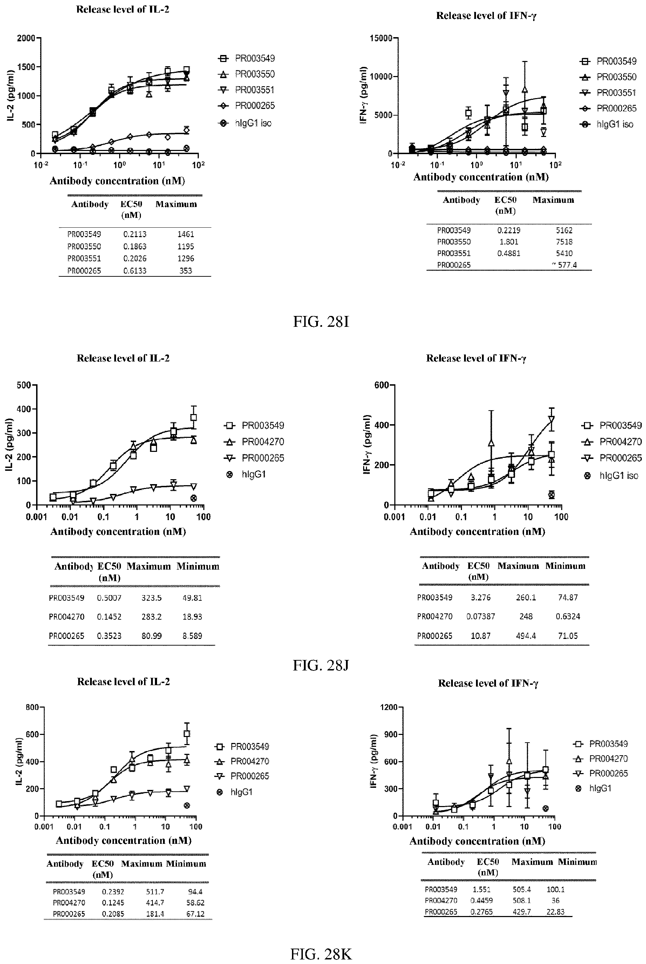

As shown in FIG. 28, in multiple independent MLR experiments (with different donor pairings), the anti-4-1BB monoclonal antibodies had a limited T cell activation effect and very little cytokine production ability; while the anti-PD-L1 monoclonal antibodies had a significant activation effect. The bispecific antibody molecules were able to further improve the function of T cells.

As shown in FIGs. 28A-28F, the bispecific antibody molecules of the IgG-VH tetravalent symmetric structure (PR003549, PR003550, PR003551, PR007130, PR007132, PR007133, PR007138, PR007139, PR007141, PR007142, PR007143, PR007145, PR007146 and PR007149) were able to cause higher release levels of cytokines and show stronger T cell activation abilities than the parent PD-L1 antibody PR000265, which indicates that the bispecific antibody molecules were superior to the anti-PD-L1 monoclonal antibodies.

As shown in FIGs. 28A and 28D, the bispecific antibody molecules of the IgG-VH tetravalent symmetric structure (PR003549, PR003550 and PR003551) were able to cause higher release levels of cytokines and show stronger T cell activation abilities than the bispecific antibody molecules of the FIT-Ig structure (PR003502 and PR000701); and the bispecific antibody molecules were superior to the anti-PD-L1 monoclonal antibodies.

As shown in FIGs. 28G and 28H, the bispecific antibody molecules of the IgG-VH tetravalent symmetric structure (PR003549 and PR003550) and the bispecific antibody molecule of the Fab-HCAb structure (PR004270) were able to cause higher release levels of cytokines and show stronger T cell activation abilities than the bispecific antibody molecules of the FIT-Ig structure (PR003502 and PR000701).

As shown in FIGs. 28I-28K, the bispecific antibody molecules of the IgG-VH tetravalent symmetric structure (PR003549, PR003550 and PR003551) and the bispecific antibody molecule of the Fab-HCAb structure (PR004270) showed strong T cell activation abilities.

### Example 4.6. Pharmacokinetic study

In this example, the pharmacokinetic properties of the PD-L1×4-1BB bispecific antibody molecule of a Fab-HCAb symmetric structure (PR004270) in mice were investigated.

The test was performed as follows: for each test antibody molecule, 6 female BALB/c mice weighing 18-22 g were selected and were given the bispecific antibody by intravenous injection at a dose of 5 mg/kg. The whole blood of 3 mice in one group was collected before the administration and 15 min, 24 h (1 day), 4 days and 10 days after the administration, and the whole blood of 3 mice in the other group was collected before the administration and 5 h, 2 days, 7 days and 14 days after the administration. The whole blood was left to stand for 30 min for coagulation, and then centrifuged, and the isolated serum sample was cryopreserved at -80 °C until it was taken for analysis. In this example, the drug concentration in the serum of mice was quantitatively determined by two ELISA methods. The ELISA method I, namely the Fc end detection (overall detection) method, was performed by capturing the antibody containing human Fc in the serum of mice using a goat anti-human Fc polyclonal antibody coating a 96-well plate, and then adding an HRP-labeled goat anti-human Fc secondary antibody; and the ELISA method II, namely the PD-L1-end detection (functional domain detection) method, was performed by capturing an antibody specifically recognizing PD-L1 in the serum of mice using a human PD-L1 protein coating a 96-well plate, and then adding an HRP-labeled goat anti-human Fc secondary antibody. Pharmacokinetic parameters were analyzed using Phoenix WinNonlin software version 8.2 by non-compartmental analysis (NCA).

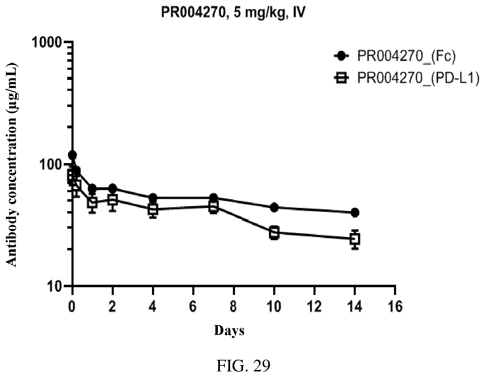

As shown in FIG. 29 and Table 32, the bispecific antibody molecule of the Fab-HCAb structure (PR004270) had a serum half-life t_{1/2} value similar to that of a conventional IgG antibody, and the t_{1/2} value was more than 10 days as measured by the functional domain detection method.

**Table 32. Pharmacokinetics of PR004270 in BALB/c mice**

| Bispecific antibody molecules | PR004270 | |
|---|---|---|
| Animals (number) | BALB/c mice (n = 6) | |
| Antibody dosage | 5 mg/kg, I.V. | |

| PK parameters | Fc end detection | PD-L1 end detection |
|---|---|---|
| T_{1/2} (hour) | 465.6 | 256.5 |
| V_{d} (mL/kg) | 75.7 | 83.9 |
| AUC (µg^{∗}hour/mL) | 17,536 | 13,126 |
| Cl (mL/hour/kg) | 0.11 | 0.23 |
| C₀ (µg/mL) | 119.7 | 81.7 |

### Summary

In this example, PD-L1×4-1BB bispecific antibody molecules of a variety of structures are constructed using the antigen-binding domain Fab of the anti-PD-L1 IgG antibody and the antigen-binding domain VH of the 4-1BB HCAb antibody. This shows the flexibility of constructing the molecular structure of bispecific antibodies based on HCAbs, and the T cell function-activating activity is regulated through different structure types, relative positions, binding valences and other parameters.

The activation of T cells by Urelumab is not target-specific, which is one of the causes for its clinical toxic and side effects. The T cell activation effect of PD-L1×4-1BB bispecific antibodies is specifically dependent on the expression of PD-L1. Crosslinking-dependent anti-4-1BB HCAb monoclonal antibodies are unable to directly activate T cells, but the PD-L1×4-1BB bispecific antibodies constructed using those HCAb monoclonal antibodies are able to specifically activate T cells in the presence of cells highly expressing PD-L1.

In one aspect, HCAb-based bispecific antibody structures, especially bispecific antibody molecules of IgG-VH tetravalent symmetric structures or bispecific antibody molecules of Fab-HCAb structures, retain the activity of the PD-L1 end and exhibit better T cell activation ability than the corresponding anti-PD-L1 parent monoclonal antibody in MLR experiments; in another aspect, the PD-L1 molecules highly expressed on target cells can mediate crosslinking and trimerization of 4-1BB to transmit T cell activation signals, and their T cell activation ability was even superior to that of Urelumab. Moreover, the bispecific antibody molecules of IgG-VH tetravalent symmetric structures or Fab-HCAb symmetric structures demonstrate better T cell activation ability than bispecific antibody molecules of FIT-Ig structures.

In conclusion, the PD-L1×4-1BB bispecific antibody molecules with good safety, outstanding functional activity and good molecular stability are constructed in this example.

References:
1. Bertram EM, Lau P, Watts TH. Temporal segregation of 4-1BB versus CD28-mediated costimulation: 4-1BB ligand influences T cell numbers late in the primary response and regulates the size of the T cell memory response following influenza infection. Journal of immunology 2002;168: 3777-85.
2. Kawalekar OU, O'Connor RS, Fraietta JA, et al. Distinct Signaling of Coreceptors Regulates Specific Metabolism Pathways and Impacts Memory Development in CAR T Cells. Immunity 2016;44: 380-90.
3. Ye Z, Hellstrom I, Hayden-Ledbetter M, Dahlin A, Ledbetter JA, Hellstrom KE. Gene therapy for cancer using single-chain Fv fragments specific for 4-1BB. Nature medicine 2002;8: 343-8.
4. Zhang H, Knutson KL, Hellstrom KE, Disis ML, Hellstrom I. Antitumor efficacy of CD137 ligation is maximized by the use of a CD137 single-chain Fv-expressing whole-cell tumor vaccine compared with CD137-specific monoclonal antibody infusion. Molecular cancer therapeutics 2006;5: 149-55.
5.Yang Y, Yang S, Ye Z, et al. Tumor cells expressing anti-CD137 scFv induce a tumor-destructive environment. Cancer research 2007;67: 2339-44.
6. Martinet O, Divino CM, Zang Y, et al. T cell activation with systemic agonistic antibody versus local 4- 1BB ligand gene delivery combined with interleukin-12 eradicate liver metastases of breast cancer. Gene therapy2002;9: 786-92.

## Claims

1. A 4-1BB-binding protein, comprising a heavy chain variable region, wherein
the heavy chain variable region comprises HCDR1, HCDR2 and HCDR3, wherein the HCDR1 comprises a sequence as set forth in SEQ ID NO: 15 or a variant 1 thereof, or SEQ ID NO: 16; the HCDR2 comprises a sequence selected from SEQ ID NO: 60 or a variant 2 thereof, and SEQ ID NO: 50 or a variant 4 thereof; the HCDR3 comprises a sequence as set forth in SEQ ID NO: 103 or a variant 3 thereof, SEQ ID NO: 333 or a variant 5 thereof, SEQ ID NO: 110, SEQ ID NO: 111, SEQ ID NO: 116, SEQ ID NO: 326, SEQ ID NO: 331, SEQ ID NO: 334 or SEQ ID NO: 96;
wherein:
the variant 1 comprises mutations of one or more of T3I, S6N/G/R and Y7F; preferably, the sequence of variant 1 is as set forth in SEQ ID NO: 19, SEQ ID NO: 20, SEQ ID NO: 22 SEQ ID NO: 23, SEQ ID NO: 300 or SEQ ID NO: 24 in the sequence listing;
the variant 2 comprises mutations of one or more of S1G/N/D, G2S/A, S3D, G5D/N/F/S/V and S6T/N/D; preferably, the sequence of variant 2 is as set forth in any one of SEQ ID NOs: 57-59, SEQ ID NO: 61, SEQ ID NO: 49, SEQ ID NOs: 308-317 and SEQ ID NOs: 63-71 in the sequence listing;
the variant 3 comprises mutations of one or more of G2R/D/A/K, S3A/T, S4G/N/A/T/H, E5T/V/M/G, T6A, D7G/S, H9Y/S/N, Y10H, Y11F, N12G/D and V13I/M/T; preferably, the amino acid sequence of variant 3 is as set forth in any one of SEQ ID NO: 101, SEQ ID NO: 102, SEQ ID NOs: 104-106, SEQ ID NO: 108, SEQ ID NO: 109 and SEQ ID NOs: 112-115 in the sequence listing;
the variant 4 comprises mutations of N1I or N1Q; preferably, the amino acid sequence of variant 4 is as set forth in SEQ ID NO: 53 or 54 in the sequence listing;
the variant 5 comprises mutations of E4A/P and/or N13Y; preferably, the amino acid sequence of variant 5 is as set forth in SEQ ID NOs: 327-330 in the sequence listing;
wherein the variant 1, the variant 2, the variant 3, the variant 5 and the variant 4 at least maintains the functions of pre-mutation sequences.

2. The 4-1BB-binding protein according to claim 1, wherein the HCDR1, the HCDR2 and the HCDR3 comprise sequences as set forth in SEQ ID NO: 16, SEQ ID NO: 50 and SEQ ID NO: 96, respectively; or SEQ ID NO: 16, SEQ ID NO: 53 and SEQ ID NO: 96, respectively; or SEQ ID NO: 16, SEQ ID NO: 54 and SEQ ID NO: 96, respectively; or SEQ ID NO: 19, SEQ ID NO: 57 and SEQ ID NO: 101, respectively; or SEQ ID NO: 15, SEQ ID NO: 58 and SEQ ID NO: 102, respectively; or SEQ ID NO: 20, SEQ ID NO: 59 and SEQ ID NO: 103, respectively; or SEQ ID NO: 20, SEQ ID NO: 60 and SEQ ID NO: 104, respectively; or SEQ ID NO: 15, SEQ ID NO: 61 and SEQ ID NO: 105, respectively; or SEQ ID NO: 15, SEQ ID NO: 60 and SEQ ID NO: 106, respectively; or SEQ ID NO: 20, SEQ ID NO: 63 and SEQ ID NO: 108, respectively; or SEQ ID NO: 20, SEQ ID NO: 60 and SEQ ID NO: 108, respectively; or SEQ ID NO: 22, SEQ ID NO: 64 and SEQ ID NO: 109, respectively; or SEQ ID NO: 15, SEQ ID NO: 60 and SEQ ID NO: 110, respectively; or SEQ ID NO: 15, SEQ ID NO: 65 and SEQ ID NO: 111, respectively; or SEQ ID NO: 22, SEQ ID NO: 66 and SEQ ID NO: 112, respectively; or SEQ ID NO: 15, SEQ ID NO: 49 and SEQ ID NO: 113, respectively; or SEQ ID NO: 20, SEQ ID NO: 60 and SEQ ID NO: 103, respectively; or SEQ ID NO: 15, SEQ ID NO: 63 and SEQ ID NO: 104, respectively; or SEQ ID NO: 15, SEQ ID NO: 60 and SEQ ID NO: 114, respectively; or SEQ ID NO: 23, SEQ ID NO: 67 and SEQ ID NO: 105, respectively; or SEQ ID NO: 24, SEQ ID NO: 68 and SEQ ID NO: 103, respectively; or SEQ ID NO: 15, SEQ ID NO: 60 and SEQ ID NO: 105, respectively; or SEQ ID NO: 20, SEQ ID NO: 69 and SEQ ID NO: 115, respectively; or SEQ ID NO: 15, SEQ ID NO: 70 and SEQ ID NO: 116, respectively; or SEQ ID NO: 20, SEQ ID NO: 71 and SEQ ID NO: 115, respectively; or SEQ ID NO: 300, SEQ ID NO: 308 and SEQ ID NO: 326, respectively; or SEQ ID NO: 300, SEQ ID NO: 309 and SEQ ID NO: 327, respectively; or SEQ ID NO: 300, SEQ ID NO: 310 and SEQ ID NO: 328, respectively; or SEQ ID NO: 300, SEQ ID NO: 308 and SEQ ID NO: 329, respectively; or SEQ ID NO: 300, SEQ ID NO: 311 and SEQ ID NO: 330, respectively; or SEQ ID NO: 300, SEQ ID NO: 312 and SEQ ID NO: 331, respectively; or SEQ ID NO: 300, SEQ ID NO: 308 and SEQ ID NO: 332, respectively; or SEQ ID NO: 300, SEQ ID NO: 313 and SEQ ID NO: 330, respectively; or SEQ ID NO: 300, SEQ ID NO: 314 and SEQ ID NO: 329, respectively; or SEQ ID NO: 300, SEQ ID NO: 315 and SEQ ID NO: 331, respectively; or SEQ ID NO: 300, SEQ ID NO: 314 and SEQ ID NO: 327, respectively; or SEQ ID NO: 300, SEQ ID NO: 316 and SEQ ID NO: 331, respectively; or SEQ ID NO: 300, SEQ ID NO: 308 and SEQ ID NO: 333, respectively; or SEQ ID NO: 300, SEQ ID NO: 317 and SEQ ID NO: 334, respectively, in the sequence listing;
preferably, the gene encoding an FR of the heavy chain variable region is derived from germline V gene IGHV4-34, IGHV3-23, IGHV3-11 or IGHV3-74;
more preferably, the heavy chain variable region comprises a sequence as set forth in any one of SEQ ID NO: 168, SEQ ID NO: 171, SEQ ID NO: 172, SEQ ID NOs: 175-180, SEQ ID NOs: 182-196, SEQ ID NOs: 337-352 and SEQ ID NO: 198 in the sequence listing.

3. The 4-1BB-binding protein according to claim 1 or 2, wherein the 4-1BB-binding protein further comprises a light chain variable region comprising LCDR1, LCDR2 and LCDR3, wherein the LCDR1 comprises a sequence as set forth in SEQ ID NO: 133, the LCDR2 comprises a sequence as set forth in SEQ ID NO: 145, and the LCDR3 comprises a sequence as set forth in SEQ ID NO: 158;
preferably, the gene encoding an FR of the light chain variable region is derived from germline V gene IGKV3-15, wherein: LFWR1 preferably comprises an amino acid sequence as set forth in SEQ ID NO: 126 or SEQ ID NO: 128, LFWR2 preferably comprises an amino acid sequence as set forth in SEQ ID NO: 140, LFWR3 preferably comprises an amino acid sequence as set forth in SEQ ID NO: 151, and LFWR4 preferably comprises an amino acid sequence as set forth in SEQ ID NO: 164;
more preferably, the light chain variable region comprises a sequence as set forth in SEQ ID NO: 201 in the sequence listing or a variant thereof, wherein the variant comprises one or more amino acid residue mutations on the basis of a sequence as set forth in SEQ ID NO: 201; the resulting light chain variable region after the mutations preferably comprises a sequence as set forth in SEQ ID NO: 204;
even more preferably, the amino acid sequence of heavy chain variable region is as set forth in SEQ ID NO: 168, and the amino acid sequence of light chain variable region is as set forth in SEQ ID NO: 201; or, the amino acid sequence of heavy chain variable region is as set forth in SEQ ID NO: 171, and the amino acid sequence of light chain variable region is as set forth in SEQ ID NO: 204; or, the amino acid sequence of heavy chain variable region is as set forth in SEQ ID NO: 172, and the amino acid sequence of light chain variable region is as set forth in SEQ ID NO: 204.

4. The 4-1BB-binding protein according to any one of claims 1-3, wherein the 4-1BB-binding protein further comprises a heavy chain constant region and/or a light chain constant region; preferably, the heavy chain constant region is selected from that of hIgG1, hIgG2, hIgG3 and hIgG4 or variants thereof, and the light chain constant region is selected from that of a κ chain and a λ chain or mutations thereof, wherein:
the variant of hIgG1 preferably comprises one or more of mutations of L234A, L235A, E345R and P329G, more preferably E345R, or a combination of L234A, L235A and E345R, or a combination of L234A, L235A and P329G; the variant of hIgG4 preferably comprises a mutation S228P.

5. The 4-1BB-binding protein according to claim 4, wherein the 4-1BB-binding protein comprises a heavy chain and a light chain, wherein the heavy chain comprises a sequence as set forth in any one of SEQ ID NO: 209, SEQ ID NO: 212, SEQ ID NO: 213, SEQ ID NOs: 216-222, SEQ ID NOs: 224-238, SEQ ID NOs: 353-368 and SEQ ID NO: 240 in the sequence listing; and/or the light chain comprises a sequence as set forth in SEQ ID NO: 246 or SEQ ID NO: 243 in the sequence listing;
preferably, the amino acid sequence of heavy chain is shown in SEQ ID NO: 209, and the amino acid sequence of light chain is as set forth in SEQ ID NO: 243; or, the amino acid sequence of heavy chain is as set forth in SEQ ID NO: 212, and the amino acid sequence of light chain is as set forth in SEQ ID NO: 246; or, the amino acid sequence of heavy chain is as set forth in SEQ ID NO: 213, and the amino acid sequence of light chain is as set forth in SEQ ID NO: 246; or, the amino acid sequence of heavy chain is as set forth in SEQ ID NO: 216, and the amino acid sequence of light chain is as set forth in SEQ ID NO: 246.

6. The 4-1BB-binding protein according to any one of claims 1-5, wherein the 4-1BB binding protein is in the form of a full-length antibody, Fab, Fab', F(ab')₂, Fv, scFv, a bispecific antibody, a multispecific antibody, a heavy-chain antibody, a single-domain antibody or a single-region antibody, or a monoclonal or polyclonal antibody prepared from an antibody as above, wherein
preferably, the full-length antibody comprises a heavy chain comprising a sequence as set forth in SEQ ID NO: 209, and a light chain comprising a sequence as set forth in SEQ ID NO: 243; or a heavy chain comprising a sequence as set forth in SEQ ID NO: 212, and a light chain comprising a sequence as set forth in SEQ ID NO: 246; or a heavy chain comprising a sequence as set forth in SEQ ID NO: 213, and a light chain comprising a sequence as set forth in SEQ ID NO: 246; or a heavy chain comprising a sequence as set forth in SEQ ID NO: 216, and a light chain comprising a sequence as set forth in SEQ ID NO: 246.

7. A bispecific antibody comprising a first protein functional region and a second protein functional region, wherein the first protein functional region is the 4-1BB-binding protein according to any one of claims 1-6, and the second protein functional region targets a tumor antigen, and is preferably an HER2 antibody or a PD-L1 antibody, wherein the HER2 antibody is preferably trastuzumab or pertuzumab, and the PD-L1 antibody is preferably atezolizumab or PR000265, wherein the PR000265 comprises a heavy chain as set forth in SEQ ID NO: 211 and a light chain as set forth in SEQ ID NO: 245.

8. The bispecific antibody according to claim 7, wherein the first protein functional region or the second protein functional region is in the form of scFv, VHH, an immunoglobulin, Fab,Fab', F(ab')₂ or a heavy chain variable region; for example, the first protein functional region is Fab and the second protein functional region is VHH; or, the first protein functional region is Fab, and the second protein functional region is an immunoglobulin; or, the first protein functional region is an immunoglobulin, and the second protein functional region is a heavy chain variable region.

9. The bispecific antibody according to claim 8, wherein the first protein functional region and the second protein functional region and/or a heavy chain variable region and a light chain variable region of the scFv are linked by a linker, wherein the amino acid sequence of the linker is GS, or as set forth in any one of SEQ ID NOs: 273-293 in the sequence listing.

10. The bispecific antibody according to claim 8 or 9, wherein the bispecific antibody comprises a polypeptide chain 1 and a polypeptide chain 2, and preferably further comprises a polypeptide chain 3;
preferably:
the amino acid sequence of polypeptide chain 1 is as set forth in SEQ ID NO: 244, and the amino acid sequence of polypeptide chain 2 is as set forth in any one of SEQ ID NOs: 251-265; or the amino acid sequence of polypeptide chain 1 is as set forth in SEQ ID NO: 245, and the amino acid sequence of polypeptide chain 2 is as set forth in SEQ ID NO: 271; or the amino acid sequence of polypeptide chain 1 is as set forth in SEQ ID NO: 249, and the amino acid sequence of polypeptide chain 2 is as set forth in SEQ ID NO: 272; or the amino acid sequence of polypeptide chain 1 is as set forth in SEQ ID NO: 245, and the amino acid sequence of polypeptide chain 2 is as set forth in SEQ ID NO: 270; or the amino acid sequence of polypeptide chain 1 is as set forth in SEQ ID NO: 245, and the amino acid sequence of polypeptide chain 2 is as set forth in SEQ ID NO: 269; or the amino acid sequence of polypeptide chain 1 is as set forth in SEQ ID NO: 245, and the amino acid sequence of polypeptide chain 2 is as set forth in SEQ ID NO: 268; or the amino acid sequence of polypeptide chain 1 is as set forth in SEQ ID NO: 245, and the amino acid sequence of polypeptide chain 2 is as set forth in any one of SEQ ID NOs: 369-382; or the amino acid sequence of polypeptide chain 1 is as set forth in SEQ ID NO: 267, the amino acid sequence of polypeptide chain 2 is as set forth in SEQ ID NO: 266, and the amino acid sequence of polypeptide chain 3 is as set forth in SEQ ID NO: 246; or the amino acid sequence of polypeptide chain 1 is as set forth in SEQ ID NO: 250, the amino acid sequence of polypeptide chain 2 is as set forth in SEQ ID NO: 249, and the amino acid sequence of polypeptide chain 3 is as set forth in SEQ ID NO: 246.

11. An isolated nucleic acid encoding the 4-1BB-binding protein according to any one of claims 1-6 or the bispecific antibody according to any one of claims 7-10.

12. An expression vector comprising the isolated nucleic acid according to claim 11.

13. A host cell comprising the expression vector according to claim 12, wherein preferably, the host cell is a prokaryotic cell or a eukaryotic cell.

14. A method for preparing a 4-1BB-binding protein comprising culturing the host cell according to claim 13 and obtaining the 4-1BB-binding protein from the culture.

15. A chimeric antigen receptor comprising the 4-1BB-binding protein according to any one of claims 1-6 or the bispecific antibody according to any one of claims 7-10.

16. An antibody-drug conjugate comprising a cytotoxic agent and the 4-1BB-binding protein according to any one of claims 1-6 or the bispecific antibody according to any one of claims 7-10, wherein preferably, the cytotoxic agent is MMAF or MMAE.

17. A pharmaceutical composition comprising the 4-1BB-binding protein according to any one of claims 1-6, the bispecific antibody according to any one of claims 7-10, and/or the antibody-drug conjugate according to claim 16.

18. Use of the 4-1BB-binding protein according to any one of claims 1-6, the bispecific antibody according to any one of claims 7-10, and the pharmaceutical composition according to claim 17 in the manufacture of a medicament for the treatment and/or prevention of cancer, wherein the cancer is preferably one associated with one or more of HER2, PD-L1 and 4-1BB, e.g., breast cancer, melanoma, lung cancer, gastric cancer, liver cancer, esophageal cancer, cervical cancer, head and neck tumor or colorectal cancer.

19. A kit comprising the 4-1BB-binding protein according to any one of claims 1-6, the bispecific antibody according to any one of claims 7-10, the chimeric antigen receptor according to claim 15, the antibody-drug conjugate according to claim 16 and/or the pharmaceutical composition according to claim 17, wherein
preferably, the kit further comprises (i) a device for administering the antibody or an antigen-binding fragment thereof or the antibody-drug conjugate or the pharmaceutical composition; and/or (ii) instructions for use.

20. A combination of kits comprising a kit A and a kit B, wherein:
the kit A comprises the 4-1BB-binding protein according to any one of claims 1-6, the bispecific antibody according to any one of claims 7-10, the chimeric antigen receptor according to claim 15, the antibody-drug conjugate according to claim 16 and/or the pharmaceutical composition according to claim 17;
the kit B comprises another anti-tumor antibody or a pharmaceutical composition comprising the anti-tumorantibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

21. A method for diagnosing, treating and/or preventing a 4-1BB-mediated disease or disorder comprising administering to a patient in need thereof a therapeutically effective amount of the 4-1BB binding protein according to any one of claims 1-6, the bispecific antibody according to any one of claims 7-10, the chimeric antigen receptor according to claim 15, the antibody-drug conjugate according to claim 16 or the pharmaceutical composition according to claim 17, or treating a patient in need thereof with the combination of kits according to claim 20.

22. The method according to claim 21, wherein the disease or disorder is a tumor, preferably a 4-1BB positive tumor, and more preferably gastric cancer, esophageal cancer, lung cancer, ovarian cancer, melanoma, renal cancer, breast cancer, colorectal cancer, liver cancer, pancreatic cancer, bladder cancer, head and neck cancer, bronchial carcinoma, glioma and/or leukemia.

23. A method for immuno-detection or determination of 4-1BB comprising using the 4-1BB binding protein according to any one of claims 1-6, the bispecific antibody according to any one of claims 7-10, the chimeric antigen receptor according to claim 15, the antibody-drug conjugate according to claim 16 or the pharmaceutical composition according to claim 17, wherein preferably, the detection is not for diagnostic and/or therapeutic purposes.

24. A combination therapy comprising administering to a patient in need thereof the 4-1BB binding protein according to any one of claims 1-6, the bispecific antibody according to any one of claims 7-10, the chimeric antigen receptor according to claim 15, the antibody-drug conjugate according to claim 16 or the pharmaceutical composition according to claim 17, and a second therapeutic agent, wherein the second therapeutic agent preferably comprises another anti-tumor antibody or a pharmaceutical composition comprising the anti-tumor antibody, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.
